# EUROPEAN PATENT SPECIFICATION

(11) **EP 4 351 789 B1**
(45) Date of publication and mention of the grant of the patent: **09.12.2026**
(21) Application number: 22820893.0
(22) Date of filing: 07.06.2022
(51) Int. Cl.: B01L 3/00, C12Q 1/6876, C12Q 1/70

(54) **CAPILLARY FLOW CONTROL VIA DELAMINATING TIMERS**
KAPILLARFLUSSSTEUERUNG ÜBER DELAMINATIONSZEITGEBER
RÉGULATION DE FLUX CAPILLAIRE PAR L'INTERMÉDIAIRE DE TEMPORISATEURS DE DÉSTRATIFICATION

(30) Priority: 07.06.2021 US 202163197711 P
(43) Date of publication of application: 17.04.2024
(73) Proprietor: Georgia Tech Research Corporation, Atlanta, GA 30318 (US)
(72) Inventor: SARIOGLU, Ali Fatih, Atlanta, Georgia 30318 (US); LEE, Dohwan, Atlanta, Georgia 30318 (US); AHMADOV, Tevhide Ozkaya, Atlanta, Georgia 30332 (US)
(74) Representative: Henderson, Helen Lee
(86) International application number: PCT/US2022/032498
(87) International publication number: WO 2022/261092

(56) References cited:
- WO-A1-2015/164112
- WO-A2-2008/049083
- US-A1- 2007 172 963
- US-A1- 2013 020 198
- US-A1- 2018 224 436
- US-A1- 2019 232 284
- US-B1- 10 968 493
- CHEN-HSUN WENG; MING-YI CHEN; CHI-HSIANG SHEN; RUEY-JEN YANG: "Colored wax-printed timers for two-dimensional and three-dimensional assays on paper-based devices", vol. 8, no. 6, 066502, 18 November 2014 (2014-11-18), Online, pages 1 - 12, XP002813186, Retrieved from the Internet <URL:chrome-extension://efaidnbmnnnibpcajpcglclefindmkaj/https://pmc.ncbi.nlm.nih.gov/articles/PMC4240780/pdf/BIOMGB-000008-066502_1.pdf> [retrieved on 20250326], DOI: https://doi.org/10.1063/1.4902246
- LEE DOHWAN ET AL: "Capillary flow control in lateral flow assays via delaminating timers", vol. 7, no. 40, 1 October 2021 (2021-10-01), US, XP093199554, ISSN: 2375-2548, Retrieved from the Internet <URL:https://www.science.org/doi/pdf/10.1126/sciadv.abf9833> DOI: 10.1126/sciadv.abf9833
- LEE ET AL.: "Point-of-Care Toolkit for Multiplex Molecular Diagnosis of SARS-CoV-2 and Influenza A and B Viruses", ACS SENSORS, vol. 6, 15 September 2021 (2021-09-15), pages 3204 - 3213, XP055946782, DOI: 10.1021/acssensors.1c00702

## Description

### BACKGROUND

The standard platform for point-of-care (POC) testing in resource-limited settings has long been the lateral flow assay (LFA) (Sackmann et al., (2014); Gong et al., (2017); Yetisen et al., (2013); Posthuma-Trumpie et al., (2009)). Lateral flow assays (LFAs) utilize capillary flow of liquids for simple detection of analytes. LFAs offer unique advantages, such as rapid analysis, low-cost, portability, ease-of-use, instrument-free operation, and compatibility with various biological samples (e.g., blood, plasma, serum, urine, sweat, and saliva), all of which make LFAs the predominant diagnostic format for POC applications (Lee et al., (2016); Banerjee et al., (2018); Kong et al., (2017); Verma et al., (2018); Fu et al., (2012); Toley at al., (2013); Lutz et al., (2013)). In fact, LFAs readily satisfy the majority of the ASSURED (Affordable, Sensitive, Specific, User-friendly, Rapid and robust, Equipment-free, and Deliverable to end-users) criteria reported by the World Health Organization in 2004 to establish capabilities of the POC devices (Verma et al., (2018); Fu et al., (2012); Toley at al., (2013); Lutz et al., (2013)). On the other hand, conventional LFAs are less prudent in performing multiplexed assays (e.g., simultaneously screening for multiple analytes within a sample) and are also less sensitive in detecting various analytes of clinical importance (Yetisen et al., (2013); Posthuma-Trumpie, et al., (2009); Lee et al., (2016)).

Microfluidic paper-based analytical devices (µPADs) (Cheng et al., (2010); Martinez et al., (2007); Martinez et al., (2008)) combine the capillary driven flow of LFAs with deterministic liquid routing enabled by microfluidic channels. In µPADs, a liquid-confining channel geometry is created on paper by patterning a hydrophobic material (e.g., photoresist (Martinez et al., (2008)), wax (Lu et al., (2009); Lu et al., (2010)), PDMS (Bruzewicz et al., (2008)), and alkyl ketene dimer (Li et al., (2010))), inkjet printing/etching (Abe et al., (2008); Abe et al., (2010)), plasma or laser treatment (Li, et al., (2008); Li et al., Cellulose (2010); Chitnis et al., (2011)), or stamping (Domelas et al., (2015)). While channels formed on paper can readily direct multiple liquids to multiple reagents in a µPAD to perform multiplexed assays in parallel, spontaneous capillary flow in paper hinders execution of multi-step bioassays that require timely application of different reagents or buffers in multiple steps, such as immunoassay, enzyme-linked immunosorbent assay (ELISA) and sample purification (Fu et al., (2012); Toley et al., (2013); Lutz et al., (2013); Cheng et al., (2010)).

The lack of an intrinsic mechanism to control capillary-driven liquid flow remains a major bottleneck for LFAs. Recent approaches to control liquid flow in LFAs such as modified paper geometry (Fu et al., (2010); Apilux et al., (2013)), volume-limited source pad (Fu et al., (2012)), tunable-delay shunts (Toley et al., (2013)), sugar deposition (Lutz et al., (2013)), pressurized paper (Shin et al., (2014)), valving function via electromagnet (Li et al., (2013); Fratzl et al., (2018)), fluidic diodes (Chen et al., (2012)), and volume-metered actuation (Kong et al., (2017);Toley et al., (2015)) are highly effective but either require exotic and unscalable fabrication processes hindering their use in practice or large dead volumes limiting their use on manipulating small-volume samples. While useful for spontaneously wicking samples, the capillary flow inherently limits performing complex reactions that require timely application of multiple solutions.

The devices and methods disclosed herein address these and other needs.

### SUMMARY

In accordance with the purposes of the disclosed materials and methods, as embodied and broadly described herein, the disclosed subject matter, in one aspect, relates to a device and methods of using thereof.

In one aspect, provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a channel; and a third line of a delaminating ink extending between the first line and the second line disposed on the first substrate side.

In some embodiments, the device comprises a lateral flow assay.

In further embodiments, the non-delaminating ink comprises a hydrophobic resin.

In certain embodiments, the width of the third line is at least 0.1 mm.

In specific embodiments, the device further comprises at least two lines of the delaminating ink extending between the first line and the second line.

In some embodiments, a contact angle between the delaminating ink and the paper is from 0° to 180°.

In further embodiments, the first adhesive material is treated with corona discharge.

In certain embodiments, the device further comprises chemicals to modulate adhesion and/or hydrophobicity.

In specific embodiments, the first adhesive material, second adhesive material, and/or substrate are treated with biomolecules.

In some embodiments, the device further comprises a sample.

In further embodiments, the sample comprises urine, blood, plasma, serum, sweat, saliva, or any combination thereof.

In certain embodiments, the device further comprises a first mechanical force applied to the first substrate side and a second mechanical force applied to the second substrate side opposite the first mechanical force.

In specific embodiments, the third line of the delaminating ink is disposed between the first mechanical force and the second mechanical force.

In some embodiments, the first mechanical force and the second mechanical force are produced by two attracting magnets.

Also provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a channel; a third line of a delaminating ink extending between the first line and the second line disposed on the first substrate side; a fourth line of a delaminating ink extending between the first line and the second line disposed on the first substrate side; and a target antigen LFA strip disposed between at least a portion of the second adhesive material and at least a portion of the second substrate side.

In further embodiments, the target antigen comprises human chorionic gonadotropin.

In some embodiments, the device further comprises a case, wherein the case envelopes the first adhesive material, the substrate, the second adhesive material, and the target antigen LFA strip.

In further embodiments, the case comprises at least one sample inlet.

In certain embodiments, the case comprises at least one auxiliary inlet.

In specific embodiments, the case comprises at least one detection window.

In some embodiments, the device further comprises at least one signal amplification reagent, wherein the signal amplification reagent is deposited in the auxiliary inlet.

In further embodiments, the device further comprises at least one control line.

In certain embodiments, the device comprises a lateral flow assay.

In specific embodiments, the non-delaminating ink comprises hydrophobic resin.

In some embodiments, a contact angle between the delaminating ink and the substrate is from 0° to 180°.

In further embodiments, the first adhesive material is treated with corona discharge.

In certain embodiments, the device further comprises chemicals to modulate adhesion and/or hydrophobicity.

In specific embodiments, the first adhesive material, the second adhesive material, and/or the substrate are treated with biomolecules.

In some embodiments, the device further comprises a first mechanical force applied to the first substrate side and a second mechanical force applied to the second substrate side opposite the first mechanical force.

In further embodiments, the third line of the delaminating ink is disposed between the first mechanical force and the second mechanical force.

In certain embodiments, the fourth line of the delaminating ink is disposed between the first mechanical force and the second mechanical force.

In specific embodiments, the first mechanical force and the second mechanical force are produced by two attracting magnets.

In some embodiments, the width of the third line is at least 0.1 mm.

In further embodiments, the width of the fourth line is at least 0.1 mm.

Also provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a first channel; a third line of the non-delaminating ink and a fourth line of the non-delaminating ink disposed on the first-substrate side, wherein the third line and the fourth line define a second channel; and a fifth line of a delaminating ink extending between the third line and the fourth line disposed on the first substrate side.

In some embodiments, the device further comprises a sixth line of the non-delaminating ink and a seventh line of the non-delaminating ink disposed on the first-substrate side, wherein the sixth line and the seventh line define a third channel.

In further embodiments, the device further comprises an eighth line of the delaminating ink and a ninth line of the delaminating ink extending between the sixth line and the seventh line disposed on the first substrate side.

In certain embodiments, the device further comprises a tenth line of the delaminating ink extending between the sixth line and the seventh line disposed on the first substrate side.

In specific embodiments, the device further comprises at least one DNA extraction spot, wherein the DNA extraction spot is disposed on the first substrate side.

In some embodiments, the device further comprises Proteinase K, deionized water, and tris-EDTA buffer disposed on the first substrate side.

In further embodiments, the device further comprises at least one sample inlet.

In certain embodiments, the device further comprises at least one auxiliary inlet.

In specific embodiments, the fifth line is from 0.8 mm to 1.2 mm wide.

In some embodiments, the eighth line, ninth line, and tenth line are from 0.8 to 1.2 mm wide.

In further embodiments, the device comprises a lateral flow assay.

In certain embodiments, the non-delaminating ink comprises hydrophobic resin.

In specific embodiments, a contact angle between the delaminating ink and the substrate is from 0° to 180°.

In some embodiments, the adhesive material is treated with corona discharge.

In further embodiments, the device further comprises chemicals to modulate adhesion and/or hydrophobicity.

In certain embodiments, the first adhesive material, the second adhesive material, and or the substrate are treated with biomolecules.

In specific embodiments, the device further comprises a first mechanical force applied to the first substrate side and a second mechanical force applied to the second substrate side opposite the first mechanical force.

In some embodiments, the fifth line of the delaminating ink, eighth line of the delaminating ink, ninth line of the delaminating ink, tenth line of the delaminating ink, or any combination thereof, is disposed between the first mechanical force and the second mechanical force.

In further embodiments, the first mechanical force and the second mechanical force are produced by two attracting magnets.

Also provided herein is a device comprising a first adhesive material; a first substrate configured to move a fluid by capillary action, wherein the first substrate has a first substrate side and a second substrate side opposite the first substrate side, further wherein the first substrate side is coupled to at least a portion of the first adhesive material; a double-sided adhesive material, wherein the double-sided adhesive material has a first adhesive side and a second adhesive side opposite the first adhesive side, further wherein the first adhesive side is coupled to at least a portion of the second substrate side; a second substrate configured to move the fluid by capillary action, wherein the second substrate has a third substrate side and a fourth substrate side opposite the third substrate side, further wherein the third substrate side is coupled to at least a portion of the second adhesive side; a second adhesive material, wherein the second adhesive material is coupled to at least a portion of the fourth substrate side; at least DNA extraction spot disposed between the second substrate side and the first adhesive side; a first line of a non-delaminating ink and a second line of the non-delaminating ink disposed on the first substrate side, wherein the first line and the second line define a first channel; a third line of the non-delaminating ink and a fourth line of the non-delaminating ink disposed on the first substrate side, wherein the third line and the fourth line define a second channel; a fifth line of the non-delaminating ink and a sixth line of the non-delaminating ink disposed on the third substrate side, wherein the fifth line and the sixth line define a third channel; a seventh line of a delaminating ink extending between the third line and the fourth line disposed on the first substrate side; an eighth line of the delaminating ink extending between the fifth line and the sixth line disposed on the third substrate side; and a first primer mixture, a second primer mixture, and a third primer mixture disposed on the third substrate side.

In some embodiments, the first primer mixture targets SARS-CoV-2 virus.

In further embodiments, the first primer mixture comprises a polynucleotide having an amino acid identified by any one of SEQ ID NOS: 1-12.

In certain embodiments, the second primer mixture targets influenza A virus.

In specific embodiments, the second primer mixture comprises a polynucleotide having an amino acid identified by any one of SEQ ID NOS: 13-18.

In some embodiments, the third primer mixture targets influenza B virus.

In further embodiments, the third primer mixture comprises a polynucleotide having an amino acid identified by any one of SEQ ID NOS: 19-24.

In certain embodiments, the device further comprises Proteinase K and DI water disposed on the first substrate side.

In specific embodiments, the device further comprises RT-LAMP buffer disposed on the third substrate side.

In some embodiments, the device further comprises an internal heating element coupled to the second adhesive material opposite the fourth substrate side.

In further embodiments, the internal heating element comprises an exothermic reaction.

In certain embodiments, the exothermic reaction comprises an oxidation reaction.

In specific embodiments, the oxidation reaction comprises calcium oxide and water. In some embodiments, the device comprises a lateral flow assay.

In further embodiments, the non-delaminating ink comprises hydrophobic resin.

In certain embodiments, a contact angle between the delaminating ink and the substrate is from 0° to 180°.

In specific embodiments, the adhesive material is treated with corona discharge.

In some embodiments, the device further comprises chemicals to modulate adhesion and/or hydrophobicity.

In further embodiments, the first adhesive material, the second adhesive material, the first substrate, and/or the second substrate are treated with biomolecules.

In certain embodiments, the device further comprises a first mechanical force applied to the first substrate side and a second mechanical force applied to the second substrate side opposite the first mechanical force.

In specific embodiments, the seventh line of the delaminating ink, eighth line of the delaminating ink, or any combination thereof, is disposed between the first mechanical force and the second mechanical force.

In some embodiments, the first mechanical force and the second mechanical force are produced by two attracting magnets.

Also provided herein is a method of detecting a target material in a sample, comprising testing the sample on a device as disclosed herein.

In some embodiments, the method further comprises measuring a concentration of the target material.

In further embodiments, the target material comprises human chorionic gonadotropin.

In certain embodiments, the sample comprises urine.

Further provided herein is a method of purifying DNA, comprising depositing a sample on a device as disclosed herein.

In some embodiments, the DNA comprises human DNA.

In further embodiments, the sample is deposited on the DNA extraction spot.

Also provided herein is a method of detecting a SARS-CoV-2 virus, an influenza A virus, and/or an influenza B virus, comprising testing a sample on a device as disclosed herein.

In some embodiments, the sample comprises saliva.

The device disclosed herein can control capillary flow on paper by imprinting roadblocks on the flow path with water-insoluble ink and utilizing the gradual formation of a void between a wetted paper and a sheath polymer tape to create timers. Timers can be drawn at strategic nodes to hold the capillary flow for a desired period and thereby can enable multiple liquids to be introduced into multi-step chemical reactions following a programmed sequence. Disclosed herein is an LFA with built-in signal amplification to detect human chorionic gonadotropin (hCG) with an order of magnitude higher sensitivity than the conventional assay and a device to extract DNA from bodily fluids without relying on laboratory instruments.

Included herein is a scalable method and device to control capillary flow in LFAs by exploiting the gradual delamination of a wetted paper from a sheath polymer tape in producing controlled delays. Adhesive polymer tapes play a role in laminating the capillary flow layer to limit external interference, as well as isolating stacked layers for 3D flow routing. Herein, the capillary flow path on a laminated paper is blocked with a water-insoluble ink, which leaves the void to be formed at the interface of the ink-infused paper and the polymer tape as the only path for the flow to proceed. By modifying the imprint geometry, the time it takes for a void to form is set and effectively creates timers that hold capillary flow for a desired duration. Because the ink is specially formulated to infuse into and remain on paper, the timers can be constructed in a practical and reliable manner throughout an LFA. By imprinting these timers at strategic nodes, the device is programmed to coordinate different capillary flows, sequentially introduce different reagents into a reaction leaving optimal incubation times in between, and autonomously perform complex assays that could otherwise not be possible with conventional LFAs.

Additional advantages are set forth in part in the description that follows. The advantages described below are realized and attained by means of the elements and combinations particularly pointed out in the appended claims. It is to be understood that both the foregoing general description and the following detailed description are exemplary and explanatory only and are not restrictive.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

The accompanying figures, which are incorporated in and constitute a part of this specification, illustrate several aspects of the disclosure, and together with the description, serve to explain the principles of the disclosure.

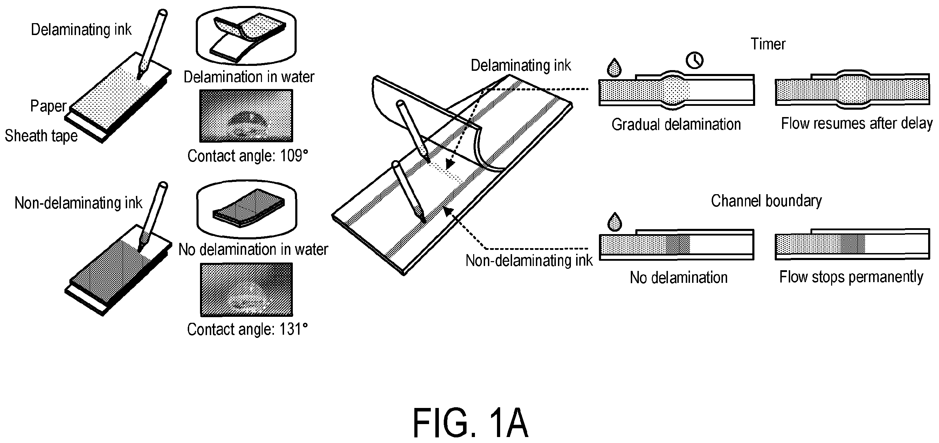

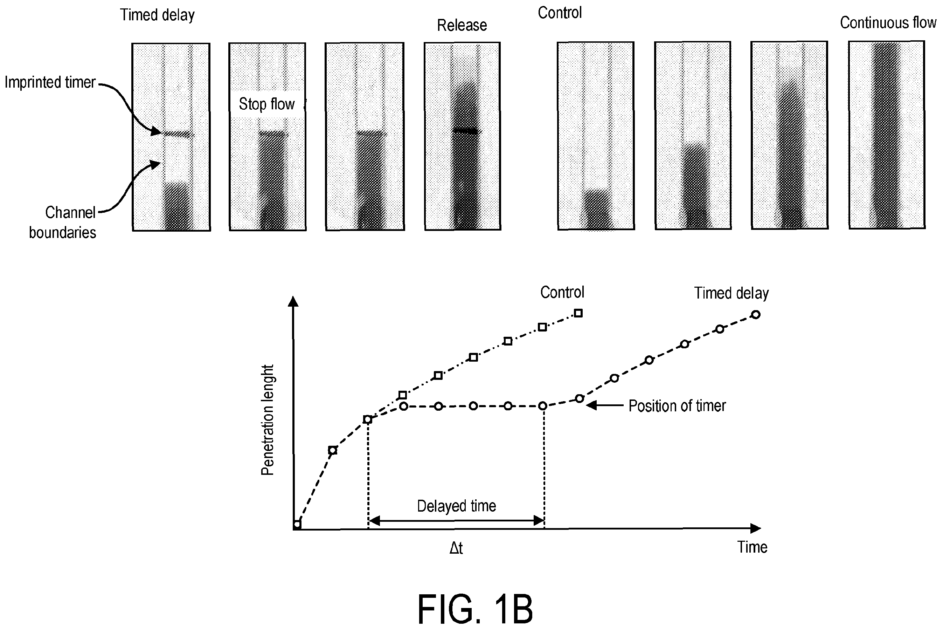

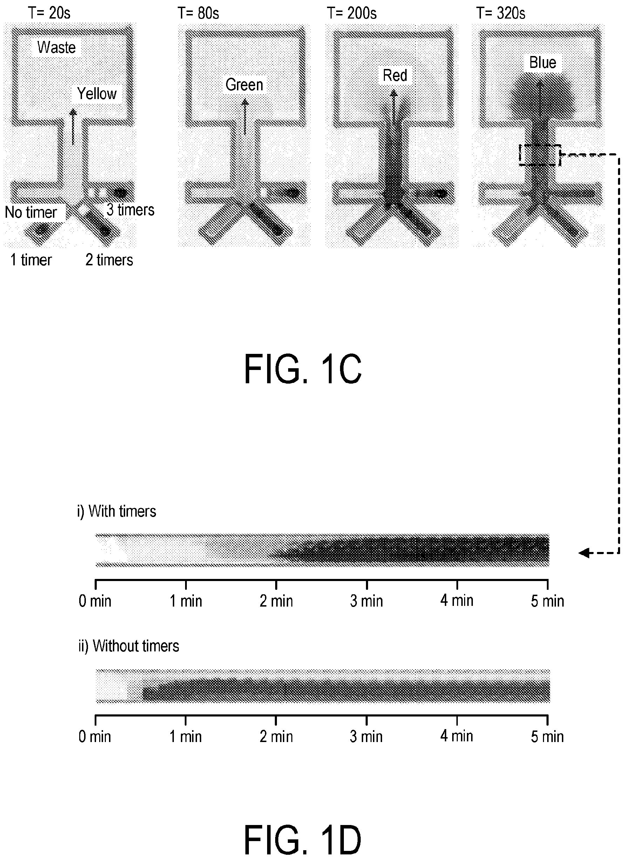

FIGS. 1A-1D show a schematic of capillary flow control via sheath tape delamination. FIG. 1A shows the manipulation of capillary flow on paper by imprinting patterns to modulate the paper-tape adhesion. The timer is drawn with a water insoluble ink that is free of hydrophobic resin (delaminating ink). When the paper is wetted, a void gradually forms at the paper-tape interface and eventually resumes the flow. The flow boundaries are defined with a water insoluble hydrophobic ink (non-delaminating). The resin remains attached to the tape and permanently blocks the flow. FIG. 1B shows time-lapse images of a dye solution progressing on paper with a timer (left) and without a timer (right). The timer is imprinted as a discrete line normal to the capillary flow. The plot shows the measured penetration distance as a function of delayed and control flow. The timer acted like a relay, temporarily stopping the flow and releasing it after a certain duration. FIG. 1C shows time-lapse images of a test paper platform with four dye solutions simultaneously introduced from different inlets. Different number of timers drawn in different inlet branches differentially delayed the capillary flows resulting in a sequential delivery. FIG. 1D shows change of observed color at the middle of the shared channel as a function of time with (top) and without timers (bottom). Discrete color transitions observed with timers demonstrate sequential routing of different capillary flows.

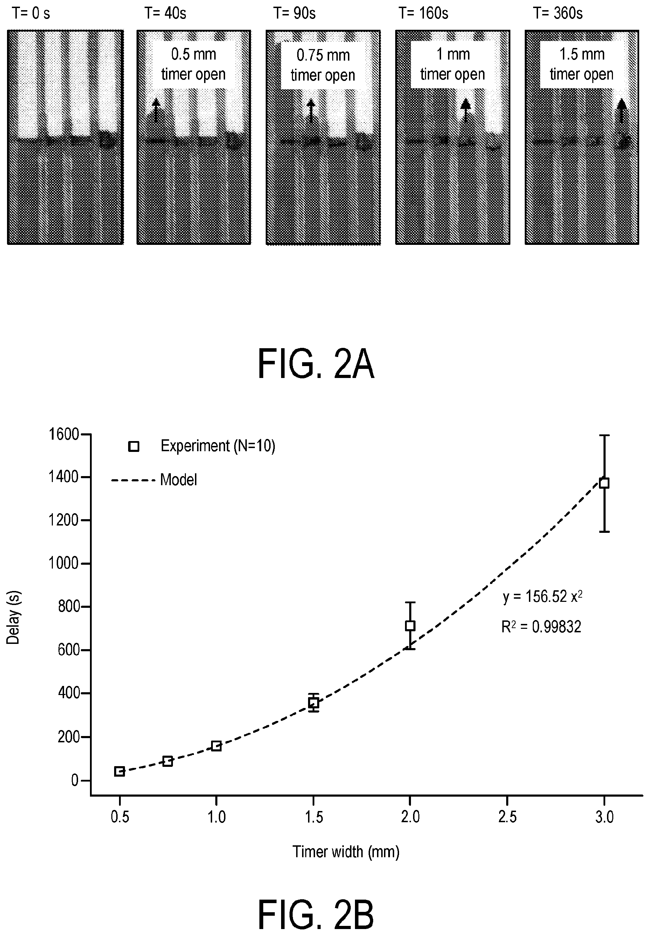

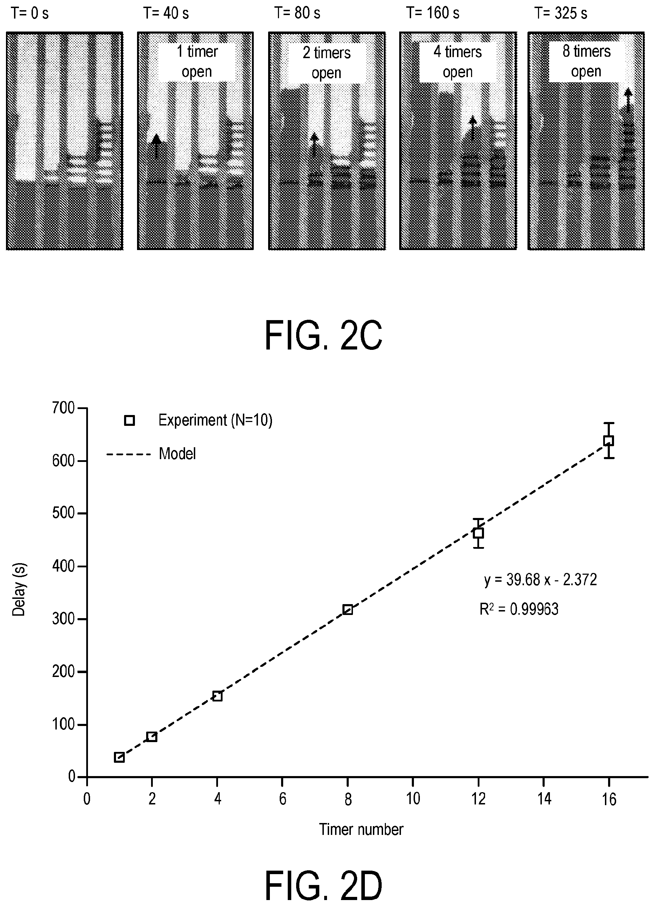

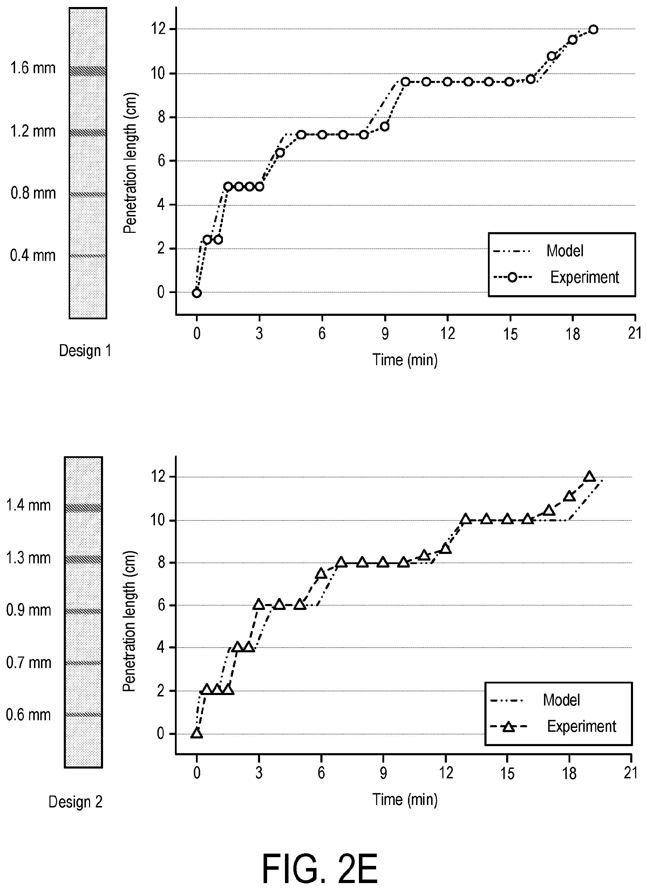

FIGS. 2A-2E show characterization and modeling of the timer delay. FIG. 2A shows time-lapse images taken of dye solution advancing on a paper test platform consisting of four lanes, each equipped with a timer of different width. The images show the capillary flow resuming after varying delays with wider timers resulting in longer delays. FIG. 2B shows measured flow delays generated by timers (N=10) as a function of their width. The measurements are overlaid with a quadratic regression model that was used to estimate delay when designing timers in this work. FIG. 2C shows time-lapse images taken of dye solution advancing on a paper test platform consisting of four lanes, each equipped with a different number of cascaded timers. The images show the capillary flow resuming after varying delays with more timers resulting in longer delays. FIG. 2D measured flow delays produced by different numbers of cascaded timers (1-16 timers) (N=10). The total delay in flow linearly increases with the number of timers on the flow path. FIG. 2E shows graphs showing measured versus model-predicted flow delays in two test designs, each equipped with multiple timers of varying widths. For the first design, four timers of 0.4, 0.8, 1.2, 1.6 mm widths were imprinted on a 12-cm long flow path at 2.4 cm intervals. For the second design, five timers of 0.6, 0.7, 0.9, 1.3, 1.4 mm widths were imprinted on a 12 cm-long channel with 2 cm intervals.

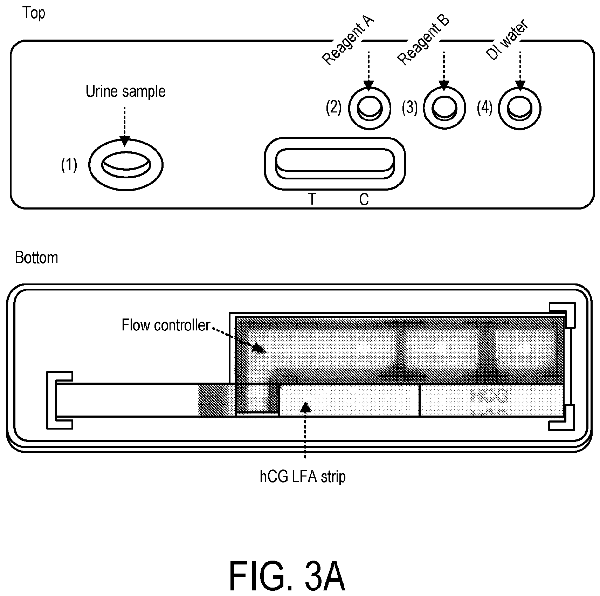

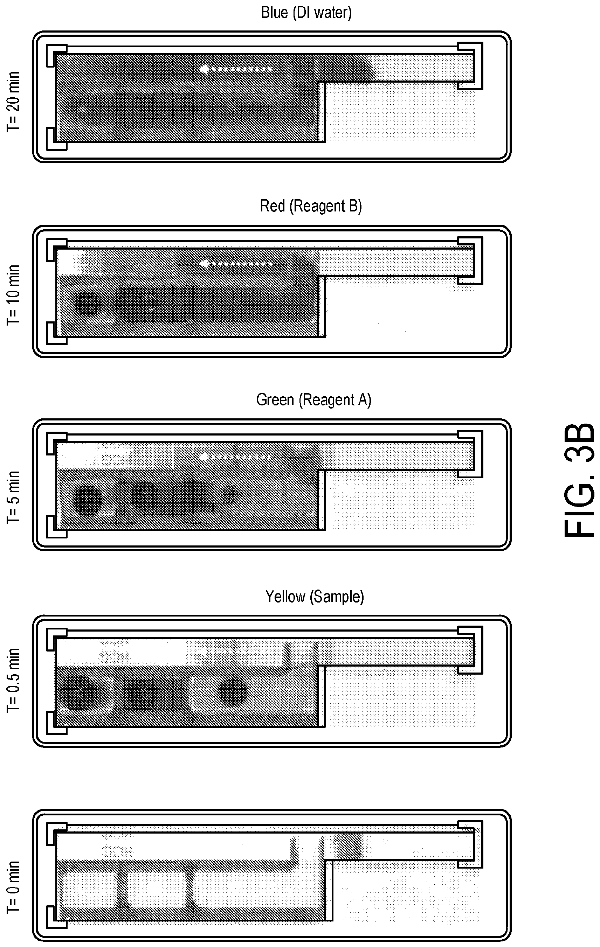

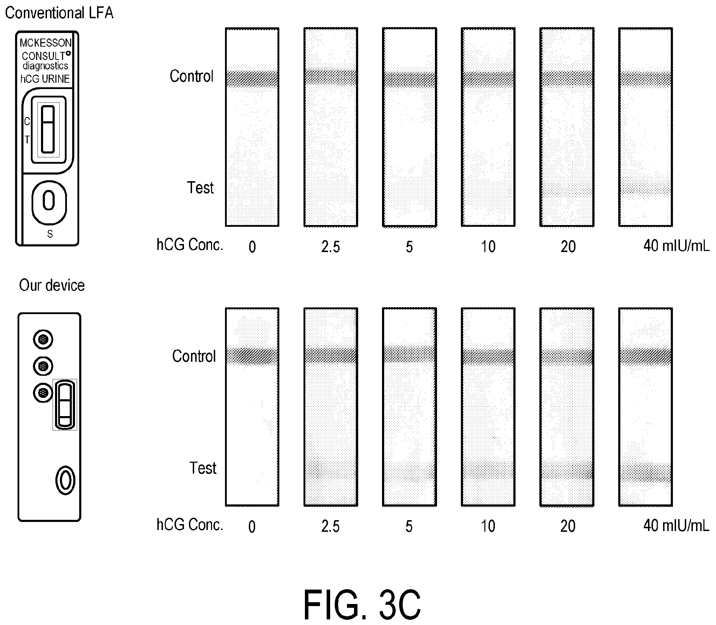

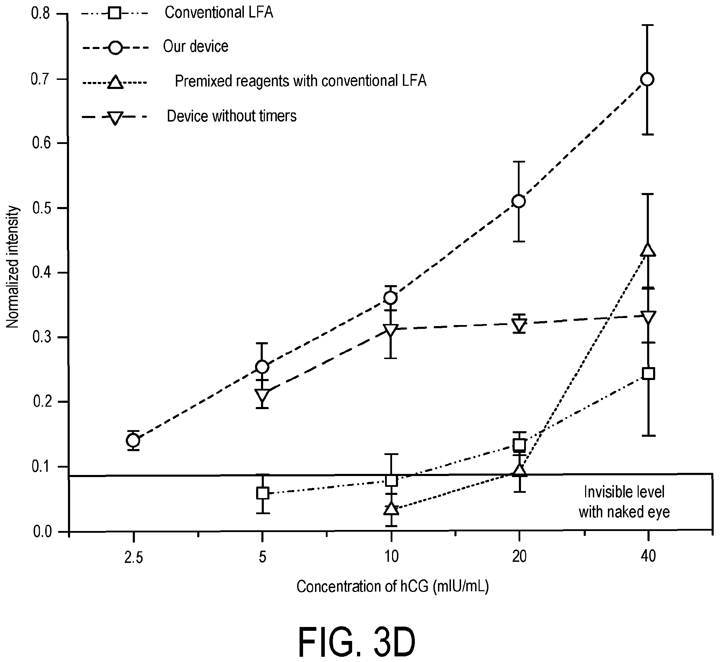

FIGS. 3A-3D show lateral flow assays with built-in signal amplification. FIG. 3A shows a photo of our device taken from the top and bottom. The device consists of a commercial hCG LFA strip integrated with a flow controller. The 3D-printed case has a sample inlet (1), three auxiliary inlets (2), (3), (4), and a detection window for observing test (T) and control (C) lines. In the flow controller, two timers (1 and 1.5 mm-wide) are placed in between three auxiliary inlets. In FIG. 3B, shows time-lapse images taken of the device when dye solutions of different colors, instead of actual solutions, were introduced from four inlets. The images confirm as-programmed sequential delivery sample (yellow), signal amplification reagent A (green), B 9red), and DI water (blue) to the test spot. FIG. 3C shows exemplary assay results corresponding to samples with different hCG concentrations obtained using the conventional LFA and our device. FIG. 3D shows plots showing the measured test line intensity as functions of the HCG concentration. In addition to our device and the conventional LFA from FIG. 3C, results are shown for two other measurement settings: Premixed reagents with conventional LFA results correspond to a urine sample premixed with two signal amplification reagents (A and B) and DI water, and processed using the conventional LFA. The device without timers corresponds to results obtained from a device that is identical to our device except that it lacks the timers to coordinate the flow between reagents.

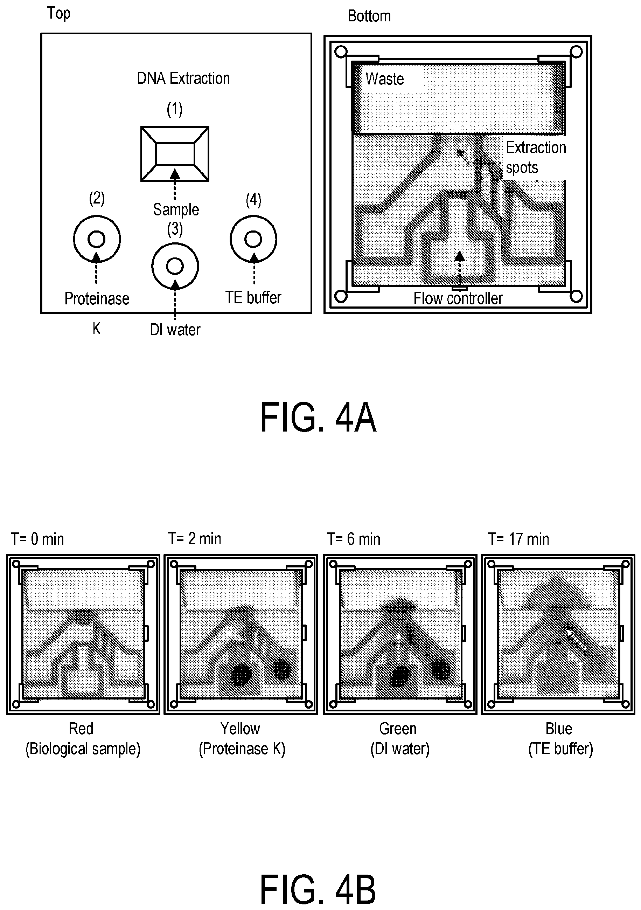

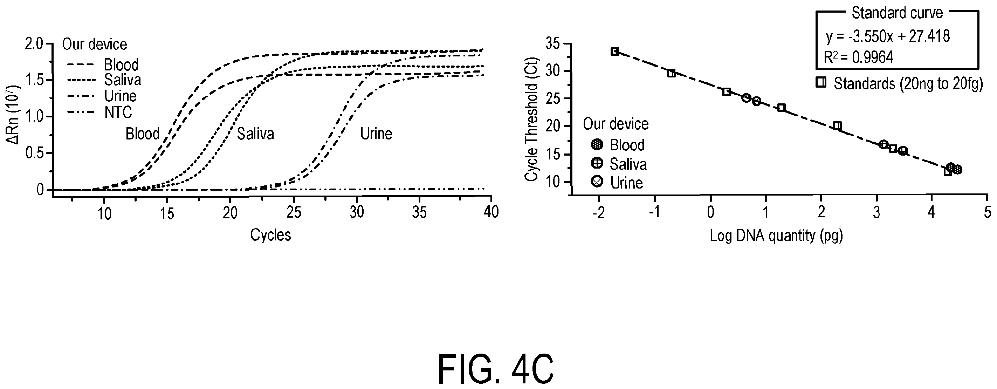

FIGS. 4A-4D show automated human DNA purification on paper. FIG. 4A shows a photo of the device taken from the top and bottom. The device is composed of three DNA extraction spots (punched out of an FTA Elute Card) integrated with a flow controller where a single timer and three timers, all 1 mm-wide, are placed at middle and right channels, respectively. The case for the assay has four openings: a sample inlet (1) and three auxiliary inlets (2), (3), and (4). FIG. 4B shows time-lapse images taken of the device when dye solutions of different colors, representative of actual solutions, were introduced from four inlets. The images confirm that once the sample (red) is loaded, dye solutions representing proteinase K (yellow), DI water (green), and TE buffer (blue) arrive at the extraction spots sequentially following the programmed order. Total process time for DNA purification with our assay is 20 minutes. FIG. 4C shows plots showing the qPCR results (left) and the corresponding cycle threshold (Ct) values (right) of DNA extracted from blood, saliva, and urine samples using the device disclosed herein. Fi. 4D shows the results of the same tests using a conventional FTA card.

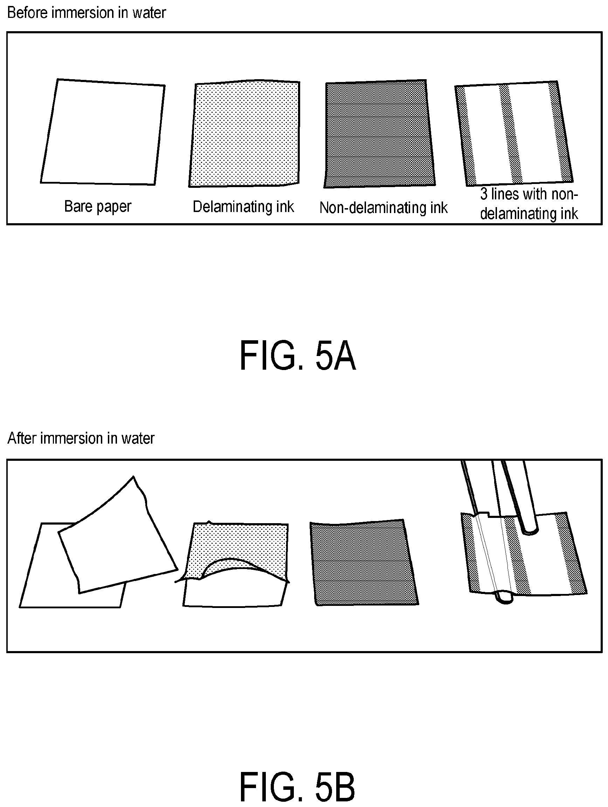

FIGS. 5A-5B show the delamination of laminated papers painted with the delaminating ink and the non-delaminating ink after immersion. FIG. 5B shows the state of exemplary laminated papers after they were immersed in water. The bare paper was fully delaminated from the sheath tape. The paper painted with the delaminating ink adhered to the sheath tape pre-immersion but delaminated when wetter. The paper painted with the non-delaminating ink was strongly tethered to the sheath tape even after immersion. For the paper on which three parallel lines were drawn with the non-delaminating ink, only the regions with no paint were delaminated while the lines still adhered to the sheath tape.

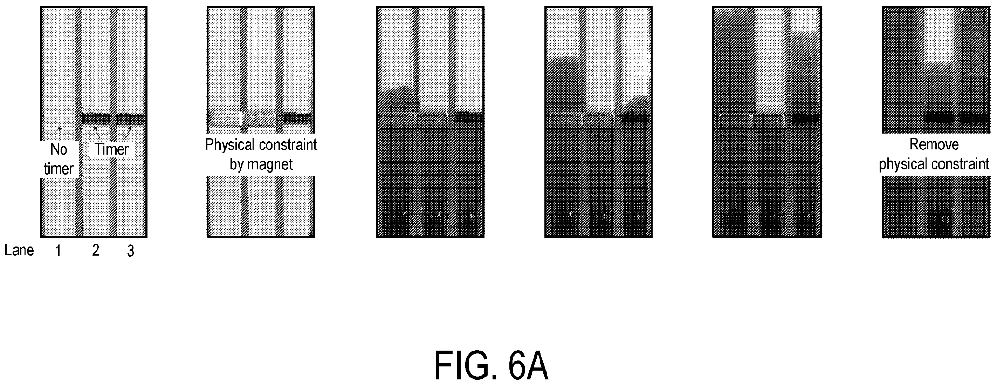

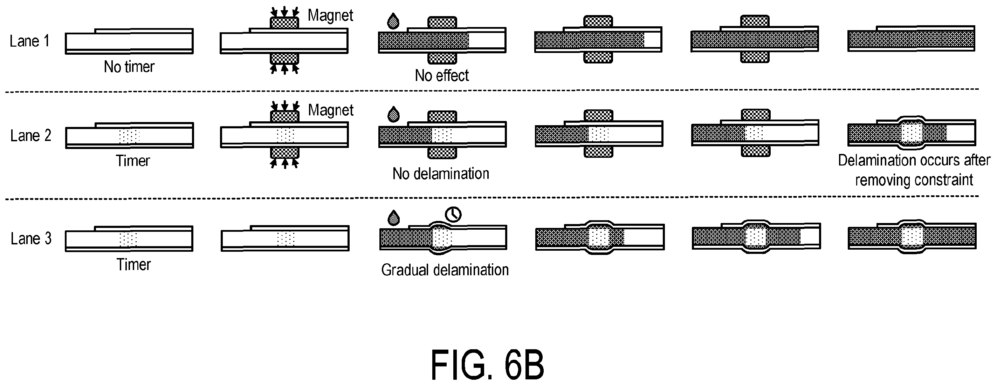

FIGS. 6A-6B show an exemplary investigation of the delay mechanism in delaminating timers. FIG. 6A shows photos taken at different time frames showing the state of three parallel lanes defined by non-delaminating ink on a laminated paper. FIG. 6B shows cross-sectional schematics illustrate the corresponding scenarios for each image. In the images, lane 1 is a control channel with no timer, while timers were drawn in lanes 2 and 3. Lanes 1 and 2 were first constrained by a mechanical force produced by two attracting magnets. The capillary flow in lane 1 was observed to be unaffected by the mechanical compression. In contrast, the capillary flow in lane 2 stopped permanently. The capillary flow in lane 3 was initially stopped by the timer and resumed after an intended amount of delay. The capillary flow in lane 2 resumed when the physical constraint was removed. Taken together, these results showed the physical separation (e.g., delamination) between the paper and the sheath tape as the mechanism for delaying the capillary flow.

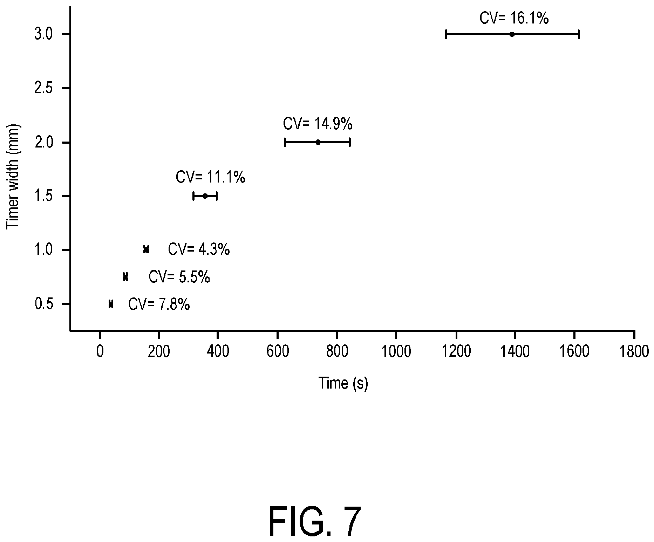

FIG. 7 shows a characterization of the variation of the time delay produced by delaminating. The time delays produced by timers were measured as a function of their width. The dots show the mean, the error bars represent the standard deviation (N=10), and CV represents the coefficient of variation.

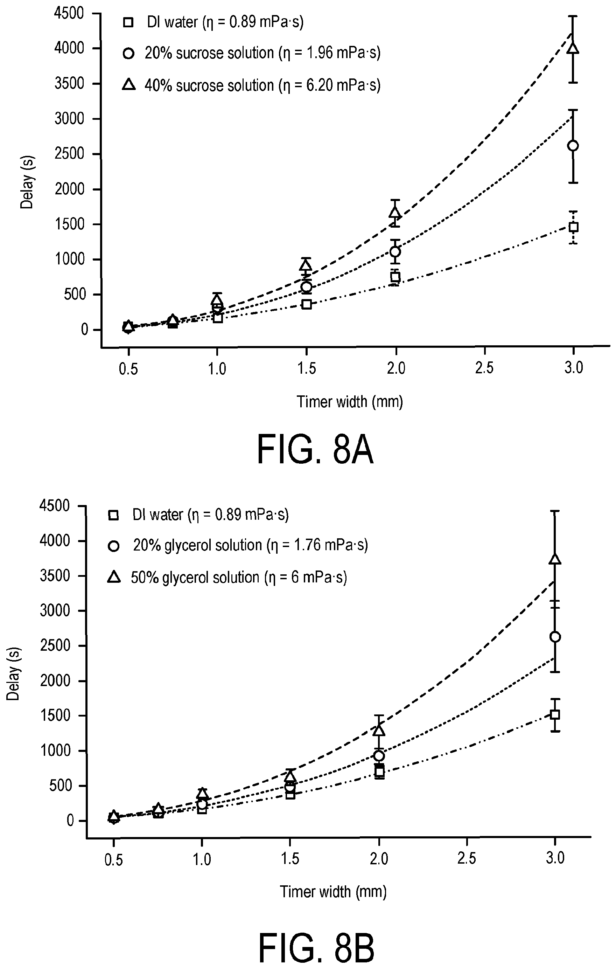

FIGS. 8A-8B show the effect of liquid viscosity on the timer delay. The plots show the mean measured timer delay as a function of the timer width in response to DI water with its viscosity manipulated by adding sucrose (FIG. 8A) and glycerol (FIG. 3B) at varying concentrations. The error bars represent standard deviation (N=10).

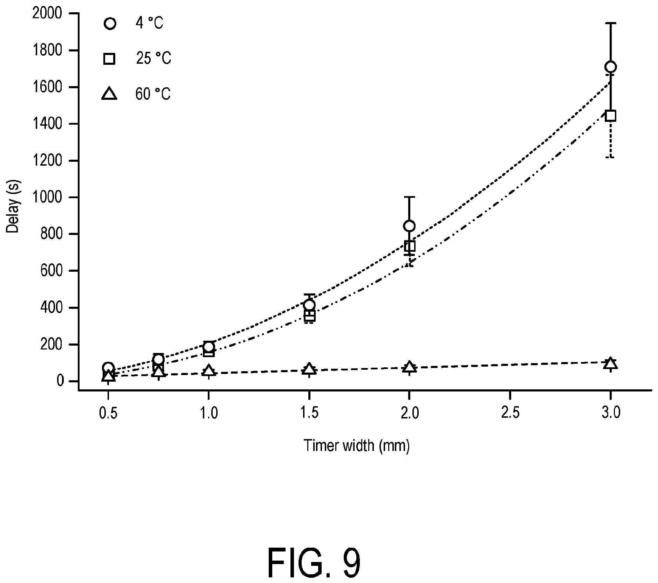

FIG. 9 shows the effect of temperature on the timer delay. The plots show the mean measured timer delay as a function of the timer width in response to DI water flow at three different temperatures: 4 °C, room temperature (25 °C), and 60 °C. The external temperature was controlled by a thermoelectric plate. The error bars represent standard deviation (N=10).

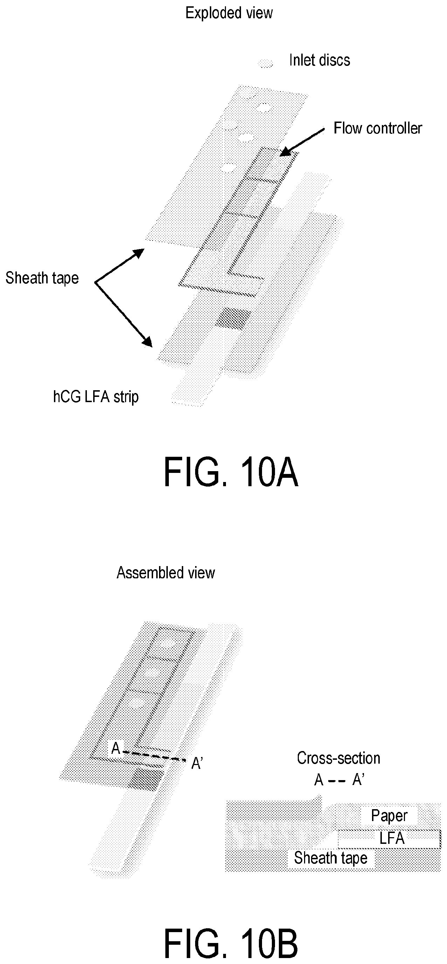

FIGS. 10A-10B show the integration of the hCG LFA strip with the flow controller. FIG. 10A shows a schematic of the exploded view of the designed device. FIG. 10B shows the assembled view of the designed device. The flow controller and commercial LFA strip were aligned and integrated on the same sheath tape at the bottom. Another sheath tape covered the flow controller from the top. To prevent any potential interference with the operation and colorimetric detection, the LFA strip was left exposed from the top with neither the top sheath tape nor the paper substrate extending over the LFA strip except at the junction point that delivers the reagents A/B and DI water from the flow controller to the LFA strip.

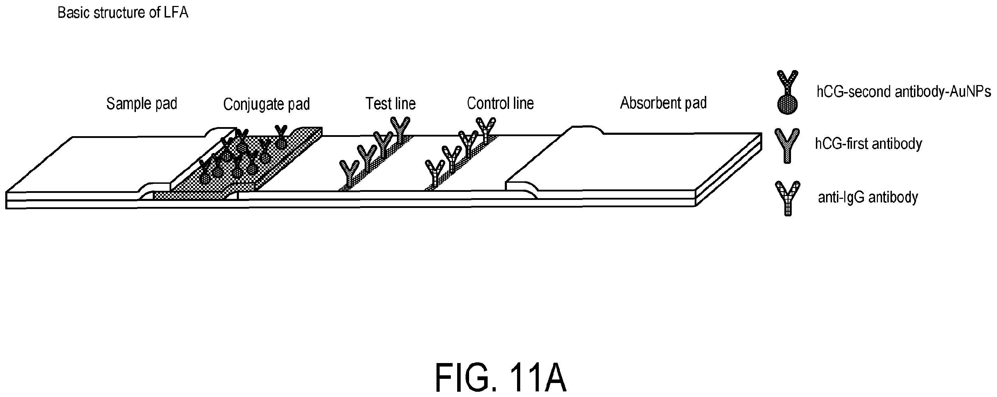

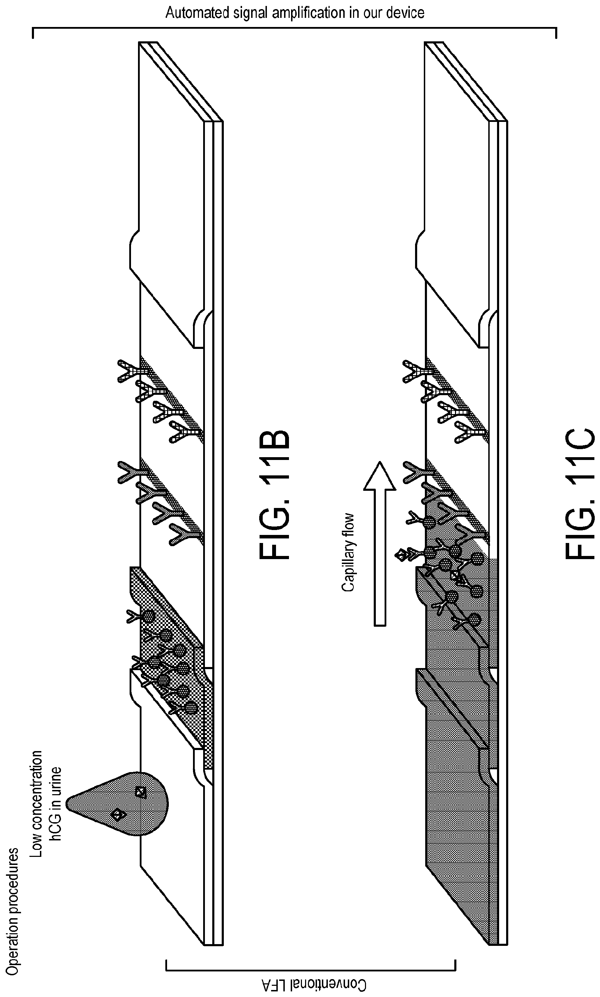

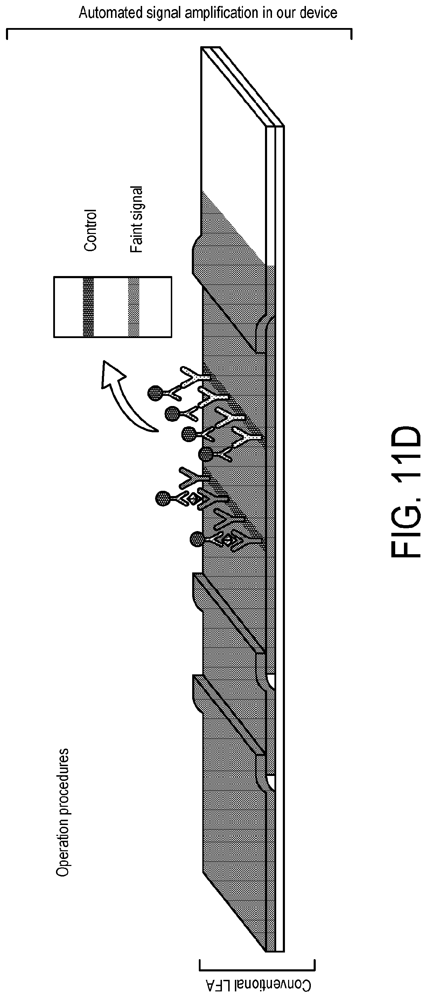

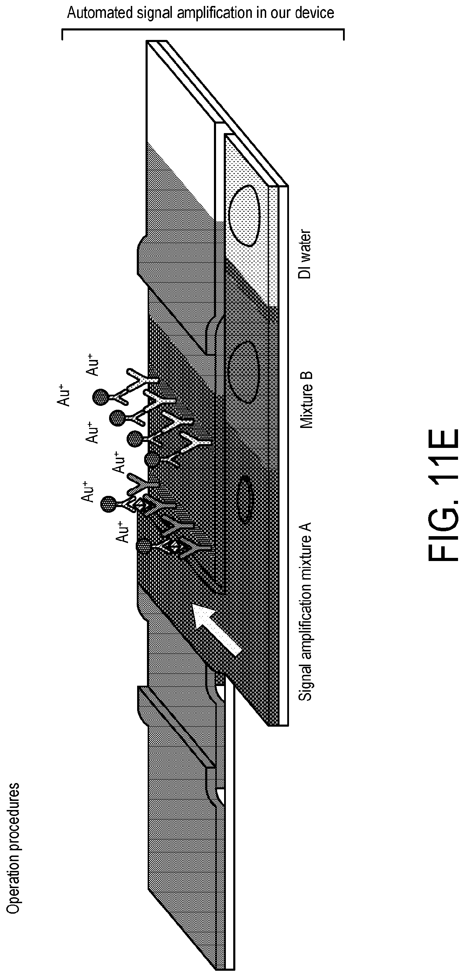

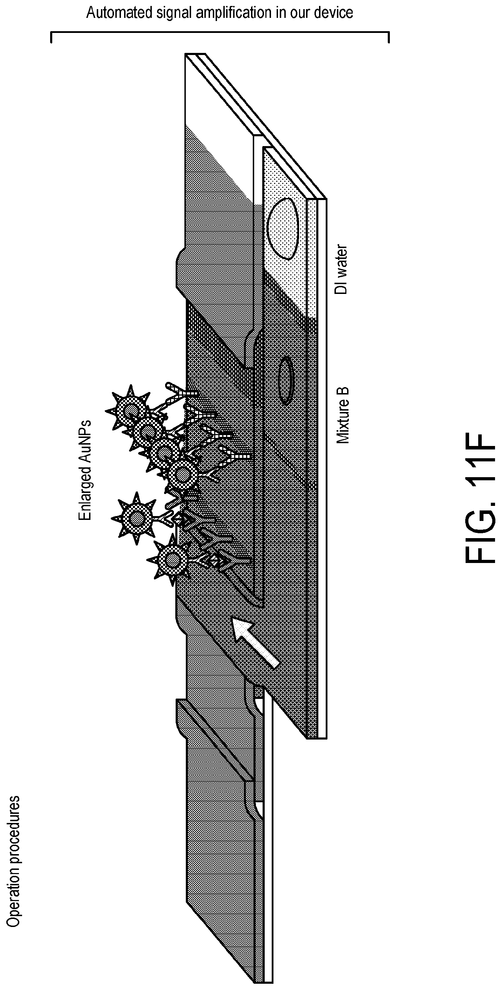

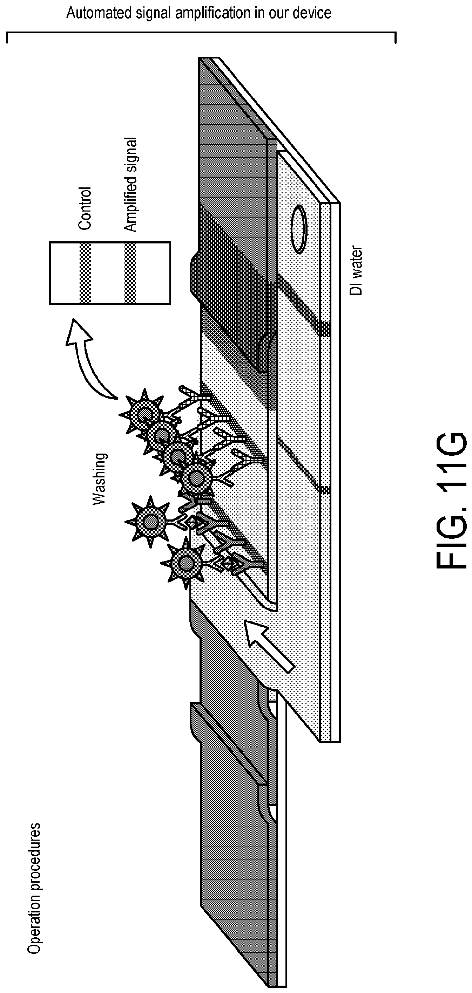

FIGS. 11A-11G show exemplary operation of the LFA strip with the flow controller. FIG. 11A show the basic structure of the conventional LFA indicating the types and placements of bio-recognition elements on the strip. The schematics of FIGS. 11B-11G show the full sequence of events that produce an amplified colorimetric signal in the device disclosed herein. Among these schematics, FIGS. 11B-11D are common with a conventional LFA strip. Schematics in FIGS. 11E-11G illustrate the automated signal amplification process through sequential delivery of the chemical reagents by the integrated flow controller.

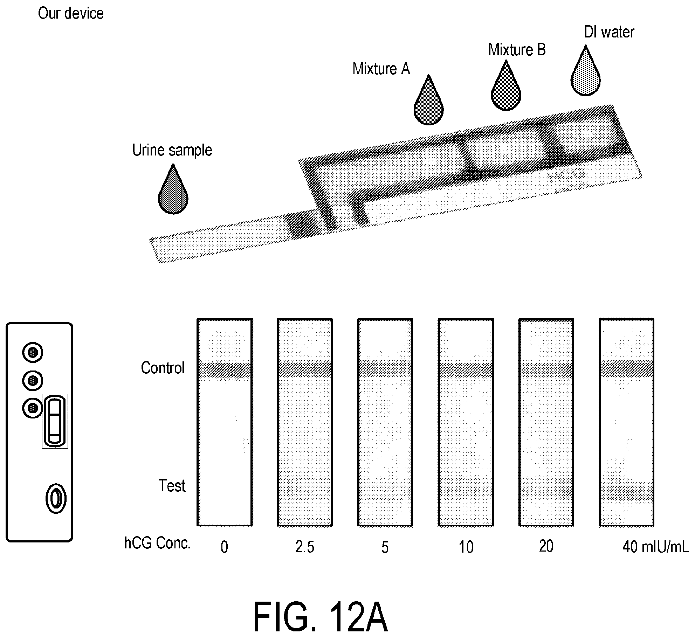

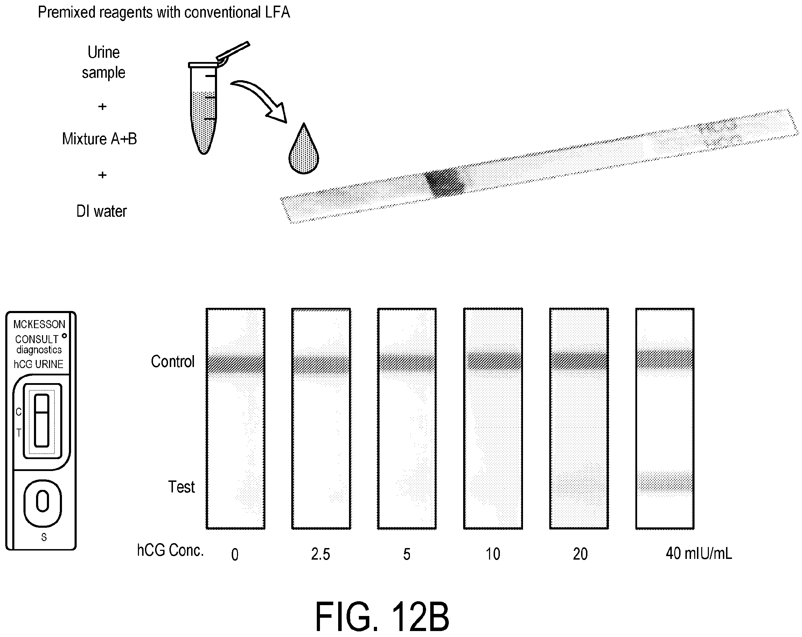

FIGS. 12A-12B show a comparison between hCG assay on the device disclosed herein and processing a sample premixed with amplification reagents on a conventional LFA. The schematics in FIGS. 12A and 12B illustrate the experimental procedures employed to process the urine samples. The urine samples were processed directly on the device of FIG. 12A, while the samples were premixed with amplification reagents for the conventional LFA in FIG. 12B. The images in the figures show the colorimetric results recorded from the two experiments for different concentrations of hCG in urine samples. The test line being visible on the device in FIG. 12A demonstrates that it is not the presence of amplification reagents, but their sequential delivery is responsible for higher sensitivity.

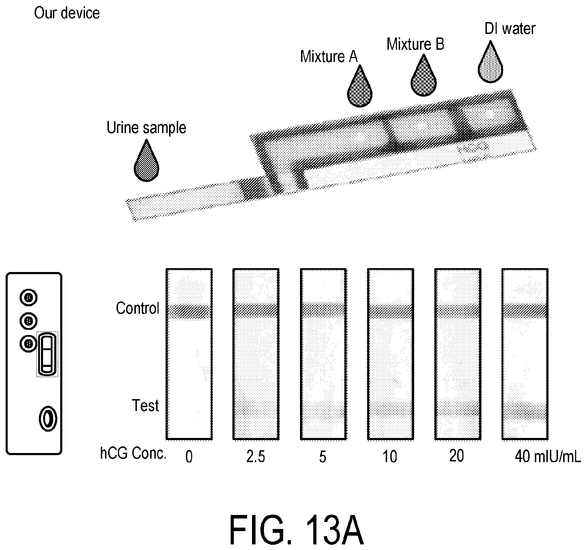

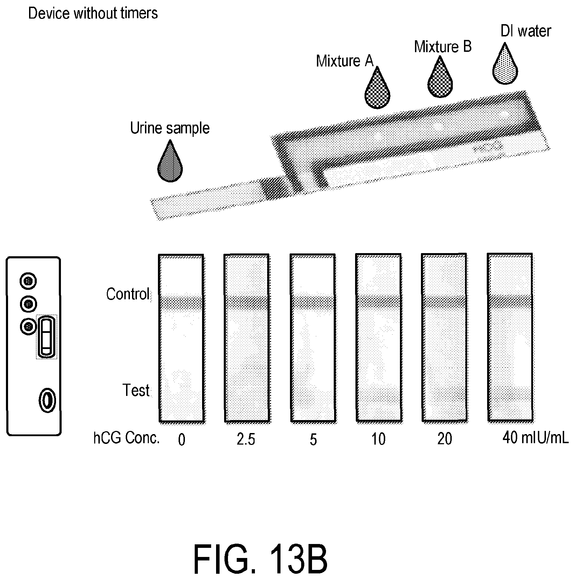

FIGS. 13A-13B show a comparison between hCG assay on an exemplary version of the device disclosed herein and an identical device without delaminating timers. The schematics in FIGS. 13A-13B illustrate the experimental procedures employed to process the urine samples. The only difference between the two devices is that the device on the right does not have the delaminating timers. The images in FIGS. 13A-13B show the colorimetric results recorded from the two experiments for different concentration of hCG in urine samples. The test line being visible on the device of FIG. 13B for a lower hCG concentration demonstrates that the specific time delays produced by delaminating timers are required to increase the assay sensitivity.

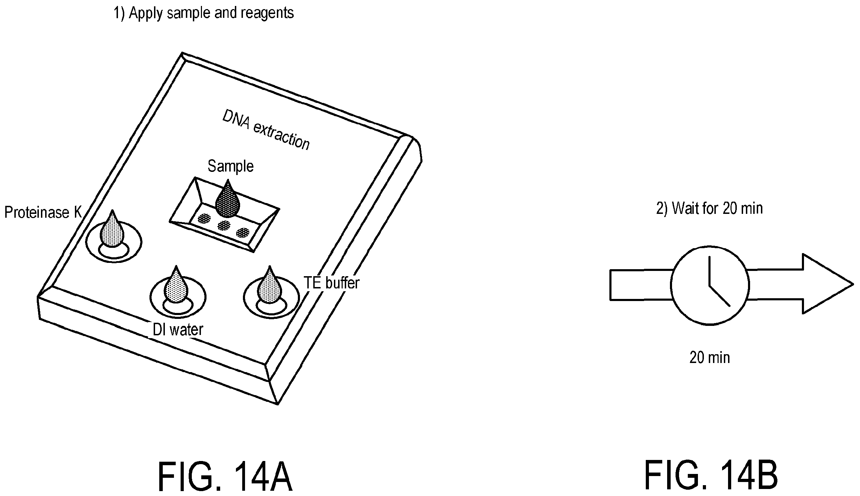

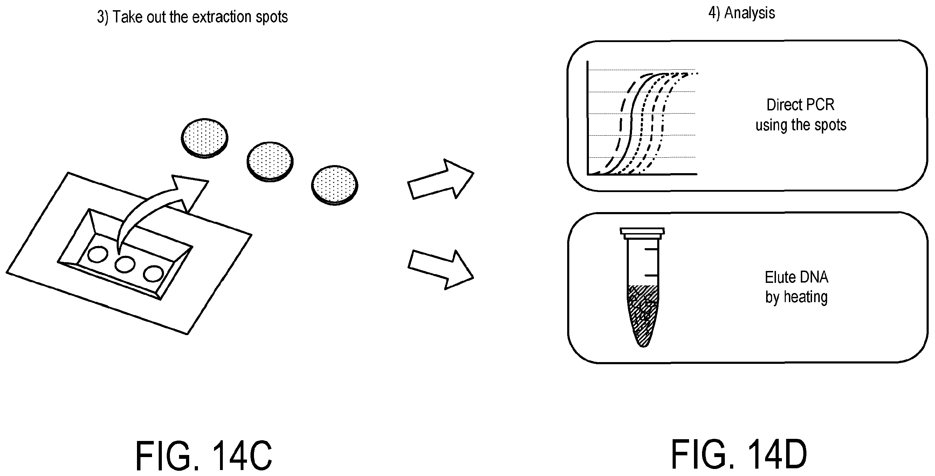

FIGS. 14A-14D show DNA extraction on an exemplary version of the device disclosed herein. The schematic illustrated the steps in the designed protocol. The biological sample is introduced to the device simultaneously with three other reagents from dedicated inlets, as shown in FIG. 14A. After 20 minutes, (FIG. 14B), the extraction spots are taken out from the device, as in FIG. 14C, and subjected to PCR for amplification of the extracted DNA, as in FIG. 14D. The purified DNA can also be obtained in suspension by immersing the extraction spots in TE or elution buffer and heating at 80°C for 30 minutes.

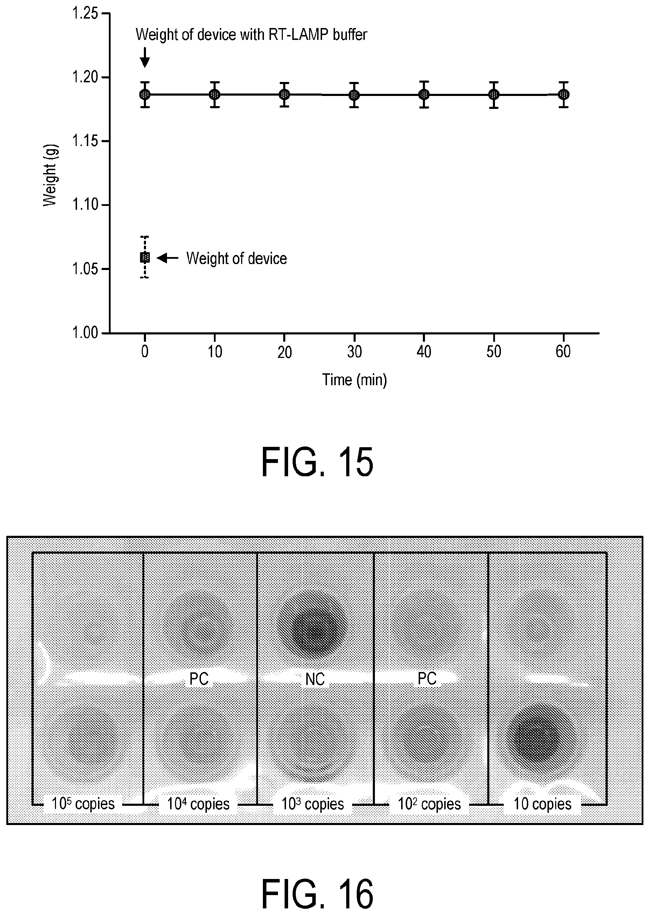

FIG. 15 shows the testing of the covered assay for possible evaporation of reagents during the amplification process because of the internally generated heat. The plot shows the measured weight of the device containing the RT-LAMP buffer (red) and the weight of the device without the RT-LAMP buffer (black). No noticeable changes were observed in the amount of the buffer (by weight) at different timepoints until 1 hour.
FIG. 16 shows functional testing of an exemplary IH module. The image shows the results from an RT-LAMP reaction performed in a well plate placed on top of the IH module. Spiked virus RNA (>100 copies) in saliva samples along with the PC were all fond to produce colorimetric signals confirming successful amplification via the heat generated by the IH module.

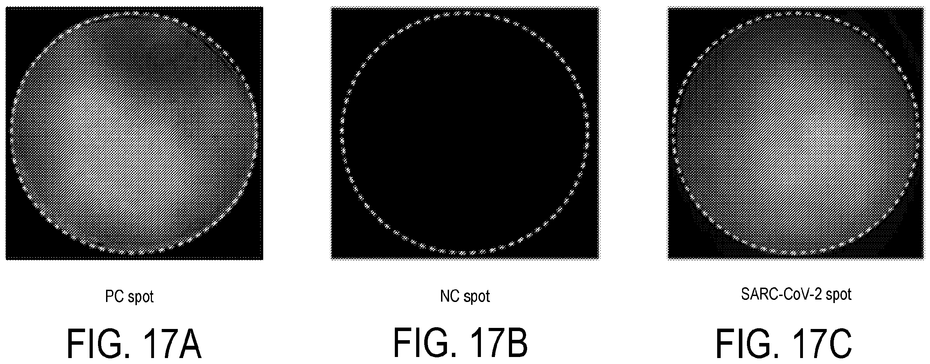

FIGS. 17A-17C show testing of the RNA amplification reaction with fluorescence measurements. The fluorescence microscope images show the PC, NC, and SARS-CoV-2 spots at the end of an amplification process that employed a fluorescent intercalating dye (1 µL of PicoGreen).

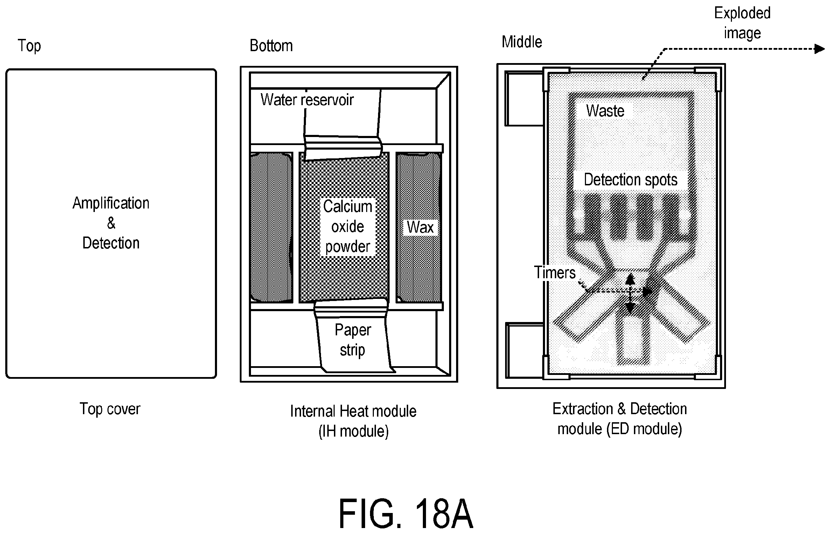

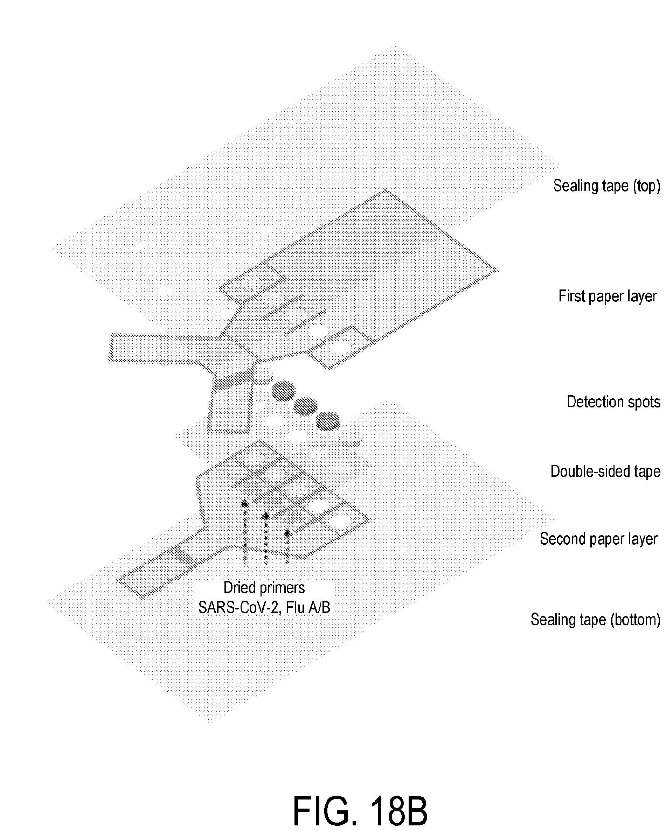

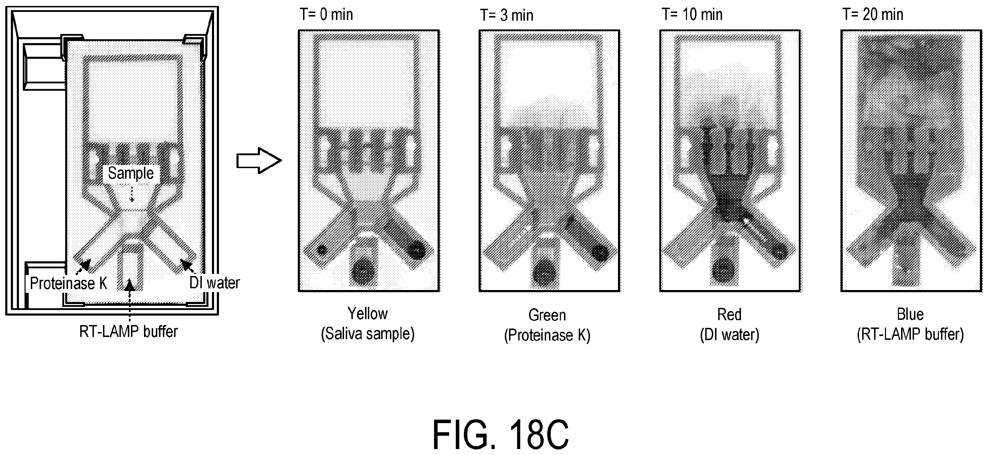

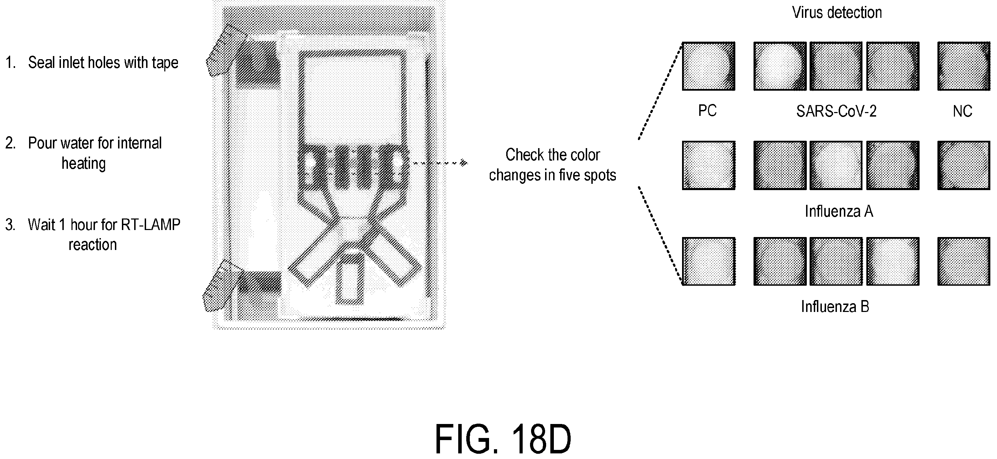

FIGS. 18A-18D show the design of an exemplary assay and its operation. FIG. 18A includes photos showing components of the developed POC toolkit. The top cover is a plastic cover for the thermal isolation of the running assay. The internal heat module (IH module) consists of several reservoirs filled with CaO powder, water, and wax. A cellulose paper strip is placed between the CaO and water reservoirs to carry the water to the CaO reservoir. The extraction & detection (ED module) is a multi-layered paper platform. The flow paths for manipulating different reagents are defined by imprinting on the paper with a water-insoluble ink (brown). Timers were imprinted (green) on the flow paths to introduce controlled amounts of delay to the flow of different reagents. FIG. 18B shows an exploded image of the ED module showing individual layers. Two paper layers imprinted with features were laminated in between polymer tapes and were coupled through the detection spots punch out of filter paper pretreated with chemical s for RNA extraction. The primer mixtures specifically targeting SARS-CoV-2, influenza A and B viruses were dried in front of the detection spots to be carried by the RT-LAMP buffer flow on the second paper layer. FIG. 18C shows time-lapse images of the ED module showing its capability to coordinate the delivery of four dye solution simultaneously loaded into the module. The dye solutions were used instead of the actual sample and reagents for visual investigation. The images show the state of the module at selected timepoints: the delivery of saliva sample (yellow), proteinase K (green), DI water (red), and RT-LAMP buffer (blue) to the detection spots. FIG. 18D shows a schematic showing the procedure to operate the developed assay. The presence of SARS-CoV-2, influenza A and B viruses in the processed samples can be visually identified by the color changes in the corresponding detection spot.

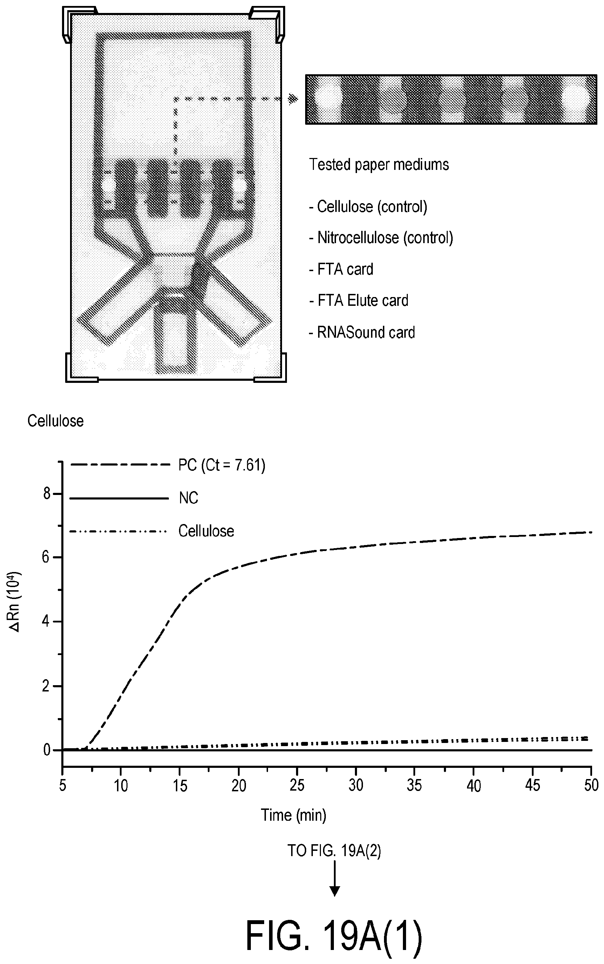

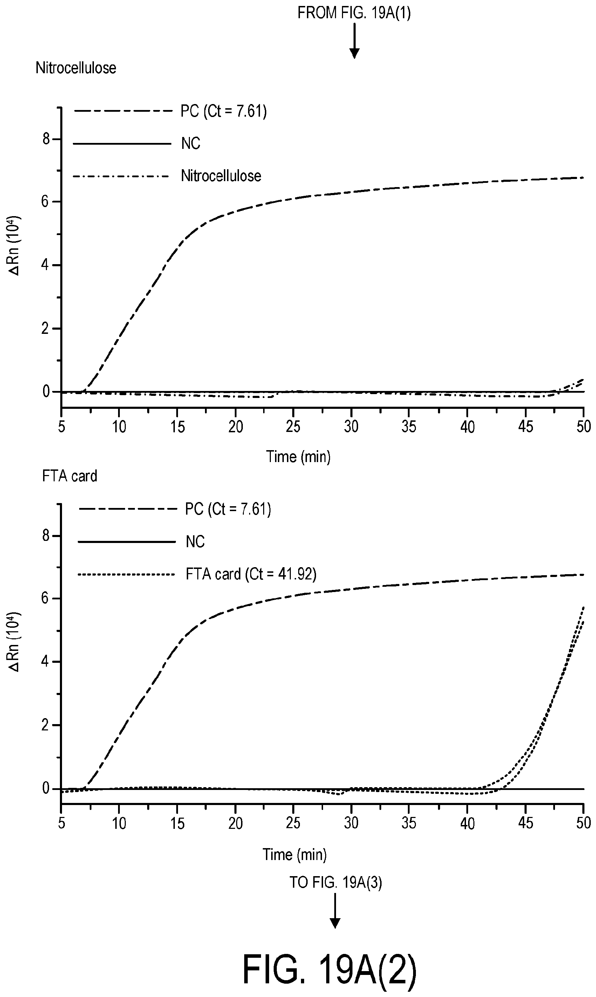

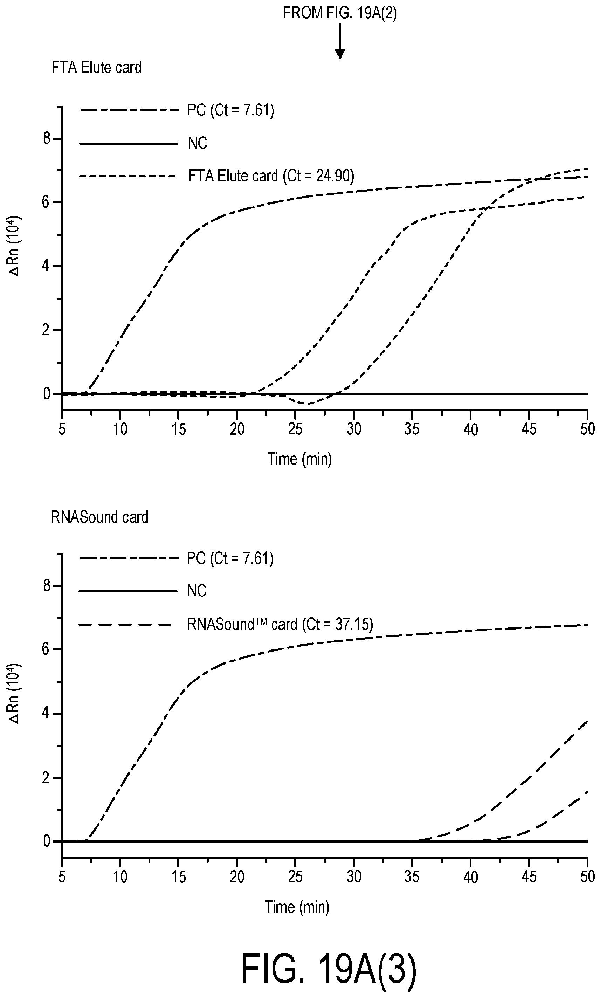

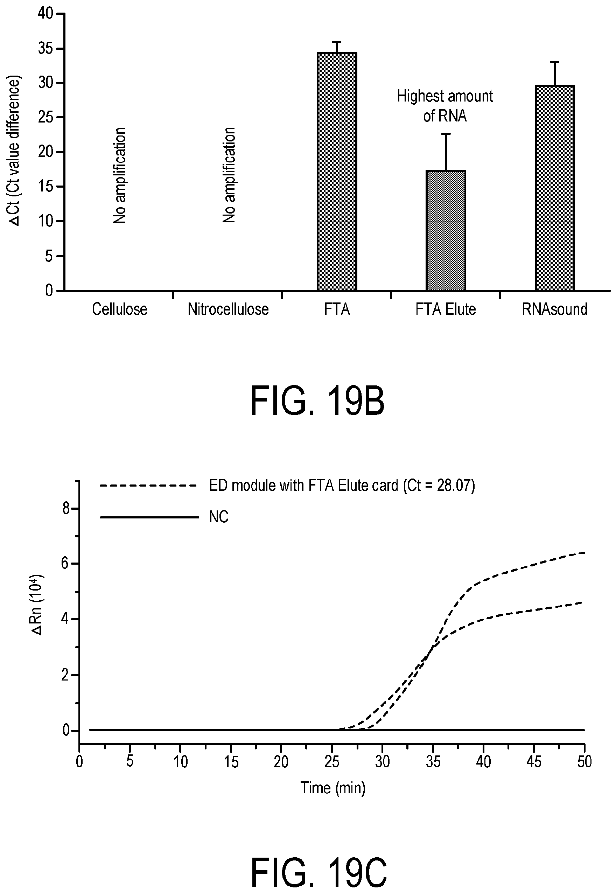

FIGS. 19A-19C show the characterization and optimization of viral RNA extraction. FIG. 19A shows the amplification plots from the real-time RT-LAMP of the extracted SARS-CoV-2 on five different filter papers: cellulose and nitrocellulose paper as controls, and three commercially available nucleic acid sampling papers. No amplification was observed on the cellulose and nitrocellulose papers. Amplification occurred on the FTA, FTA Elute, and *RNASound* cards with different Ct values. For each amplification, measurements were performed in pairs to ensure against artifacts. FIG. 19B shows the measured mean Ct value differences (ΔCt) for different papers tested. The error bars represent the standard deviation (n=3). FIG. 19C shows the measured amplification curve when the SARS-CoV-2 RNA was autonomously extracted by an ED module equipped with detection spots made out of the FTA Elute card and was subsequently subjected to real-time RT-LAMP outside of the device.

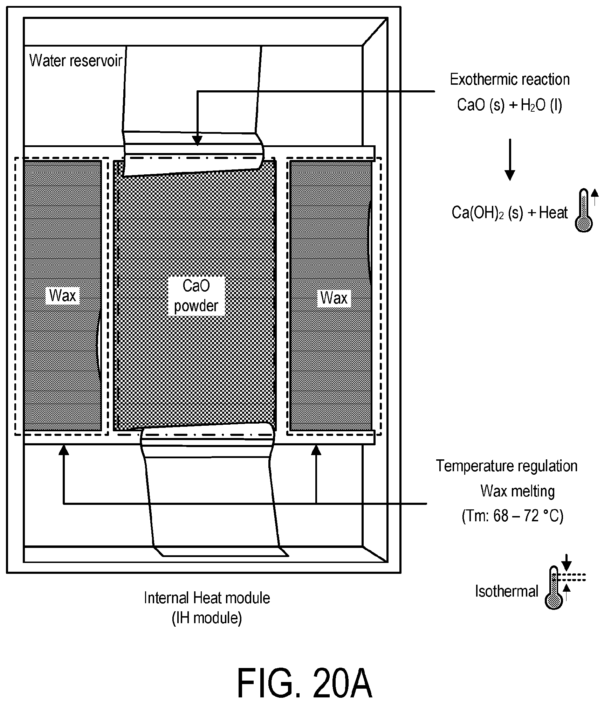

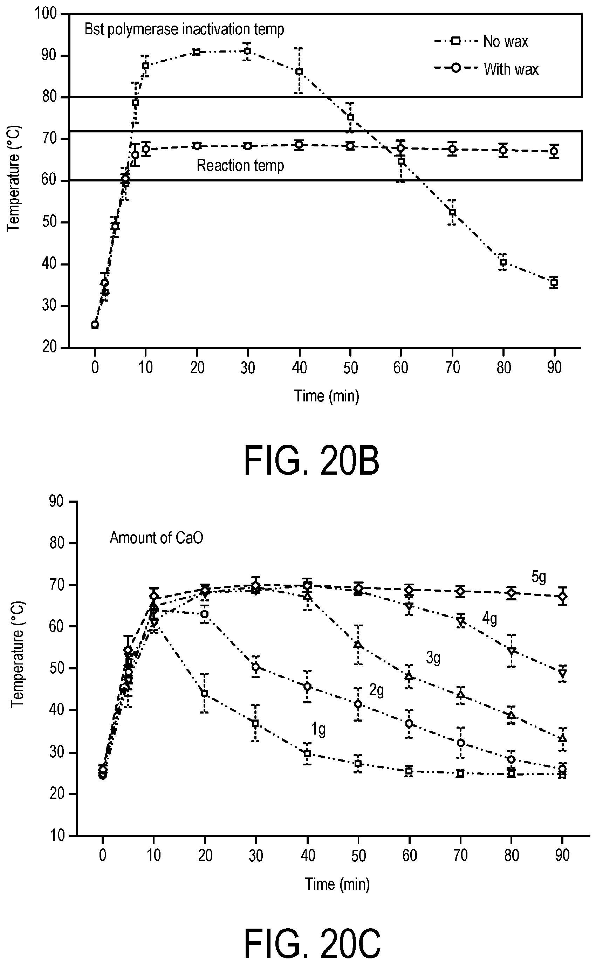

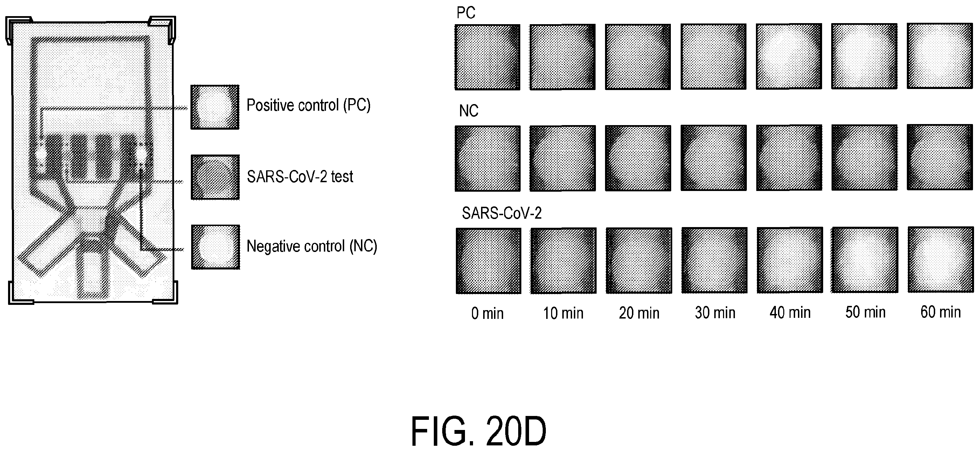

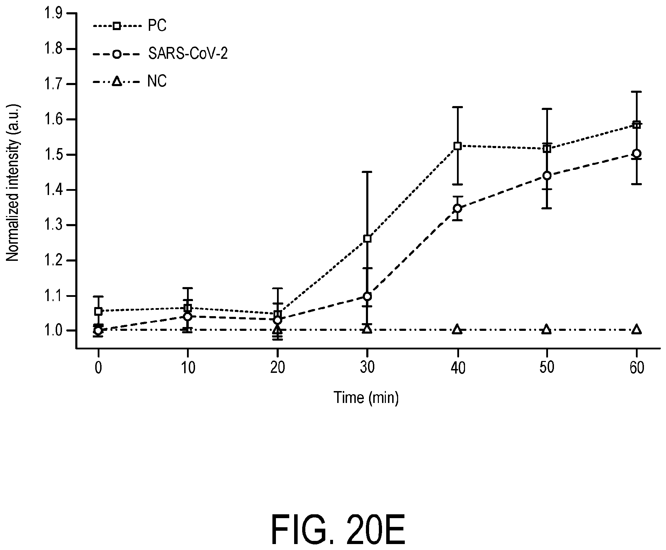

FIGS. 20A-20E show the autonomous RNA amplification and colorimetric detection. FIG. 20A shows a schematic of the function of individual components of the IH module. The exothermic reaction of CaO with water produces heat and the melting of the wax regulates the temperature of the device to the desired range for the RNA amplification. FIG. 20B shows the measured temperature of the device with (red) and without (black) the wax following the onset of the exothermic reaction. The melting of the wax regulates the device temperature at approximately 68 - 72 °C and prevented a temperature spike that would inactivate the *Bst* polymerase. FIG. 20C shows a measured device temperature loaded with different amounts of CaO. Five grams of CaO could maintain an isothermal environment at approximately 68 °C for > 90 mins. FIG. 20D shows images of the detection spots taken at different timepoints showing the changes in the spot colors as the reaction progresses. The images show the color on PC and SARS-CoV-2 spot changing from pink to yellow after 40 mins, while the color on NC spot remains pink for the whole tested duration. FIG. 20E shows the measured color intensity of the PC, NC, and SARS-CoV-2 spots as functions of the reaction time. Error bars in all panels represent the standard deviation (n=3).

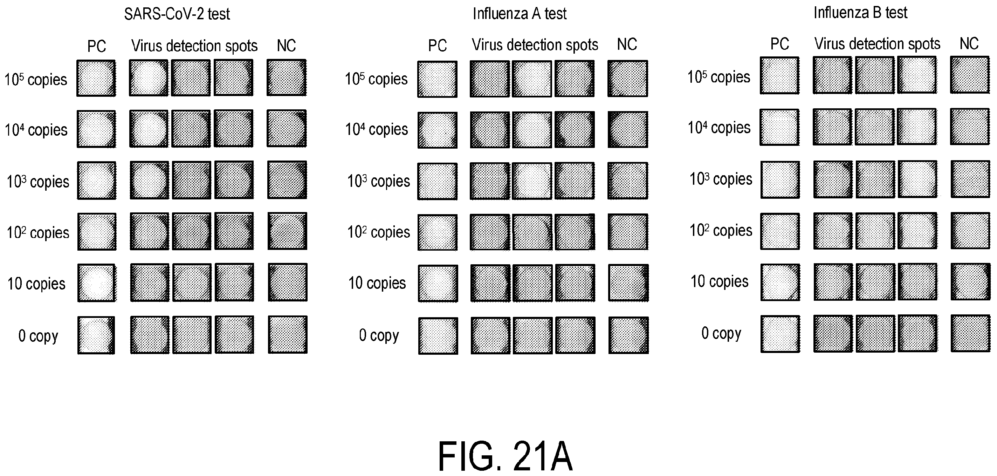

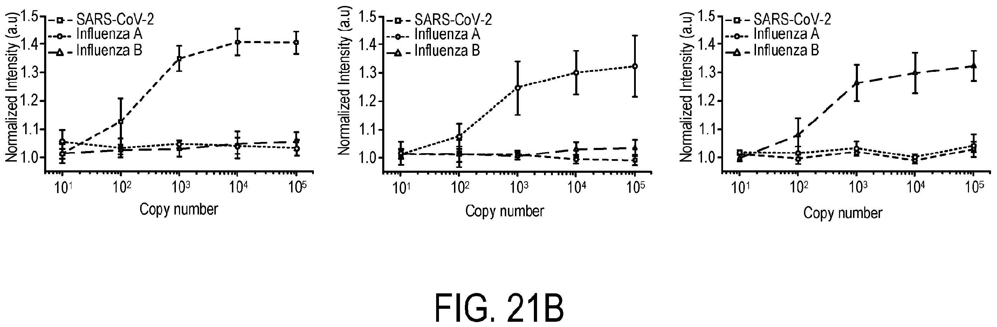

FIGS. 21A-21B show multiplexed detection of SARS-CoV-2, influenza A and B viruses. FIG. 21A shows the assay results corresponding to the saliva samples spiked with different copy numbers of SARS-CoV-2, influenza A, and influenza B viruses. Among the three central detection spots, , the left one tests for the presence of SARS-CoV-2, the middle one tests for influenza A and the right spot tests for the influenza B virus. Change in the color of a spot indicates a positive result for the targeted virus. FIG. 21B shows the plots showing the measured color intensities for each test as functions of virus copy number. Error bars represent the standard deviation (n=3).

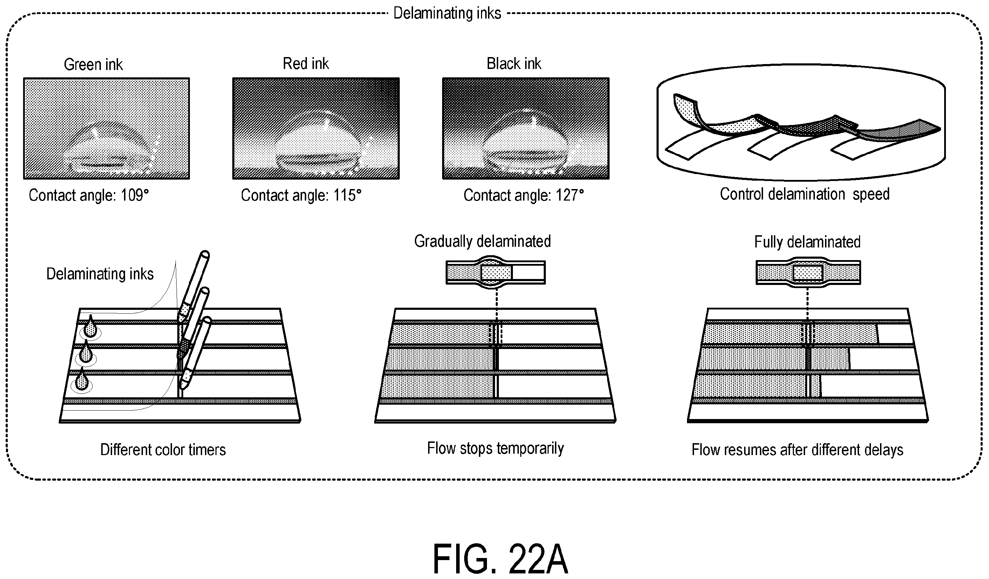

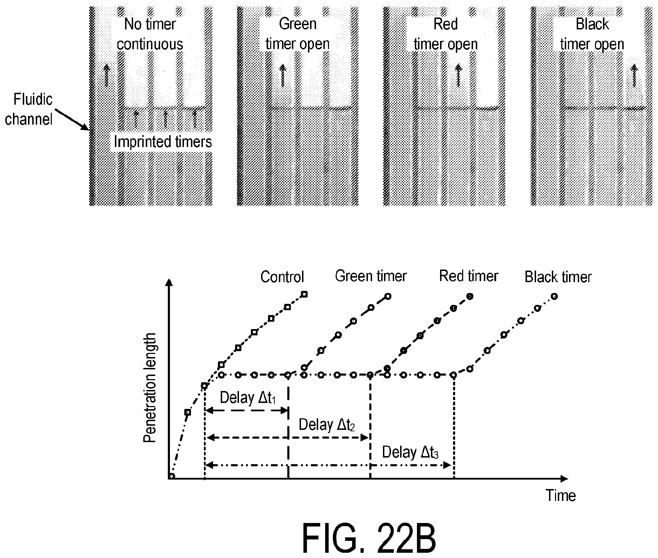

FIGS. 22A-22B show the effect of different color inks on capillary flow delay. FIG. 22A shows the measurements of the contact angles of the marked paper, which was painted with green, red, and black ink. It was observed that each ink showed a different contact angle, green (approximately 109°) < red (approximately 115°) < black (approximately 127°). The timers drawn with these inks showed different capillary flow delays; the higher the contact angle, the longer the flow delay, as demonstrated in FIG. 22B.

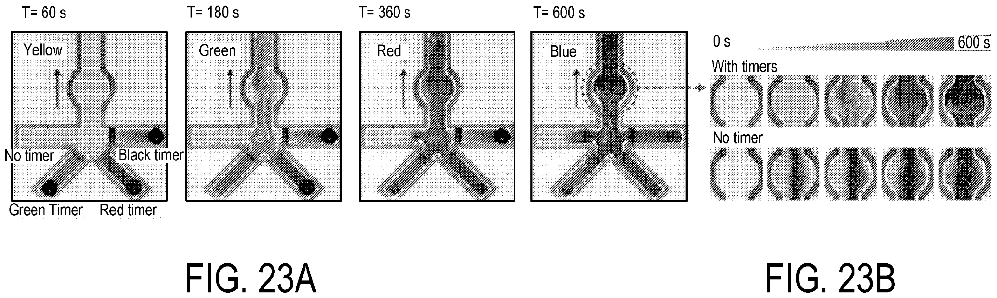

FIGS. 23A-23B show the sequential delivery of multiple solutions. In FIG. 22A, it was observed that four dyed solutions (water, simultaneously introduced to the test platform, reached the shared channel sequentially, with the flow from the branch without a timer arriving the first (yellow) and the one from the branch equipped with a black timer arriving last (blue). FIG. 22B show a control experiment on an otherwise identical device with no timers, which resulted in the simultaneous arrival of all dyed solutions to the shared channel.

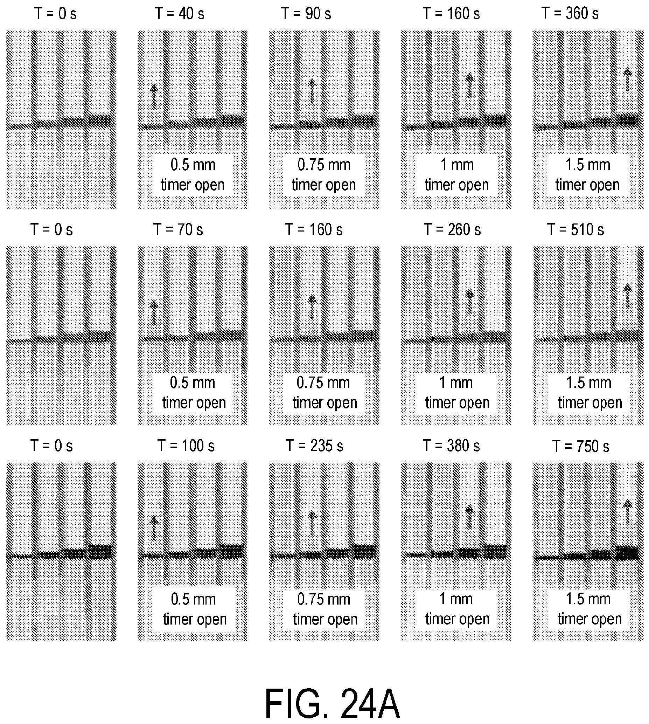

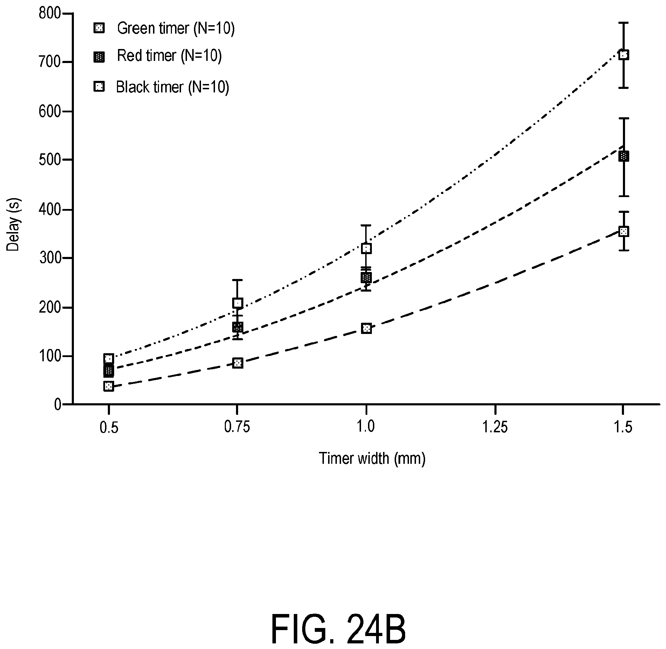

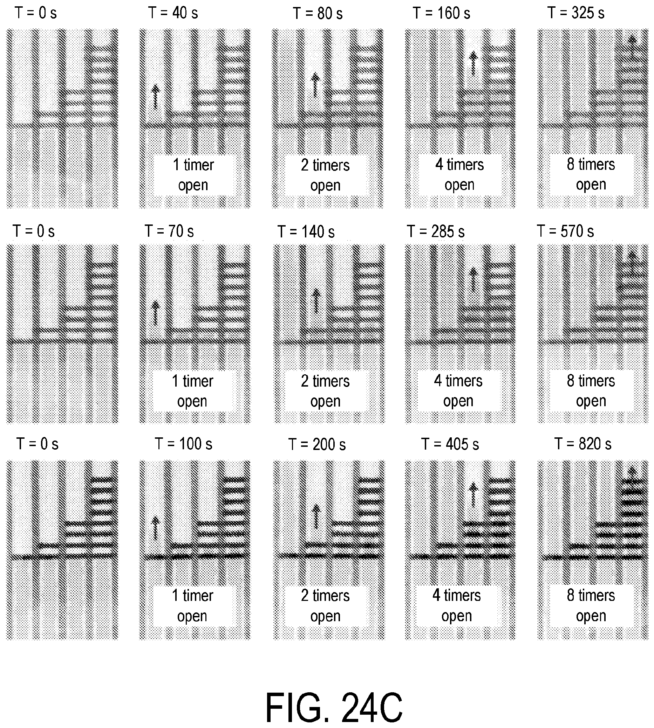

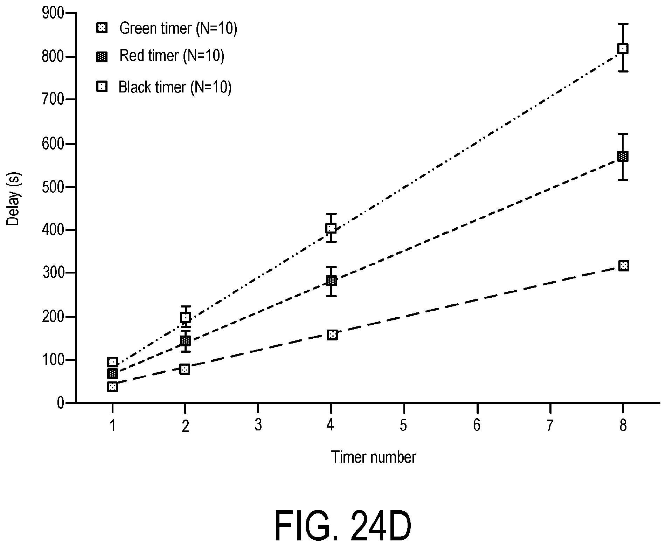

FIGS. 24A-24D show the characterization of different color delaminating inks. The length of delays generated by three colored timers of different widths (0.5 to 1.5 mm) was measured in independent experiments by monitoring the progress of dyed solutions through these timers, as shown in FIG. 24A. The delays introduced by three different colored timers increased with their width nonlinearly, as shown in FIG. 24B, as the 0.5-mm-wide timer resulted in an average flow delay of 37.6 s (green), 70 s (red), and 94.1 s (black), while a 1.5-mm-wide timer produced an average flow delay of approximately 6 minutes (green), approximately 8.5 minutes (red), and approximately 12 minutes (black). FIG. 24C shows the cascaded timer arrangements (1 to 8 timers) to achieve linear control over the flow delay. FIG. 24D shows a plot that confirms the linear dependence of the total flow delay on the number of timers in all different colored timers.

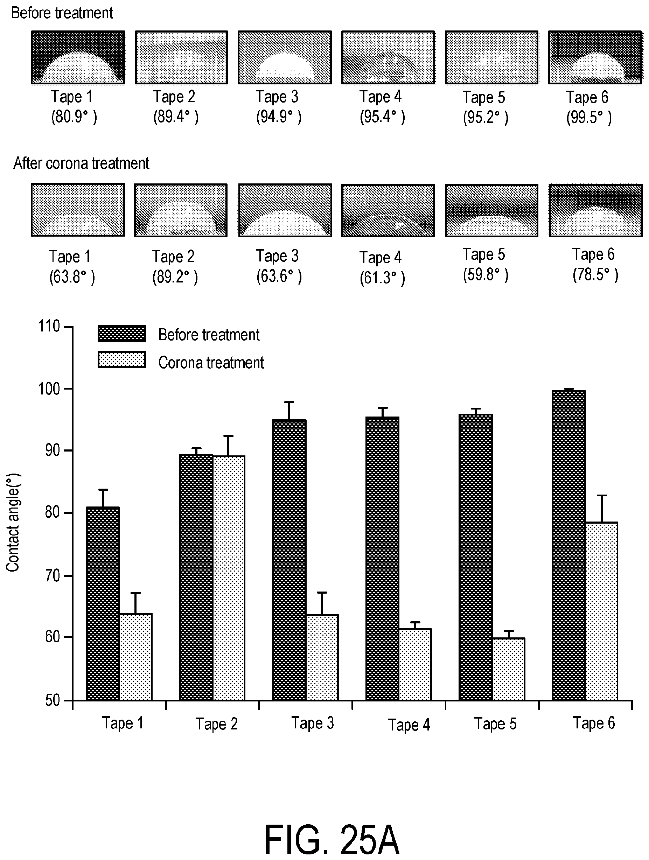

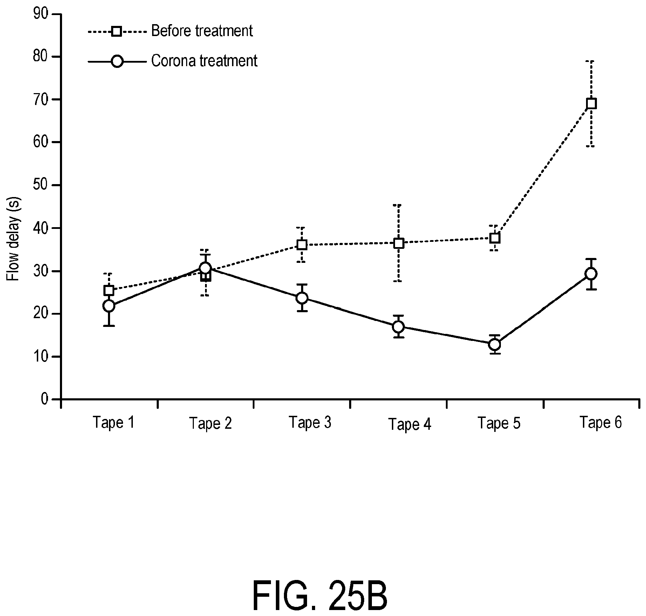

FIGS. 25A-25B show the effect of the sheath tapes and corona treatment on capillary flow delay. FIG. 25A shows the contact angle measurements for 6 different sheath tapes. FIG. 25A also shows a plot of the change in contact angle on the tape surfaces after treatment with corona discharge to render the surface more hydrophilic. FIG. 25B shows a plot of the flow delay produced by a green 0.5 mm timer laminated to the corresponding tape and a plot of the flow delays on tape surfaces treated with a corona discharge.

### DETAILED DESCRIPTION

The following description of the disclosure is provided as an enabling teaching of the disclosure in its best, currently known embodiments. Many modifications and other embodiments disclosed herein will come to mind to one skilled in the art to which the disclosed compositions and methods pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. The skilled artisan will recognize many variants and adaptations of the aspects described herein. These variants and adaptations are intended to be included in the teachings of this disclosure and to be encompassed by the claims herein.

Although specific terms are employed herein, they are used in a generic and descriptive sense only and not for purposes of limitation.

As can be apparent to those of skill in the art upon reading this disclosure, each of the individual embodiments described and illustrated herein has discrete components and features which may be readily separated from or combined with the features of any of the other several embodiments without departing from the scope or spirit of the present disclosure.

Any recited method can be carried out in the order of events recited or in any other order that is logically possible. That is, unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

It is also to be understood that the terminology used herein is for the purpose of describing particular aspects only and is not intended to be limiting. Unless defined otherwise, all technical and scientific terms used herein have the same meaning as commonly understood by one of ordinary skill in the art to which the disclosed compositions and methods belong. It can be further understood that terms, such as those defined in commonly used dictionaries, should be interpreted as having a meaning that is consistent with their meaning in the context of the specification and relevant art and should not be interpreted in an idealized or overly formal sense unless expressly defined herein.

Prior to describing the various aspects of the present disclosure, the following definitions are provided and should be used unless otherwise indicated. Additional terms may be defined elsewhere in the present disclosure.

### Definitions

In this specification and in the claims that follow, reference will be made to a number of terms, which shall be defined to have the following meanings.

As used herein, "comprising" is to be interpreted as specifying the presence of the stated features, integers, steps, or components as referred to, but does not preclude the presence or addition of one or more features, integers, steps, or components, or groups thereof. Moreover, each of the terms "by", "comprising," "comprises", "comprised of," "including," "includes," "included," "involving," "involves," "involved," and "such as" are used in their open, non-limiting sense and may be used interchangeably. Further, the term "comprising" is intended to include examples and aspects encompassed by the terms "consisting essentially of" and "consisting of." Similarly, the term "consisting essentially of" is intended to include examples encompassed by the term "consisting of."

As used in the specification and the appended claims, the singular forms "a," "an" and "the" include plural referents unless the context clearly dictates otherwise. Thus, for example, reference to "a compound", "a composition", or "a disorder", includes, but is not limited to, two or more such compounds, compositions, or disorders, and the like.

It should be noted that ratios, concentrations, amounts, and other numerical data can be expressed herein in a range format. It can be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. Ranges can be expressed herein as from "about" one particular value, and/or to "about" another particular value. Similarly, when values are expressed as approximations, by use of the antecedent "about," it can be understood that the particular value forms a further aspect. For example, if the value "about 10" is disclosed, then "10" is also disclosed.

When a range is expressed, a further aspect includes from the one particular value and/or to the other particular value. For example, where the stated range includes one or both of the limits, ranges excluding either or both of those included limits are also included in the disclosure, e.g., the phrase "x to y" includes the range from 'x' to 'y' as well as the range greater than 'x' and less than 'y'. The range can also be expressed as an upper limit, e.g., 'about x, y, z, or less' and should be interpreted to include the specific ranges of 'about x', 'about y', and 'about z' as well as the ranges of 'less than x', less than y', and 'less than z'. Likewise, the phrase 'about x, y, z, or greater' should be interpreted to include the specific ranges of 'about x', 'about y', and 'about z' as well as the ranges of 'greater than x', greater than y', and 'greater than z'. In addition, the phrase "about 'x' to 'y''', where 'x' and 'y' are numerical values, includes "about 'x' to about 'y'".

It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a numerical range of "about 0.1% to 5%" should be interpreted to include not only the explicitly recited values of about 0.1% to about 5%, but also include individual values (e.g., about 1%, about 2%, about 3%, and about 4%) and the sub-ranges (e.g., about 0.5% to about 1.1%; about 5% to about 2.4%; about 0.5% to about 3.2%, and about 0.5% to about 4.4%, and other possible sub-ranges) within the indicated range.

As used herein, the terms "about," "approximate," "at or about," and "substantially" mean that the amount or value in question can be the exact value or a value that provides equivalent results or effects as recited in the claims or taught herein. That is, it is understood that amounts, sizes, formulations, parameters, and other quantities and characteristics are not and need not be exact but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In such cases, it is generally understood, as used herein, that "about" and "at or about" mean the nominal value indicated ±10% variation unless otherwise indicated or inferred. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise. As used herein, the term "substantially free," when used in the context of a composition or component of a composition that is substantially absent, is intended to refer to an amount that is then about 1 % by weight or less, e.g., less than about 0.5 % by weight, less than about 0.1 % by weight, less than about 0.05 % by weight, or less than about 0.01 % by weight of the stated material, based on the total weight of the composition.

The term "patient" refers to a human in need of treatment for any purpose. However, the term "patient" can also refer to non-human animals, preferably mammals such as dogs, cats, horses, cows, pigs, sheep and non-human primates, among others, that are in need of treatment.

### Devices

Provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a channel; and a third line of a delaminating ink extending between the first line and the second line disposed on the first substrate side.

As used herein, an adhesive material refers to a material that is capable of holding materials together in a functional manner. In some embodiments, adhesive material can include tape. In further embodiments, the adhesive material can be chemically active such that it can selectively cause the ink to delaminate faster, slower, or not at all based on the chemical content of the sample.

As used herein, substrate can include porous materials such as paper, cellulose fiber filters, or woven meshes. In some embodiments, the device can be multilayered such that the adhesive materials and substrates can be alternatively stacked on top of each other.

As used herein, non-delaminating ink refers to ink that is comparatively more hydrophobic than delaminating ink and remains tethered to an adhesive material for a longer period of time than the delaminating ink. In certain embodiments, non-delaminating ink can be used to create channel boundaries to guide the flow of the liquid in the device.

In some embodiments, it is required that the non-delaminating ink is more hydrophobic than the delaminating ink. In further embodiments, the non-delaminating ink can eventually become untethered from the adhesive material when the sample has fully flowed through the device. In certain embodiments, the non-delaminating ink includes dye. In specific embodiments, the non-delaminating ink does not include dye. As used herein, channel refers to a feature on or in an article or the substrate that at least partially directs the flow of a fluid.

As used herein, delaminating ink refers to ink that is comparatively less hydrophobic than non-delaminating ink and separates from an adhesive material more quickly than the non-delaminating ink. Herein, delaminating ink can be used to temporarily hold the flow of liquid in the device. Delaminating ink is used in some embodiments of the invention as a line that extends between the boundaries of the channels and herein can also be referred to as a timer. In certain embodiments, the delaminating ink includes dye. In specific embodiments, the delaminating ink does not include dye.

In some embodiments, the device comprises a lateral flow assay.

A lateral flow assay (LFA) is a paper-based platform for the detection and quantification of analytes in a mixture, where the sample is placed on a test device and the results are then displayed. LFAs can be used for the qualitative and/or quantitative detection of specific antigens and antibodies, as well as products of gene amplification. Biological samples that can be tested using LFAs include, but are not limited to, urine, saliva, sweat, serum, plasma, and whole blood.

In further embodiments, the non-delaminating ink comprises a hydrophobic resin. As used herein, the hydrophobic resin is a resin with low affinity to water.

In certain embodiments, the width of the third line is at least 0.1 mm. In some embodiments, the width of the third line is from 0.1 mm to 0.5 mm, 0.5 mm to 1.0 mm, 1.0 mm to 1.5 mm, 1.5 mm to 2.0 mm, 2.0 mm to 2.5 mm, 2.5 mm to 3.0 mm, 3.0 mm to 3.5 mm, 3.5 mm to 4.0 mm, 4.0 mm to 4.5 mm, 4.5 mm to 5.0 mm, 5.0 mm to 5.5 mm, 5.5 mm to 6.0 mm, 6.0 mm to 6.5 mm, 6.5 mm to 7.0 mm, 7.0 mm to 7.5 mm, 7.5 mm to 8.0 mm, 8.0 mm to 8.5 mm, 8.5 mm to 9.0 mm, 9.0 mm to 9.5 mm, or 9.5 mm to 10 mm. In further embodiments, the width of the third line is from 0.1 mm to 1.0 mm, 0.1 mm to 2.0 mm, 0.1 mm to 3.0 mm, 0.1 mm to 4.0 mm, 0.1 mm to 5.0 mm, 0.1 mm to 6.0 mm, 0.1 mm to 7.0 mm, 0.1 mm to 8.0 mm, 0.1 mm 9.0 mm, or 0.1 mm to 10 mm. In certain embodiments, the width of the third line is from 10 mm or less, 9 mm or less, 8 mm or less, 7 mm or less, 6 mm or less, 5 mm or less, 4 mm or less, 3 mm or less, 2 mm or less, or 1 mm or less. In some embodiments, the width of the third line is from 0.1 mm to 2 mm, 2 mm to 4 mm, 4 mm to 6 mm, 6 mm to 8 mm, or 8 mm to 10 mm. In further embodiments, the width of the third line is from 0.1 mm to 10 mm, 10 mm to 20 mm, 20 mm to 30 mm, 30 mm to 40 mm, 40 mm to 50 mm, 50 mm to 60 mm, 60 mm to 70 mm, 70 mm to 80 mm, 80 mm to 90 mm, to 90 mm to 100 mm. In some embodiments, the width of the third line is at least 5 mm, at least 10 mm, at least 15 mm, at least 20 mm, at least 25 mm, at least 30 mm, at least 35 mm, at least 40 mm, at least 45 mm, or at least 50 mm. In certain embodiments, the width of the third line is from 10 mm to 12 mm, 12 mm to 14 mm, 14 mm to 16 mm, 16 mm to 18 mm, or 18 mm to 20 mm. In specific embodiments, the width of the third line is from 0.1 mm to 10 mm, 0.1 mm to 15 mm, 0.1 mm to 20 mm, 0.1 mm to 25 mm, 0.1 mm to 30 mm, 0.1 mm to 35 mm, 0.1 mm to 40 mm, 0.1 mm to 45 mm, or 0.1 mm to 50 mm. In some embodiments, there is no limit on the thickness of the third line.

In specific embodiments, the device further comprises at least two lines of the delaminating ink extending between the first line and the second line. In some embodiments, the device further comprises at least 3 lines, at least 4 lines, at least 5 lines, at least 6 lines, at least 7 lines, at least 8 lines, at least 9 lines, at least 10 lines, at least 11 lines, at least 12 lines, at least 13 lines, at least 14 lines, at least 15 lines, or at least 16 lines of the delaminating ink extending between the first line and the second line. In further embodiments, the device further comprises from 2 to 5, 2 to 10, 2 to 15, or 2 to 20 lines of the delaminating ink extending between the first line and the second line. In certain embodiments, the device further comprises from 2 to 10, 2 to 20, 2 to 30, 2 to 40, or 2 to 50 lines of the delaminating ink extending between the first line and the second line. In some embodiments, the maximum number of lines of the delaminating ink extending between the first line and the second line is the number of lines that fit based on the length of the channel defined by the first line and second line.

In some embodiments, a contact angle between the delaminating ink and the paper is from 0° to 180°. Contact angle is a measure of the ability of a liquid to wet the surface of a solid. The contact angle is an angle formed by a liquid at the three-phase boundary where a liquid, gas, and solid intersect. As contact angle decreases, surface energy increases and surface tension decreases. In some embodiments, the contact angle is from 0° to 60°, 60° to 120°, or 120° to 180°. In further embodiments, the contact angle is from 0° to 20°, 20° to 40°, 40° to 60°, 60° to 80°, 80° to 100°, 100° to 120°, 120° to 140°, 140° to 160°, or 160° to 180°. In specific embodiments, the contact angle is from 0° to 30°, 0° to 60°, 0° to 90°, 0° to 120°, 0° to 150°, or 0° to 180°. In some embodiments, the contact angle is from 0° to 15°, 15° to 30°, 30° to 45°, 45° to 60°, 60° to 75°, 75° to 90°, 90° to 105°, 105° to 120°, 120° to 135°, 135° to 150°, 150° to 165°, or 165° to 180°. In certain embodiments, the contact angle is from 90° or less, 80° or less, 70° or less, 60° or less, 50° or less, 40° or less, 30° or less, 20° or less, or 10° or less.

In further embodiments, the first adhesive material is treated with corona discharge. Corona discharge is a moderately low-power electric discharge that can occur at or near atmospheric pressure. The corona can be produced by strong electric fields associated with small diameter wires, needles, or sharp edges on an electrode. The free electron density in corona discharges is approximately 10⁸ electrons/cm³. Corona discharge occurs when an electrode at a high electric potential ionizes the gas surrounding it. The gas discharges the potential. If a plastic film is passed between the high-potential electrode and a grounded electrode, some of the ionized gas particles will undergo chemical reactions with the plastic surface, introducing reactive groups to the surface and increasing surface roughness. Functional groups such as carbonyls, hydroxyls, hydroperoxides, aldehydes, ethers, esters, carboxylic acids, and unsaturated bonds can be produced during this process. Corona discharge treatment has industrial applications which include electrophotography, printers, textile processing, and in-powder coating. Corona discharge can operate in atmospheric pressure, so air can be used as a reagent gas. Air plasma treatment can tailor the physical and chemical properties of the surface of materials resulting in enhanced hydrophilicity of the material by incorporating a variety of polar functional groups, such as oxygen and nitrogen containing groups.

In certain embodiments, the device further comprises chemicals to modulate adhesion and/or hydrophobicity. Chemicals that modulate adhesion can include, but are not limited to, adhesive polymers, such as acrylic, polyethylene, polystyrene, or any combination thereof. Chemicals that modulate hydrophobicity can include, but are not limited to, hydrophilic or hydrophobic chemicals, such as polyvinyl alcohol, trichloro(octyl(silane, or any combination thereof. The use of chemicals that modulate adhesion and/or hydrophobicity can help to control the required energy for delamination.

In specific embodiments, the first adhesive material, second adhesive material, and/or substrate are treated with biomolecules. As used herein, biomolecules can modulate the affinity between the adhesive materials and the substrates. In some embodiments, the biomolecules comprise a set of biomolecules. In further embodiments, sets of biomolecules can include an antigen and an antibody, biotin and streptavidin, an enzyme and a substrate, or hybridization of DNA. In modulating the affinity between the adhesive materials and the substrates, the biomolecules manipulate the levels of delamination.

In some embodiments, the device further comprises a sample. In further embodiments, the sample comprises urine, blood, plasma, serum, sweat, saliva, or any combination thereof.

In certain embodiments, the device further comprises a first mechanical force applied to the first substrate side and a second mechanical force applied to the second substrate side opposite the first mechanical force. In specific embodiments, the third line of the delaminating ink is disposed between the first mechanical force and the second mechanical force. In some embodiments, the first mechanical force and the second mechanical force are produced by two attracting magnets.

As used herein, mechanical force can be applied to the lines of delaminating ink to further control flow of the sample through the device. In some embodiments, the mechanical force can close the gap formed from the line of delaminating ink so as to temporarily prevent the sample from flowing through the device. In further embodiments, the mechanical forces applied to the line of delaminating ink can prevent delamination entirely, such that the sample does not flow through the channel of the device wherein the line of delaminating ink that is subject to the magnets is positioned.

Also provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a channel; a third line of a delaminating ink extending between the first line and the second line disposed on the first substrate side; a fourth line of a delaminating ink extending between the first line and the second line disposed on the first substrate side; and a target antigen LFA strip disposed between at least a portion of the second adhesive material and at least a portion of the second substrate side.

As used herein, target antigen LFA strip refers to a device that can detect the presence or absence of a target antigen in a sample provided by a patient.

In certain embodiments, the target antigen comprises human chorionic gonadotropin. Human chorionic gonadotropin (hCG) is a hormone produced primarily by syncytiotrophoblastic cells of the placenta during pregnancy. Smaller amounts of hCG are also produced in the pituitary gland, the liver, and the colon. The hormone stimulates the corpus luteum to produce progesterone to maintain the pregnancy.

In some embodiments, the device further comprises a case, wherein the case envelopes the first adhesive material, the substrate, the second adhesive material, and the target antigen LFA strip. As used herein, the case is a structure that encompasses at least a portion of the elements of the devices as disclosed herein. In some embodiments, the device can be created using a 3D printer. In further embodiments, after creating the case, it can be immersed in mineral oil at 65°C for the dewaxing of the uncured supporting material and then washed with soapy water, deionized water, and ethanol, sequentially. In certain embodiments, filler primer can be sprayed on the case to render the surface smooth. In some embodiments, after drying for 10 minutes, white paint was sprayed and fully dried.

In further embodiments, the case comprises at least one sample inlet. Sample inlet refers to the inlet of the device described herein in which the sample is deposited when using the device.

In certain embodiments, the case comprises at least one auxiliary inlet. Auxiliary inlet refers to the inlet of the device described herein in which the reagents used when operating the device are deposited.

In specific embodiments, the case comprises at least one detection window. Detection window refers to an aperture in the case through which the test line and control line can be observed when using the device.

In some embodiments, the device further comprises at least one signal amplification reagent, wherein the signal amplification reagent is deposited in the auxiliary inlet. As used herein, signal amplification reagents can include, but are not limited to, enhancers, activators, initiators, or buffers. These agents can include, but are not limited to, Proteinase K, deionized water, tris-EDTA buffer, or gold enhancement reagents, such as gold-based autometallography reagents.

In further embodiments, the device further comprises at least one control line. The control line refers to the line that indicates that the sample has flowed through the test strip.

In certain embodiments, the device comprises a lateral flow assay.

In specific embodiments, the non-delaminating ink comprises hydrophobic resin.

In some embodiments, a contact angle between the delaminating ink and the substrate is from 0° to 180°. In some embodiments, the contact angle is from 0° to 60°, 60° to 120°, or 120° to 180°. In further embodiments, the contact angle is from 0° to 20°, 20° to 40°, 40° to 60°, 60° to 80°, 80° to 100°, 100° to 120°, 120° to 140°, 140° to 160°, or 160° to 180°. In specific embodiments, the contact angle is from 0° to 30°, 0° to 60°, 0° to 90°, 0° to 120°, 0° to 150°, or 0° to 180°. In some embodiments, the contact angle is from 0° to 15°, 15° to 30°, 30° to 45°, 45° to 60°, 60° to 75°, 75° to 90°, 90° to 105°, 105° to 120°, 120° to 135°, 135° to 150°, 150° to 165°, or 165° to 180°. In certain embodiments, the contact angle is from 90° or less, 80° or less, 70° or less, 60° or less, 50° or less, 40° or less, 30° or less, 20° or less, or 10° or less.

In further embodiments, the first adhesive material is treated with corona discharge.

In certain embodiments, the device further comprises chemicals to modulate adhesion and/or hydrophobicity. Chemicals that modulate adhesion can include, but are not limited to, adhesive polymers, such as acrylic, polyethylene, polystyrene, or any combination thereof. Chemicals that modulate hydrophobicity can include, but are not limited to, hydrophilic or hydrophobic chemicals, such as polyvinyl alcohol, trichloro(octyl(silane, or any combination thereof. The use of chemicals that modulate adhesion and/or hydrophobicity can help to control the required energy for delamination.

In specific embodiments, the first adhesive material, the second adhesive material, and/or the substrate are treated with biomolecules. As used herein, biomolecules can modulate the affinity between the adhesive materials and the substrates. In some embodiments, the biomolecules comprise a set of biomolecules. In further embodiments, sets of biomolecules can include an antigen and an antibody, biotin and streptavidin, an enzyme and a substrate, or hybridization of DNA. In modulating the affinity between the adhesive materials and the substrates, the biomolecules manipulate the levels of delamination.

In some embodiments, the device further comprises a first mechanical force applied to the first substrate side and a second mechanical force applied to the second substrate side opposite the first mechanical force.

In further embodiments, the third line of the delaminating ink is disposed between the first mechanical force and the second mechanical force.

In certain embodiments, the fourth line of the delaminating ink is disposed between the first mechanical force and the second mechanical force.

In specific embodiments, the first mechanical force and the second mechanical force are produced by two attracting magnets.

In some embodiments, the width of the third line is at least 0.1 mm. In some embodiments, the width of the third line is from 0.1 mm to 0.5 mm, 0.5 mm to 1.0 mm, 1.0 mm to 1.5 mm, 1.5 mm to 2.0 mm, 2.0 mm to 2.5 mm, 2.5 mm to 3.0 mm, 3.0 mm to 3.5 mm, 3.5 mm to 4.0 mm, 4.0 mm to 4.5 mm, 4.5 mm to 5.0 mm, 5.0 mm to 5.5 mm, 5.5 mm to 6.0 mm, 6.0 mm to 6.5 mm, 6.5 mm to 7.0 mm, 7.0 mm to 7.5 mm, 7.5 mm to 8.0 mm, 8.0 mm to 8.5 mm, 8.5 mm to 9.0 mm, 9.0 mm to 9.5 mm, or 9.5 mm to 10 mm. In further embodiments, the width of the third line is from 0.1 mm to 1.0 mm, 0.1 mm to 2.0 mm, 0.1 mm to 3.0 mm, 0.1 mm to 4.0 mm, 0.1 mm to 5.0 mm, 0.1 mm to 6.0 mm, 0.1 mm to 7.0 mm, 0.1 mm to 8.0 mm, 0.1 mm 9.0 mm, or 0.1 mm to 10 mm. In certain embodiments, the width of the third line is from 10 mm or less, 9 mm or less, 8 mm or less, 7 mm or less, 6 mm or less, 5 mm or less, 4 mm or less, 3 mm or less, 2 mm or less, or 1 mm or less. In some embodiments, the width of the third line is from 0.1 mm to 2 mm, 2 mm to 4 mm, 4 mm to 6 mm, 6 mm to 8 mm, or 8 mm to 10 mm. In further embodiments, the width of the third line is from 0.1 mm to 10 mm, 10 mm to 20 mm, 20 mm to 30 mm, 30 mm to 40 mm, 40 mm to 50 mm, 50 mm to 60 mm, 60 mm to 70 mm, 70 mm to 80 mm, 80 mm to 90 mm, to 90 mm to 100 mm. In some embodiments, the width of the third line is at least 5 mm, at least 10 mm, at least 15 mm, at least 20 mm, at least 25 mm, at least 30 mm, at least 35 mm, at least 40 mm, at least 45 mm, or at least 50 mm. In certain embodiments, the width of the third line is from 10 mm to 12 mm, 12 mm to 14 mm, 14 mm to 16 mm, 16 mm to 18 mm, or 18 mm to 20 mm. In specific embodiments, the width of the third line is from 0.1 mm to 10 mm, 0.1 mm to 15 mm, 0.1 mm to 20 mm, 0.1 mm to 25 mm, 0.1 mm to 30 mm, 0.1 mm to 35 mm, 0.1 mm to 40 mm, 0.1 mm to 45 mm, or 0.1 mm to 50 mm. In some embodiments, there is no limit on the thickness of the third line.

In further embodiments, the width of the fourth line is at least 0.1 mm. In some embodiments, the width of the fourth line is from 0.1 mm to 0.5 mm, 0.5 mm to 1.0 mm, 1.0 mm to 1.5 mm, 1.5 mm to 2.0 mm, 2.0 mm to 2.5 mm, 2.5 mm to 3.0 mm, 3.0 mm to 3.5 mm, 3.5 mm to 4.0 mm, 4.0 mm to 4.5 mm, 4.5 mm to 5.0 mm, 5.0 mm to 5.5 mm, 5.5 mm to 6.0 mm, 6.0 mm to 6.5 mm, 6.5 mm to 7.0 mm, 7.0 mm to 7.5 mm, 7.5 mm to 8.0 mm, 8.0 mm to 8.5 mm, 8.5 mm to 9.0 mm, 9.0 mm to 9.5 mm, or 9.5 mm to 10 mm. In further embodiments, the width of the fourth line is from 0.1 mm to 1.0 mm, 0.1 mm to 2.0 mm, 0.1 mm to 3.0 mm, 0.1 mm to 4.0 mm, 0.1 mm to 5.0 mm, 0.1 mm to 6.0 mm, 0.1 mm to 7.0 mm, 0.1 mm to 8.0 mm, 0.1 mm 9.0 mm, or 0.1 mm to 10 mm. In certain embodiments, the width of the fourth line is from 10 mm or less, 9 mm or less, 8 mm or less, 7 mm or less, 6 mm or less, 5 mm or less, 4 mm or less, 3 mm or less, 2 mm or less, or 1 mm or less. In some embodiments, the width of the fourth line is from 0.1 mm to 2 mm, 2 mm to 4 mm, 4 mm to 6 mm, 6 mm to 8 mm, or 8 mm to 10 mm. In further embodiments, the width of the fourth line is from 0.1 mm to 10 mm, 10 mm to 20 mm, 20 mm to 30 mm, 30 mm to 40 mm, 40 mm to 50 mm, 50 mm to 60 mm, 60 mm to 70 mm, 70 mm to 80 mm, 80 mm to 90 mm, to 90 mm to 100 mm. In some embodiments, the width of the fourth line is at least 5 mm, at least 10 mm, at least 15 mm, at least 20 mm, at least 25 mm, at least 30 mm, at least 35 mm, at least 40 mm, at least 45 mm, or at least 50 mm. In certain embodiments, the width of the fourth line is from 10 mm to 12 mm, 12 mm to 14 mm, 14 mm to 16 mm, 16 mm to 18 mm, or 18 mm to 20 mm. In specific embodiments, the width of the fourth line is from 0.1 mm to 10 mm, 0.1 mm to 15 mm, 0.1 mm to 20 mm, 0.1 mm to 25 mm, 0.1 mm to 30 mm, 0.1 mm to 35 mm, 0.1 mm to 40 mm, 0.1 mm to 45 mm, or 0.1 mm to 50 mm. In some embodiments, there is no limit on the thickness of the fourth line.

Also provided herein is a device comprising a first adhesive material; a substrate configured to move a fluid by capillary action, wherein the substrate has a first substrate side and a second substrate side opposite the first substrate side, further wherein the first substrate side is coupled to at least a portion of the first adhesive material; a second adhesive material, wherein the second adhesive material is coupled to at least a portion of the second substrate side; a first line of a non-delaminating ink and a second line of the non-delaminating ink disposed on the first substrate side, wherein the first line and the second line define a first channel; a third line of the non-delaminating ink and a fourth line of the non-delaminating ink disposed on the first-substrate side, wherein the third line and the fourth line define a second channel; and a fifth line of a delaminating ink extending between the third line and the fourth line disposed on the first substrate side.

In some embodiments, the device further comprises a sixth line of the non-delaminating ink and a seventh line of the non-delaminating ink disposed on the first-substrate side, wherein the sixth line and the seventh line define a third channel.

In further embodiments, the device further comprises an eighth line of the delaminating ink and a ninth line of the delaminating ink extending between the sixth line and the seventh line disposed on the first substrate side.

In certain embodiments, the device further comprises a tenth line of the delaminating ink extending between the sixth line and the seventh line disposed on the first substrate side.

In specific embodiments, the device further comprises at least one DNA extraction spot, wherein the DNA extraction spot is disposed on the first substrate side. DNA extraction spot refers to the disks removed from cards used for DNA collection, shipment, archiving, and purification. In certain embodiments, these cards can be FTA cards. In some embodiments, the FTA cards from which the DNA extraction spots are removed are designed for room temperature collection, shipment, archiving, and purification of nucleic acids from biological samples. In further embodiments, the DNA extraction spots can be impregnated with a patented chemical formula that lyses cells and denatures proteins upon contact.

In some embodiments, the device further comprises Proteinase K, deionized water, and tris-EDTA buffer disposed on the first substrate side. Proteinase K is a subtilisin-related serine protease that can hydrolyze a variety of peptide bonds. Proteinase K can digest protein and remove contamination from preparations of nucleic acid. Addition of Proteinase K to nucleic acid preparations can rapidly inactivate nucleases that could otherwise degrade the DNA or RNA during purification. It can also be used for the release of nucleic acids as it can inactivate DNases and RNases. Deionized water is water from which dissolved ionic salts have been removed by ion-exchange techniques. Tris-EDTA (TE) buffer comprises tris, a common pH buffer, and EDTA (ethylenediaminetetraacetic), a molecule that can chelate cations such as Mg²⁺. TE buffer can solubilize DNA or RNA while protecting it from degradation.

In further embodiments, the device further comprises at least one sample inlet.

In certain embodiments, the device further comprises at least one auxiliary inlet.

In specific embodiments, the fifth line is from 0.8 mm to 1.2 mm wide. In some embodiments, the seventh line is form 0.8 mm to 0.9 mm, 0.9 mm to 1.0 mm, 1.0 mm to 1.1 mm, 1.1 mm to 1.2 mm. In further embodiments, the seventh line is from 0.8 mm to 0.85 mm, 0.85 mm to 0.9 mm, 0.9 mm to 0.95 mm, 0.95 mm to 1.0 mm, 1.0 mm to 1.05 mm, 1.05 mm to 1.1 mm, or 1.1 mm to 1.2 mm. In certain embodiments, the seventh line is from 0.95 mm to 0.96 mm, 0.96 mm to 0.97 mm, 0.98 mm to 0.99 mm, 0.99 mm to 1.0 mm, 1.0 mm to 1.1 mm, 1.1 mm to 1.2 mm, 1.2 mm to 1.3 mm, 1.3 mm to 1.4 mm, or 1.4 mm to 1.5 mm. In some embodiments, the seventh line is from 0.95 mm to 0.96 mm, 0.95 mm to 0.97 mm, 0.95 mm to 0.98 mm, 0.95 mm to 0.99 mm, 0.95 mm to 1.0 mm, 0.95 mm to 1.1 mm, 0.95 mm to 1.2 mm, 0.95 mm to 1.3 mm, 0.95 mm to 1.4 mm, or 0.95 mm to 1.5 mm.

In some embodiments, the eighth line, ninth line, and tenth line are from 0.8 to 1.2 mm wide. In some embodiments, the seventh line is form 0.8 mm to 0.9 mm, 0.9 mm to 1.0 mm, 1.0 mm to 1.1 mm, 1.1 mm to 1.2 mm. In further embodiments, the seventh line is from 0.8 mm to 0.85 mm, 0.85 mm to 0.9 mm, 0.9 mm to 0.95 mm, 0.95 mm to 1.0 mm, 1.0 mm to 1.05 mm, 1.05 mm to 1.1 mm, or 1.1 mm to 1.2 mm. In certain embodiments, the seventh line is from 0.95 mm to 0.96 mm, 0.96 mm to 0.97 mm, 0.98 mm to 0.99 mm, 0.99 mm to 1.0 mm, 1.0 mm to 1.1 mm, 1.1 mm to 1.2 mm, 1.2 mm to 1.3 mm, 1.3 mm to 1.4 mm, or 1.4 mm to 1.5 mm. In some embodiments, the seventh line is from 0.95 mm to 0.96 mm, 0.95 mm to 0.97 mm, 0.95 mm to 0.98 mm, 0.95 mm to 0.99 mm, 0.95 mm to 1.0 mm, 0.95 mm to 1.1 mm, 0.95 mm to 1.2 mm, 0.95 mm to 1.3 mm, 0.95 mm to 1.4 mm, or 0.95 mm to 1.5 mm.

In further embodiments, the device comprises a lateral flow assay.

In certain embodiments, the non-delaminating ink comprises hydrophobic resin.

In specific embodiments, a contact angle between the delaminating ink and the substrate is from 0° to 180°. In some embodiments, the contact angle is from 0° to 60°, 60° to 120°, or 120° to 180°. In further embodiments, the contact angle is from 0° to 20°, 20° to 40°, 40° to 60°, 60° to 80°, 80° to 100°, 100° to 120°, 120° to 140°, 140° to 160°, or 160° to 180°. In specific embodiments, the contact angle is from 0° to 30°, 0° to 60°, 0° to 90°, 0° to 120°, 0° to 150°, or 0° to 180°. In some embodiments, the contact angle is from 0° to 15°, 15° to 30°, 30° to 45°, 45° to 60°, 60° to 75°, 75° to 90°, 90° to 105°, 105° to 120°, 120° to 135°, 135° to 150°, 150° to 165°, or 165° to 180°. In certain embodiments, the contact angle is from 90° or less, 80° or less, 70° or less, 60° or less, 50° or less, 40° or less, 30° or less, 20° or less, or 10° or less.

In some embodiments, the adhesive material is treated with corona discharge.

In further embodiments, the device further comprises chemicals to modulate adhesion and/or hydrophobicity. Chemicals that modulate adhesion can include, but are not limited to, adhesive polymers, such as acrylic, polyethylene, polystyrene, or any combination thereof. Chemicals that modulate hydrophobicity can include, but are not limited to, hydrophilic or hydrophobic chemicals, such as polyvinyl alcohol, trichloro(octyl(silane, or any combination thereof. The use of chemicals that modulate adhesion and/or hydrophobicity can help to control the required energy for delamination.

In certain embodiments, the first adhesive material, the second adhesive material, and or the substrate are treated with biomolecules. As used herein, biomolecules can modulate the affinity between the adhesive materials and the substrates. In some embodiments, the biomolecules comprise a set of biomolecules. In further embodiments, sets of biomolecules can include an antigen and an antibody, biotin and streptavidin, an enzyme and a substrate, or hybridization of DNA. In modulating the affinity between the adhesive materials and the substrates, the biomolecules manipulate the levels of delamination.

In specific embodiments, the device further comprises a first mechanical force applied to the first substrate side and a second mechanical force applied to the second substrate side opposite the first mechanical force.

In some embodiments, the fifth line of the delaminating ink, eighth line of the delaminating ink, ninth line of the delaminating ink, tenth line of the delaminating ink, or any combination thereof, is disposed between the first mechanical force and the second mechanical force.

In further embodiments, the first mechanical force and the second mechanical force are produced by two attracting magnets.

Also provided herein is a device comprising a first adhesive material; a first substrate configured to move a fluid by capillary action, wherein the first substrate has a first substrate side and a second substrate side opposite the first substrate side, further wherein the first substrate side is coupled to at least a portion of the first adhesive material; a double-sided adhesive material, wherein the double-sided adhesive material has a first adhesive side and a second adhesive side opposite the first adhesive side, further wherein the first adhesive side is coupled to at least a portion of the second substrate side; a second substrate configured to move the fluid by capillary action, wherein the second substrate has a third substrate side and a fourth substrate side opposite the third substrate side, further wherein the third substrate side is coupled to at least a portion of the second adhesive side; a second adhesive material, wherein the second adhesive material is coupled to at least a portion of the fourth substrate side; at least one DNA extraction spot disposed between the second substrate side and the first adhesive side; a first line of a non-delaminating ink and a second line of the non-delaminating ink disposed on the first substrate side, wherein the first line and the second line define a first channel; a third line of the non-delaminating ink and a fourth line of the non-delaminating ink disposed on the first substrate side, wherein the third line and the fourth line define a second channel; a fifth line of the non-delaminating ink and a sixth line of the non-delaminating ink disposed on the third substrate side, wherein the fifth line and the sixth line define a third channel; a seventh line of a delaminating ink extending between the third line and the fourth line disposed on the first substrate side; an eighth line of the delaminating ink extending between the fifth line and the sixth line disposed on the third substrate side; and a first primer mixture, a second primer mixture, and a third primer mixture disposed on the third substrate side.

In some embodiments, the first primer mixture targets SARS-CoV-2 virus. Primer mixture refers to a mixture comprising a nucleic acid molecule including DNA, RNA, or analogs thereof, suitable for DNA-related techniques including, but not limited to, hybridization, strand extension, amplification, or sequencing. In some embodiments, primers can be DNA. In further embodiments, primers can be single-stranded DNA. In certain embodiments, primers can be from 18 to 25 nucleotides long.

Severe acute respiratory syndrome coronavirus 2 (SARS-CoV-2) is a type of huma coronavirus. Representative examples of human coronavirus can also include, but are not limited to, human coronavirus 229E (HCoV-229E), human coronavirus OC43 (HCoV-OC43), human coronavirus HKU1 (HCoV-HKU1), Human coronavirus NL63 (HCoV-NL63), severe acute respiratory syndrome coronavirus (SARS-CoV), and Middle East respiratory syndrome-related coronavirus (MERS-CoV).

In some embodiments, the coronavirus infection can be caused an avian coronavirus (IBV), porcine coronavirus HKU15 (PorCoV HKU15), Porcine epidemic diarrhea virus (PEDV), HCoV-229E, HCoV-OC43, HCoV-HKU1, HCoV-NL63, SARS-CoV, SARS-CoV-2, or MERS-CoV.

As used herein, "COVID-19" refers to the infectious disease caused by SARS-CoV-2 and characterized by, for example, fever, cough, respiratory symptoms, rhinorrhea, sore throat, malaise, headache, chills, repeated shaking with chills, diarrhea, new loss of smell or taste, muscle pain, or a combination thereof.

In some embodiments, the subject with a coronavirus exhibits one or more symptoms associated with mild COVID-19, moderate COVID-19, mild-to-moderate COVID-19, severe COVID-19 (e.g., critical COVID-19), or exhibits no symptoms associated with COVID-19 (asymptomatic). It should be understood that in reference to the treatment of patients with different COVID-19 disease severity, "asymptomatic" infection refers to patients diagnosed with COVID-19 by a standardized RT-PCR assay that do not present with fever, cough, respiratory symptoms, rhinorrhea, sore throat, malaise, headache, or muscle pain.

In some embodiments, the subject with a coronavirus exhibits one or more symptoms selected from dry cough, shortness of breath, and fever. In other embodiments, the subject exhibits no symptoms associated with COVID-19 but has been exposed to another subject known or suspected of having COVID-19.

In further embodiments, the first primer mixture comprises a polynucleotide having an amino acid identified by any one of SEQ ID NOS: 1-12. In further embodiments, the first primer mixture comprises a polynucleotide having an amino acid identified by any one of SEQ ID NOS: 1-6. In further embodiments, the first primer mixture comprises a polynucleotide having an amino acid identified by any one of SEQ ID NOS: 7-12.

In certain embodiments, the second primer mixture targets influenza A virus. Influenza A virus is a negative-sense, single-stranded, segmented RNA virus. The subtypes are labeled according to an H number (for the type of hemagglutinin) and an N number (for the type of neuraminidase). There are 18 different known H antigens and 11 different known N antigens. Influenza A can cause influenza in birds and some mammals. Strains of all subtypes of influenza A virus have been isolated from wild birds. Some isolates of influenza A virus cause severe disease in domestic poultry, and, rarely, in humans. The virus can be transmitted from wild aquatic birds to domestic poultry, which can cause an outbreak or give rise to human influenza pandemics. Influenza A includes avian influenza, which further includes, but is not limited to, subtypes such as H6N1, H6N2, H7N6, H7N7, H7N9, H9N6, H9N7, H9N8, H9N9, H10N3, H10N4, H10N5, H10N6, H10N7, and H10N8.

In specific embodiments, the second primer mixture comprises a polynucleotide having an amino acid identified by any one of SEQ ID NOS: 13-18.

In some embodiments, the third primer mixture targets influenza B virus. Influenza B is a type of influenza that can only pass from human to human. Influenza B can be highly contagious and can have dangerous effects in a patient's health in more severe cases. Symptoms of influenza B can include, but are not limited to, fever, chills, sore throat, coughing, runny nose and sneezing, fatigue, and muscle aches and body aches. The influenza B genome is

In further embodiments, the third primer mixture comprises a polynucleotide having an amino acid identified by any one of SEQ ID NOS: 19-24.

In certain embodiments, the device further comprises Proteinase K and DI water disposed on the first substrate side.

In specific embodiments, the device further comprises RT-LAMP buffer disposed on the third substrate side. RT-LAMP (reverse transcription loop-mediated isothermal amplification) buffer is a buffer chemical used for an RT-LAMP reaction. RT-LAMP is a method of amplifying and identifying the transcripts of a targeted pathogen. More specifically, RT-LAMP can be used for viral RNA detection.

In some embodiments, the device further comprises an internal heating element coupled to the second adhesive material opposite the fourth substrate side. In further embodiments, the internal heating element comprises an exothermic reaction. In certain embodiments, the exothermic reaction comprises an oxidation reaction. In specific embodiments, the oxidation reaction comprises calcium oxide and water.

As used herein, the internal heating element can include an internal heating module with an exothermic reaction that when triggered, produces the appropriate heat, and can initiate the RT-LAMP reaction. An exothermic reaction is a reaction in which energy is released into the surroundings in the form of light or heat. Exothermic reactions can include, but are not limited to, combustion, polymerization, neutralization, respiration, and oxidation. An oxidation reaction is a type of chemical reaction that involves a transfer of electrons between two species and as such, a change in the oxidation number of a molecule, atom, or ion by gaining or losing an electron. Oxidation reactions include combination reactions, which entail the combining of elements to form a chemical compound. Oxidation reactions can include the reaction between calcium oxide and water to form calcium hydroxide, as demonstrated below.

*CaO* (*s*) *+ H*₂*O* (*l*) → *Ca(OH)*₂ (*s*) *ΔHᵣ* = -65.2 *kJ*/*mol*

In one embodiment, calcium oxide powder can be made to react with water continuously carried from a neighboring reservoir via a cellulose paper strip (Fig. 20A). In further embodiments, temperature regulation of the reaction can be accomplished with a candelilla wax that surrounds the reaction chamber in which the reaction takes place, wherein the wax melts at from approximately 68°C to 72°C. In some embodiments, the wax melts at from 68°C to 69°C, 69°C to 70°C, 70°C to 71°C, or 71°C to 72°C.

The internal heating element can also include a different exothermic reaction, wherein the exothermic reaction provides the required heat for the RT-LAMP reaction. In further embodiments, the internal heating element can include an electric heating element. In some embodiments, the internal heating element includes a regulator, such as candelilla wax.

In some embodiments, the device comprises a lateral flow assay.

In further embodiments, the non-delaminating ink comprises hydrophobic resin.

In certain embodiments, a contact angle between the delaminating ink and the substrate is from 0° to 180°. In some embodiments, the contact angle is from 0° to 60°, 60° to 120°, or 120° to 180°. In further embodiments, the contact angle is from 0° to 20°, 20° to 40°, 40° to 60°, 60° to 80°, 80° to 100°, 100° to 120°, 120° to 140°, 140° to 160°, or 160° to 180°. In specific embodiments, the contact angle is from 0° to 30°, 0° to 60°, 0° to 90°, 0° to 120°, 0° to 150°, or 0° to 180°. In some embodiments, the contact angle is from 0° to 15°, 15° to 30°, 30° to 45°, 45° to 60°, 60° to 75°, 75° to 90°, 90° to 105°, 105° to 120°, 120° to 135°, 135° to 150°, 150° to 165°, or 165° to 180°. In certain embodiments, the contact angle is from 90° or less, 80° or less, 70° or less, 60° or less, 50° or less, 40° or less, 30° or less, 20° or less, or 10° or less.

In specific embodiments, the adhesive material is treated with corona discharge.

In some embodiments, the device further comprises chemicals to modulate adhesion and/or hydrophobicity. Chemicals that modulate adhesion can include, but are not limited to, adhesive polymers, such as acrylic, polyethylene, polystyrene, or any combination thereof. Chemicals that modulate hydrophobicity can include, but are not limited to, hydrophilic or hydrophobic chemicals, such as polyvinyl alcohol, trichloro(octyl(silane, or any combination thereof. The use of chemicals that modulate adhesion and/or hydrophobicity can help to control the required energy for delamination.

In further embodiments, the first adhesive material, the second adhesive material, the first substrate, and/or the second substrate are treated with biomolecules. As used herein, biomolecules can modulate the affinity between the adhesive materials and the substrates. In some embodiments, the biomolecules comprise a set of biomolecules. In further embodiments, sets of biomolecules can include an antigen and an antibody, biotin and streptavidin, an enzyme and a substrate, or hybridization of DNA. In modulating the affinity between the adhesive materials and the substrates, the biomolecules manipulate the levels of delamination.

In certain embodiments, the device further comprises a first mechanical force applied to the first substrate side and a second mechanical force applied to the second substrate side opposite the first mechanical force.

In specific embodiments, the seventh line of the delaminating ink, eighth line of the delaminating ink, or any combination thereof, is disposed between the first mechanical force and the second mechanical force.

In some embodiments, the first mechanical force and the second mechanical force are produced by two attracting magnets.

### Methods

### Method of Detecting a Target Material in a Sample

The present disclosure, in one aspect, provides for a method of detecting a target material in a sample, comprising testing the sample on a device as disclosed herein. As used herein, "detect" or "detecting" with respect to a virus includes the use of the device disclosed herein to observe the signal corresponding to the presence of the virus and the relevant materials to generate that signal.

In some embodiments, the device further comprises measuring a concentration of the target material. In further embodiments, the target material comprises human chorionic gonadotropin. In certain embodiments, the sample comprises urine.

In some embodiments, the method of detecting a target material in a sample includes collecting a sample, loading the sample into the sample inlet, loading signal amplification reagents, and deionized water, into the auxiliary inlet, and detecting the presence of a signal of a target material in the detection window by examining the resulting signal and the control line. In further embodiments the signal can include, but is not limited to, colorimetric signals or fluorescent signals. In some embodiments, detection can be done visually (e.g., the change is measured by the user's eye) or measured by an instrument (e.g., digital image processing, a photodiode, photomultiplier, or the like). In certain embodiments, the device includes three auxiliary inlets for varying signal amplification reagents and one timer is placed between the first and second auxiliary inlets and the second timer is placed between the second and third auxiliary inlets, such that when the sample is transported on the substrate via capillary action, the sample contacts the reagents in a specified order and in a timely manner. (See Example 3.)

### Method of Purifying Nucleic Acids

Also provided herein are a method of purifying DNA, comprising depositing a sample on a device as disclosed herein.

In some embodiments, the DNA comprises human DNA. In further embodiments, the sample is deposited on the DNA extraction spot.

Also provided herein are a method of purifying a nucleic acid, comprising depositing a sample on a device as disclosed herein. In some embodiments, the nucleic acid comprises DNA. In some embodiments, the nucleic acid comprises RNA.

In some embodiments, the method of purifying nucleic acids includes collecting a sample of DNA (e.g., punching out DNA extraction spots from filter paper pretreated with chemical for lysing cells and preserving extracted DNA, also referred to as FTA paper), depositing the DNA extraction spots in the device, (e.g., in a sample inlet), and placing the reagents proteinase K, deionized water, and tris-EDTA buffer into the device, (e.g., in separate auxiliary inlets), after which point the DNA extraction spots include the purified DNA. In further embodiments, the sample is subjected to proteinase K, deionized water, and then TE buffer, in that order, due to the inclusion of timers in the device. (See Example 4.)

### Method of Detecting SARS-Cov-2, an Influenza A Virus, and/or an Influenza B Virus

Provided herein is a method of detecting a pathogen, comprising testing a sample on a device as disclosed herein. In some embodiments, the pathogen is a virus. In some embodiments, one or more pathogens are detected. In some embodiments, one or more viruses are detected.

Further provided herein is a method of detecting a SARS-Cov-2 virus, an influenza A virus, and/or an influenza B virus, comprising testing a sample on a device as disclosed herein.

In some embodiments, the sample comprises saliva.

In further embodiments, a method of detecting a pathogen includes collecting a sample (e.g., nasal swab, saliva swab, blood sample, etc.), depositing a sample in the device (e.g., in a sample inlet), depositing reagents (e.g., proteinase K, deionized water, RT-LAMP buffer) in the device (e.g., in separate auxiliary inlets), depositing three primer mixtures in the device, wherein the primer mixtures target SARS-CoV-2, influenza A, and influenza B, heating the device to a desired temperature using the internal heat module, and detecting the presence of a signal (e.g., colorimetric, fluorescent) for a target (e.g., SARS-CoV-2, influenza A, and/or influenza B). In certain embodiments, the samples can be deposited in the device in the form of DNA extraction spots. In specific embodiments, images of the signals can be captured using the ImageJ program. In some embodiments, the sample is subjected to the reagents in the following order: proteinase K, deionized water, and RT-LAMP buffer, due to the inclusion of timers in the device. (See Example 12.)

A number of embodiments of the disclosure have been described. Nevertheless, it will be understood that various modifications may be made without departing from the spirit and scope of the invention. Accordingly, other embodiments are within the scope of the following claims.

By way of non-limiting illustration, examples of certain embodiments of the present disclosure are given below.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present invention, which are apparent to one skilled in the art.

Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, temperatures, pressures, and other reaction ranges and conditions that can be used to optimize the product purity and yield obtained from the described process. Only reasonable and routine experimentation will be required to optimize such process conditions.

### Example 1: Capillary flow control via sheath tape delamination

To control capillary flow in LFAs, a technique was introduced that utilized local voids forming between a wetted paper and a sheath polymer tape. by selectively modulating the paper-tape adhesion through imprinting patterns on the paper, the long-overlooked polymer sheath tape was transformed into an extrinsic flow path that can be programmed to manipulate the flow within the paper. The technique can be implemented with different combinations of paper, ink, and sheath tapes. With the expansion of available materials, the delamination process within timers could be manipulated through a variety of physical and chemical processes, which would extend the capabilities of the introduced technology beyond non-discriminatively delaying capillary flows and introduce complex gating mechanisms to LFAs integrated with delaminating timers.

The ability to sequentially deliver different reagents into a reaction via programmable timers imprinted on paper makes it possible to automate multi-step assays, that otherwise could only be performed in laboratories or with manual intervention. Immunoassays were created that are an order of magnitude more sensitive than commercial counterparts or POC devices that can extract, purify, and store DNA from a variety of bodily fluids with higher efficiency than the established laboratory protocols. As such, the capabilities provided by the presented technology can make it possible to develop LFAs that can rival ELISA- or PCR-based assays for sensitive and specific detection of pathogenic targets such a Zika virus, human immunodeficiency virus, hepatitis B virus, or diseases like malaria. This example can make complex and traditionally labor-intensive assays available in assay formats as simple and frugal as conventional LFAs.

### Discussion

To manipulate capillary flow on a laminated paper, patterns were created by imprinting with two water-insoluble inks with different levels of hydrophobicity: the less hydrophobic one was to eventually separate from the adhesive sheath tape when wetter (e.g., delaminating ink) and hence was used to mark spots where the flow was temporarily held. The more hydrophobic ink was required to remain permanently tethered to the sheath tape during the assay leaving no voids for flow (e.g., non-delaminating ink) and was employed to create channel boundaries to guide the flow (Fig. 1A). Those inks with desired properties were identified among commercially available permanent markers by measuring water contact angle on marked paper (Fig. 1A) and by verifying desired delamination response in water (Figs. 5A-5B). The main compositional difference between the two inks was that the non-delaminating ink contained a hydrophobic resin, which formed a water-impenetrable bond with adhesive polymer on the sheath tape.

To test the functionality of the approach for capillary flow control, dye solutions were used to track the leading edge of the flow (Fig. 1B). In these experiments, the timer was created as a discrete line normal to the flow direction within channel boundaries that were imprinted by the non-delaminating ink. The advance of the dye on paper was observed to completely stop when it reached the timer and only resumed its progress after a delay. Moreover, once the timer was cleared, the flow showed no memory effect and advanced as if there were no obstacles in the first place. Finally, the colorants within the timer were not washed away with the flow, a result that suggested the wetted timer retained its integrity as intended.

Using the same experimental setup, the mechanism of action for the timer was investigated. First, compared were the timer response on laminated versus naked paper and it was observed that the capillary flow was permanently blocked by the same timer when it was imprinted on a naked paper. These results invalidated dissolving of the timer or flow leaking through it as potential causes behind the delay observed in the laminated paper. Considering that the formation of a void between the polymer tape and the paper would necessitate mechanical displacement, it was next attempted to constrain the paper-polymer tape stack by a compression force. When the timer in the laminated paper was placed in between tow attracting magnets, the capillary flow indeed stopped permanently (Figs. 6A-6F). In contrast, the control setup that only included the compressing magnets, but no timer resulted in a continuous capillary flow. These analyses established the delamination of polymer tape from the paper as the mechanism of action or delaying capillary flow.

Next, it was attempted to coordinate multiple capillary flow streams through a group of imprinted timers on paper. As a test platform, a simple flow layout was created, where four inlet branches merged into a single channel (Fig. 1C). In each branch, varying numbers of timers were placed to differentially delay the flow between different branches. to distinguish between different capillary flows on the device, each inlet received a distinctly colored dye solution. Observed color pattern clearly showed four fluids, simultaneously introduced to the device, reached the shared channel sequentially, with the flow from the branch without any timers arriving first (yellow) and the one from the branch equipped with most timers arriving last (blue) (Fig. 1D). In contrast, a control experiment on an otherwise identical device with no timers resulted in the simultaneous arrival of all dyed fluids to the shared channel, thereby proving the role of timers in achieving a sequential flow pattern. Taken together, these results demonstrated the feasibility of performing multi-step assays in an automated fashion with imprinted timers that delaminate from the sheath tape.

### Example 2: Characterization and modeling of the timer delay

The effect of timer geometry on the amount of time delay introduced into the capillary flow on a paper was studied. The length of delays generated by timers of different widths (0.5-3 mm) was measured in independent experiments by monitoring the progress of dye solutions through these timers (Fig. 2A). It was found that the delay introduced by the timer increased with its width, as wider timers comprise a larger bonding interface with the polymer tape making it harder to form a void. Furthermore, the increase in delay was nonlinear as a 0.5 mm-wide timer comprise a larger bonding interface with the polymer tape making it harder to form a void. Furthermore, the increase in delay was nonlinear as a 0.5 mm-wide timer resulted in an average delay of 3.76 seconds, while a 3-mm timer produced an average flow delay of approximately 23 minutes (Fig. 2B). Capillary flow delays ranging from 30 s to >18 hours using imprinted timers. The minimum achievable delay was limited by the smallest feature size (approximately 0.5 mm) that cold reliably be imprinted on the paper using the experimental setup. On the other hand, the flow delay could be arbitrarily increased in the experiments as long as the space permitted for wider or more timers. The maximum achievable delay will likely be limited by eventual disintegration of wetted paper, dissolution of permanent ink, or sample evaporation. Taken together, it could be concluded that the width of the timer, a parameter that can easily be tweaked, was a viable lever to set specific time delays for capillary flow on paper.

While all tested timers produced relatively reproducible delays over repeated measurements (N=10), wider timers led to more noise in the produced time delay (with a coefficient of variation of approximately 16.1% for a 3 mm-wide timer), likely due to higher entropy in the delamination process (Fig. 7). Inevitable variations of the timer geometry from imprinting on paper also produced variability in time delays, which were more pronounced for narrower timers. Variations in delamination duration for individual timers can be treated as noise and ultimately determined the temporal resolution of flow control using our technology.

While the nonlinear increase in delay with wider timers is advantageous when long delays are to be implemented, cascade timer arrangements were also studied with the goal of achieving linear control over capillary flow delay (Fig. 2C). The measurements confirmed the linear dependence of the total flow delay on the number of timers imprinted in the channel and showed high repeatability (N=10) (Fig. 2D). The ability to modulate capillary flow delay only through creating new instances of the same delay element is important as it simplifies the LFA manufacturing process by eliminating the need to tune the timer geometry at different spots on a device. In addition, less variability from narrower timers can be used to achieve the same time delay with a higher temporal resolution with cascaded timers compared to a single wider timer.

The effect of liquid viscosity and temperature on the timer response was also investigated. To manipulate the liquid viscosity, sucrose was added into DI water and the measurements with DI water mixed with glycerol were repeated to ensure against ingredient-specific artifacts. In both studies, longer delays were consistently measured with increasing viscosity (Figs. 8A-8B).

Based on the characterization data, it was attempted to mode the capillary flow in channels equipped with timers. First, a quadratic relation was empirically derived that relates the flow delay to the width of the timer through regression analysis (Fig. 2B), which allowed us to predict the flow delay per timer as ${t}_{d} = 156.52 {d}^{2} ,$ (1) where *t_{d}* is the flow delay (in s) and d is the width of the timer (in mm). To model the capillary flow on paper, the Lucas-Washburn equation was employed (2, 32), which depicts the capillary flow penetration distance (*l*) into a porous material at a given time point as $l = \sqrt{\frac{γ r cosθ}{2 μ} t} ,$ (2) where *γ* is the liquid-air surface tension, *r* is the effective pore radius, *θ* is the contact angle, *µ* is the dynamic viscosity of the fluid and *t* is the wicking time. The two models were then combined to estimate the time it takes for the capillary flow to progress in a paper channel equipped with timers. Through tests on channels with different timer arrangements, it was found that the model was highly accurate (Fig. 2E), and it was used for the rest of the work in designing complex LFAs that sequentially routed different flow streams with pre-set delays to perform multi-step chemical processes.

### Example 3: Lateral flow ``assays with built-in signal amplification

Because conventional LFAs are limited to assays that can be performed in a single step, they cannot benefit from already available chemistries to amplify their colorimetric results. Given the ability to coordinate multiple capillary flows on paper, an LFA was designed that was programmed to automatically apply amplifying reagents to enhance the sensitivity. Without loss of generality, an assay was built to detect human chorionic gonadotropin (hCG) (Fig. 3A). The device was built by incorporating a commercial hCG LFA strip with a paper-based low controller followed by sealing it in a 3D-printed enclosure. (See Figs. 10A-10B for the detailed device layout as well as integration and alignment of different layers.) The commercial LFA strip (McKesson, Irving, TX) harbored recognition antibodies conjugated with gold nanoparticles (AuNPs), which produced a visible color change on the test line (Figs. 11A-11G). the flow controller consisted of two timers placed in between three auxiliary inlets, which supplied chemical reagents designed to increase the size of AuNPs for enhanced visibility. Coordinating the flow of solutions loaded into these auxiliary inlets, the timers ensured their orderly and timely arrival to the reaction spot for maximal signal amplification.

First, to confirm automatic, as-programmed routing of liquids within the fabricated device, dye solutions were input in lieu of actual chemical and the time each dye solution arrived at the test spot based on changes in color was recorded (Fig. 3B). The sample (yellow) reached the test spot in approximately 30 seconds, while the signal amplification reagent A (green) and B (red) took 5 and 10 minutes, respectively. Finally, the test spot was washed with DI water (blue) at 20 minutes to quench the amplification for consistent readings (see Figs. 11A-11G).

The assay was tested by loading a 60 µL of urine sample spiked with a known amount of hCG hormone into the inlet 1, and 20 µL of each signal amplification reagents (A: enhancer + activator, B: initiator + buffer), along with 40 µL of deionized (DI) water into auxiliary inlets 2, 3 and 4, respectively. At the completion of the reaction after 20 minutes, the color change on the test line indicated the hCG presence and a positive control line ensured against potential artifacts (Fig. 3C). The intensity of the test line, which was quantified by digital image processing, linearly increased with the hCG concentration changing 0 - 40 mIU/mL (Fig. 3D, red dotted line). Importantly, the limit-of-detection (LOD) was determined to be 2.5 mIU/mL, a level that corresponds to approximately an order of magnitude higher sensitivity than the conventional LFA with its manufacturer-specified LOD of 20 mIU/mL (Fig. 3D, black dotted line). In direct comparisons using matched samples, the conventional LFA signal level at a concentration of 20 mIU/mL hCG was indeed comparable to the signal from our assay obtained at 2.5 mIU/mL (Fig. 3D).

Next, an assay was run without timers in order to test whether the LFA signal was enhanced solely due to the extra reagents introduced to the reaction or from the sequential delivery of those reagents coordinated by embedded timers. First, a urine sample was premixed with two signal amplification reagents (A and B) and DI water, and then processed the mixture using the conventional LFA (Figs. 12A-12B). Premixing reagents resulted in less sensitivity that the timer-controlled assay and gave a nonlinear response 9Fig. 3D, blue dotted line). The signal levels were particularly low (even less than the conventional LFA) for hCG concentration ≤ 20 mIU/mL, likely due to the reducing agent interfering with the antigen/antibody interaction (33), which produced a pronounced effect at lower concentrations of hCG. Next, an assay was run without the timers imprinted on paper (Figs. 13A-13B). Removing timers resulted in premature mixing of reagents in locations external to the test spot, which resulted in limited signal amplification for hCG concentrations ≥ 10 mIU/mL (Fig. 3D, green dotted line). Reduced sensitivity at higher concentrations was due to the decrease in the amount of gold ions still available for signal amplification as some were already reduced by the concurrently running reagents before reaching the test spot. Taken together, the results demonstrated the crucial role played by the imprinted timers on paper in running a multi-step chemical reaction in an optimal manner by coordinating timely delivery of reagents into the reaction.

### Example 4: Automated human DNA purification on paper

To demonstrate the utility of the timers for automatic a sample preparation process, a device was developed for huma DNA purification from bodily fluids (Fig. 4A). The paper device consisted of flow channels defined by the non-delaminating ink and 1 mm-wide timers imprinted with the delaminating ink. The sample collection zone consisted of three DNA extraction spots (pink color) punched out from a Whatman FTA Elute Card, a specialized filter paper pretreated with chemicals for lysing the cells and preserving the extracted DNA. The device was designed to purify the DNA extracted by the FTA card through sequentially delivering solutions to the extraction spots from three different inlets. Specifically, the extraction spots were first subjected to a proteinase K solution for denaturing proteins, then to DI water for washing the denatured proteins, cell debris, and potential inhibitors away and finally to the TE buffer for stabilizing the purified DNA for downstream analysis. This sequence was programmed onto the device by imprinting different numbers of timers on different flow paths.

Automated and timely routing of different fluids within the device was verified by introducing dye solutions from all inlets and measuring their times of arrival to the extraction spots (Fig. 4B). It was observed that after loading the sample (red), it took the proteinase K solution (yellow) approximately 2 minutes to reach the extraction spots. The DI water (green) surpassed a single timer and reach the extraction spots at approximately 6 minutes. Finally, the TE buffer (blue) had to flow through 3 cascaded timers, and it took approximately 17 minutes to reach the extraction spots. With this design, the aim was a total process time of 20 minutes.

Using the device, DNA was extracted from blood, saliva, and urine samples collected from consenting participants according to IRB-approved protocols and quantified the extracted amount of DNA using quantitative polymerase chain reaction (qPCR) (Fig. 4C). In these experiments, , the DNA purification was performed by loading a 3 - 5 µL of the specimen to the sample inlet (1) and 30 µL of proteinase K solution, 90 µL of DI water, and 30 µL of TE buffer to the reagent inlets (2), (3), and (4), respectively. Once the assay was complete, the purified DNA was first recovered from the device by removing the extraction spots and then amplified in a thermal cycler through qPCR. The amount of DNA extracted per each sample was calculated based on a calibration curve constructed from processing standard DNA templates. Also, each sample was processed twice to demonstrate the consistency of the purification process on the device. The qPCR results confirmed the successful extraction of DNA from blood, saliva, and urine samples (Fig. 4C). The extracted DNA amount was found to be the highest from the blood sample and the lowest from the urine sample (Table 1). The fact that qPCR could successfully amplify the DNA from our device confirmed that capillary flow through our delaminating timers did not introduce inhibitor contaminants to the reaction.

**Table 1. The amount of purified DNA by our device and the FTA card.**

| Biological sample | Our Device | | FTA Elute Card | |
|---|---|---|---|---|
| | Ct value | Mean amount of DNA | Ct value | Mean amount of DNA |
| Blood | 11.587 | 25.8 ng | 16.790 | 953.6 pg |
| | 11.949 | | 16.894 | |
| Saliva | 15.022 | 2.3 ng | 20.280 | 63.9 pg |
| | 16.257 | | 22.430 | |
| Urine | 24.430 | 5.8 pg | 24.867 | 4.3 pg |
| | 25.044 | | 25.545 | |

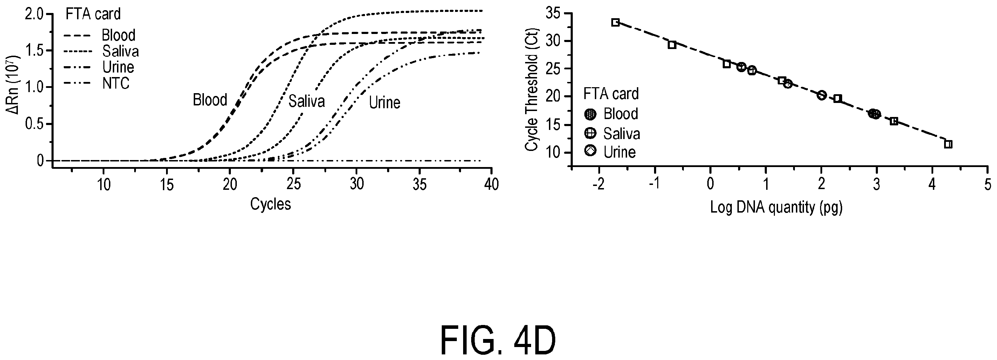

The assay results were compared with those obtained from the conventional DRA card DNA extraction protocol applied on matched samples (Fig. 4D). For all samples, but particularly for blood and saliva samples, our device was able to extract more purified DNA than the FTA card using less sample (5 µL vs. 50 µL) and in less time (20 minutes vs. 3 hours) (Table 1). The higher purified DNA extraction efficiency of our device was likely due to gentler sample handling compared to vortex washing steps required for the conventional processing of FTA cards. Moreover, our device offers a simpler solution (Figs. 14A-14D). that can be carried out by non-specialists to purify DNA samples in POC settings compared to conventional methods, such as filter-based ultracentrifugation, magnetic purification, or processing of FTA cards. Taken together, these results demonstrated the feasibility of performing a molecular biology protocol, traditionally performed using laboratory instrumentation, on a paper-based assay with built-in flow control to run multi-step chemical reactions.

### Example 5: Fabrication of imprinted paper-based device

The assays were fabricated by drawing lines on tissue paper (Kimtech Science^{®} Kimwipes delicate task wipers, Kimberly-Clark, Irving, TX) with two types of permanent inks. The non-delaminating ink (Sharpie^{®} metallic permanent marker, manufacturer part number: SHP 1823889) was used for drawing channel boundaries, and the delaminating ink (Sharpie^{®} ultra-fine point marker, manufacturer part number: SHP 37243) was used to draw timers. Before drawing the lines, one side of the tissue paper was covered with clear tape (manufacturer part number: 3850-60) and the drawing was conducted manually or using an automatic drawing machine (Silhouette CAMEO^{®}), Silhouette America, Lindon, Utah). The machine was only used to fabricate the devices when there was a need to precisely control the line geometry. After drawing all lines with these two markers, the remaining side of the paper was also covered by tape with holes punched to be used as reagent inlets.

### Example 6: Fabrication of device case

The device cases (Fig. 3 and Fig. 4) were designed using Solidworks software (SolidWorks Corp., Waltham, MA) and printed using a 3D printer (ProJet 3510 HD) with VisiJet^{®} M3-X as a structural material. After printing, the device was immersed in mineral oil (Durvet, Blue Springs, MO) at 65°C for the dewaxing of the uncured supporting material, and washed with soapy water, DI water, and ethanol, sequentially. Then, filler primer (Rust-Oleum, Automotive Filler Primer Spray) was sprayed on the device to render the surface smooth. After drying for 10 min, white paint (Rust-Oleum, Painters Touch 2X Spray Paint Matte White) was sprayed and fully dried.

### Example 7: Mathematical model of the timer delay

The model to predict the capillary flow in channels equipped with the timer was established in MATLAB by employing the following two equations as ${t}_{d} = \left(156.52\frac{s}{{mm}^{2}}\right) {d}^{2}$ (1) $l = \sqrt{\frac{γ r cosθ}{2 μ} t}$ (2) , where *γ* = 72 mN/m, *θ* = 81.82, and *µ* = 0.89 mPa·s (34). For the effective pore radius *r,* 6.5 µm was chosen by averaging the pore sizes obtained from microscopy images of the paper. Initially, *l* follows the equation (2) for a duration of *t*₁ until it reaches the first timer. Upon reaching it, *l* remains constant for the assigned delay *t*_{*d*1} according to the equation (1). After the delay, *l* resumes to progress following equation (2) with *t = t - t*_{*d*1} until it reaches the next timer and repeats this process whenever encountering the subsequent timers with *t = t - t*_{*d*1} *- t*_{*d*2} ···.

### Example 8: Human chorionic gonadotropin (hCG) signal amplification

Urine samples were collected according to a protocol approved by Institutional Review Board (IRB) of Georgia Institute of Technology. The hCG positive control (McKesson, Irving, TX) (urine matrix, 200 mIU/mL) was serially diluted with urine to set the concentration from 2.5 to 40 mIU/mL. The samples were directly loaded into the gold nanoparticle (AuNP)-based hCG pregnancy test kit (McKesson, Irving, TX) embedded in our device from the designated sample inlet concurrently with gold enhancement solution (Nanoprobes, Yaphank, NY) and DI water from auxiliary inlets. The hCG - AuNPs complex and unbound AuNPs were captured at the test and control lines, respectively, and produced a red color initially (Figs. 11A-11D). Following the delivery of gold enhancement reagents, AuNPs were enlarged due to the autometallographic reaction, which made the test control line colors darker yielding better contrast and high signal intensity (Figs. 11E-11G).

### Example 9: Quantification of color intensity

The test and control line images captured before and after the signal amplification were individually analyzed using the ImageJ program (http://imagej.nih.gov/ij/). The raw images were first converted into monochrome. Measured gray tones at the test and control lines as well as in the background were then used to calculate the normalized intensity based on the following equation $Normalized intensity = \frac{{I}_{t} - {I}_{b}}{{I}_{c} - {I}_{b}}$ , where *It, I_{c},* and *I_{b}* are the measured gray values at test, control line, and background, respectively.

### Example 10: DNA purification by the conventional FTA card

Whole blood, saliva, and urine samples were each collected from healthy donors according to protocols approved by Institutional Review Board (IRB) of Georgia Institute of Technology. To purify DNA by the FTA card, Whatman FTA Elute Card (GE Healthcare, UK) was used according to manufacturer-provided protocol. 50 µL samples were deposited onto the FTA cards within the circular spot and the samples were dried thoroughly for at least three hours at room temperature. The dried samples on the FTA card were punched out using a 3 mm biopsy punch, and each of them was placed into sterile microcentrifuge tubes for the following washing steps. The punched discs were then triple-washed in 500 µL of DI water through vortexing and centrifugation. Following the final wash, the extracted water was aspirated from the tube, and the washed discs were directly used for qPCR analysis.

### Example 11: Measurement of purified DNA with qPCR

To measure the amount of DNA purified by our device and by the FTA card, qPCR was conducted using Femto Human DNA Quantification Kit (Zymo Research, Orange, CA) on QuantStudio 6 Flex Real-Time PCR System (Applied Biosystems, Foster City, CA). Whenever qPCR was conducted, standards were operated together in duplicates with 10-fold serial dilutions of standard DNA templates (20 ng - 20 fg), which enabled us to construct a calibration curve (Fig. 4C and Fig. 4D) between the amount of human DNA and Ct (cycle threshold) values. The calibration curve was y = -3.55 x + 27.418, and the amount of DNA purified by our device and the FTA card were estimated based on the calibration curve.

### Example 12: Point-of-care toolkit for multiplex molecular diagnosis of SARS-CoV-2, influenza A and B viruses

Severe acute respiratory syndrome-coronavirus 2 (SARS-CoV-2) is still spreading around the globe causing immense public health and socioeconomic problems. As the infection can progress with mild symptoms that that can be misinterpreted as the flu, self-testing methods that can positively identify SARS-CoV-2 are needed to effectively track and prevent the transmission of the virus. This study discusses a point-of-care toolkit for multiplex molecular diagnosis of SARS-CoV-2, influenza A and B viruses in saliva samples. The assay is physically programmed to run a sequence of chemical reactions on a paper substrate and internally generate heat to drive those reactions for an autonomous extraction, purification, and amplification of the viral RNA. This assay can detect SARS-CoV-2 and influenza viruses at concentrations as low as 5 copies/ µL visually from a colorimetric analysis. The capability to autonomously perform a traditionally labor-intensive genetic assay on a disposable platform enables frequent, on demand self-testing, a critical need to track and contain this and future outbreaks.

Since the first report of atypical pneumonia cases in Wuhan, China at the end of 2019, the causative agent has been identified as a new betacoronavirus named severe acute respiratory syndrome-coronavirus 2 (SARS-CoV-2). (Tian et al., (2020); Zhang et al., (2020); Salje et al., (2020); Chinazzi et al., (2020); Menni et al., (2020); Liu et al., (2020)). Due to its rapid spread around the globe, the World Health Organization (WHO) declared the coronavirus disease 2019 (COVID-19) a pandemic on 11 March 2020. Tian et al., (2020); Zhang et al., (2020); Salje et al., (2020); Chinazzi et al., (2020); Menni et al., (2020); Liu et al., (2020)). Besides its efficient transmission, a challenging aspect of the disease is that it could be asymptomatic, or its symptoms could easily be interpreted as due to flu complicating identification of people infected with the virus. (Menni et al., (2020); Liu et al., (2020); Wölfel et al., (2020); Carter et al., (2020); van Kasteren et al., (2020); Udugama et al., (2020)). In fact, the Center for Disease Control and Prevention recently emphasized the importance of differentiating between SARS-CoV-2, influenza A, and B viruses, all of which led to similar respiratory symptoms. As such, there is an urgent need for tests that can positively identify COVID-19 cases to enable timely response.

SARS-CoV-2 can currently be detected by molecularly amplifying the virus RNA sampled through a nasal swab or from saliva as the reverse transcription-quantitative polymerase chain reaction (RT-qPCR) kit is, in fact, widely used to diagnose COVID-19. (Wölfel et al., (2020); Carter et al., (2020); van Kasteren et al., (2020); Udugama et al., (2020)). Although RT-qPCR is a sensitive and accurate method, it has several limitations to be employed in decentralized settings. First, the process typically employs a bulky and expensive thermal cycler leading to a prolonged amplification process. (Zhao et al., (2015); Qian et al., (2020); Becherer et al., (2020); Liu et al., (2015); Gill et al., (2008); Notomi et al., (2000)). Moreover, the RT-qPCR is vulnerable to inhibitors such as body fluids (blood, urine, sweat, *etc*.), which demands painstaking steps for high purity RNA extraction by trained technicians with sophisticated laboratory instruments. (Gill et al., (2008); Notomi et al., (2000); Trinh et al., (2019); Lee et al., *Biosens. Bioelectron.* (2016)). On the other hand, tracking the transmission of a virus with non-specific symptoms require frequent self-testing with standalone kits that can perform molecular amplification in a point-of-care (POC) assay setting.

Isothermal PCR techniques that amplify a specific region of an RNA without the need for thermocycling are attractive for developing POC molecular tests. (Zhao et al., (2015); Qian et al., (2020); Becherer et al., (2020); Liu et al., (2015); Gill et al., (2008); Notomi et al., (2000)). The reverse transcription loop-mediated isothermal amplification (RT-LAMP) is a particularly robust and highly sensitive technique with rapid response. (Notomi et al., (2000); Trinh et al., (2019); Lee et al., Biosens. Bioelectron. (2016); Lee et al., Anal. Chem. (2016), Connelly et al., (2015)). The RT-LAMP employs four to six primers to specifically amplify target RNA sequences at 60 - 72 °C even in the presence of the inhibitors. (Gill et al., (2008); Notomi et al., (2000); Trinh et al., (2019)). Furthermore, the *Bst* polymerase used in the RT-LAMP is highly resistant to high concentrations of inhibitory substances. (Lee et al., Biosens. Bioelectron. (2016); Lee et al., Anal. Chem. (2016); Connelly et al., (2015)). Also, because the *Bst* polymerase exhibits strong reverse transcription activity at elevated temperatures up to 72 °C, the RT-LAMP does not require an additional enzyme for reverse transcription of viral RNA to cDNA. (Lee et al., Anal. Chem. (2016); Zhang, et al. (2019)). While the RT-LAMP was successfully performed on a paper substrate together with colorimetric detection of amplicons, (Connelly et al., (2015); Zhang et al., (2019); Seok et al., (2017); Kaarj, et al., (2018); Kaur et al., (2018); Choi et al., Analyst (2016), Choi et al., Lab Chip (2016), Batule et al., (2020)), these attempts fell short of achieving a standalone, sample-to-answer POC assay as the RNA extraction and amplification still required human intervention or external instruments.

Discussed herein is a fully-integrated POC toolkit for multiplex molecular diagnosis of SARS-CoV-2, influenza A and B viruses from saliva. To extract, amplify, and detect viral RNAs, a paper-based deice was designed with built-in capillary flow regulation, enabled by timers imprinted on flow paths to produce controlled delays. The controlled routing of the capillary flow on paper enabled multiple reagents to be sequentially delivered to the assigned detection spots providing a mechanism for automated extraction of the viral RNA and also a timely introduction of reaction buffer for amplification. The colorimetric RT-LAMP was then automatically performed on all detection spots at a fixed temperature maintained by a calibrated exothermic reaction. The presence of any of the SARS-CoV-2, influenza A and B viruses in the sample were then expose through color changes on corresponding detection spots.

### Results and Discussion

Assay design and operation.

The assay is composed of a multi-layered paper platform for RNA extraction, amplification, and colorimetric detection (ED module) sandwiched between an internal heat module for driving the RT-LAMP process (IH module) at the bottom and a top cover for thermal isolation (Fig. 18A). To generate heat within the assay, the IH module contains calcium oxide (CaO), which reacts with water in an exothermic reaction, and a peripheral wax that functions as a temperature regulator by melting at the desired temperature. The exothermic reaction is triggered through a cellulose strip that carries water from a reservoir at a fixed rate to an otherwise isolated CaO. The chemical reactions are carried out on the paper component (ED module), which routes multiple capillary flow streams to sequentially introduce reagents to the detection spot for RNA extraction and RT-LAMP reaction. The flow paths on the paper were created by simply imprinting with different types of water-insoluble inks when defining channel boundaries or timers to delay flow for a preset duration.

The 3D capillary flow network in the assay was established through stacked layers of paper laminated in between polymer tapes (Fig. 18B). The tape at the top was patterned to create inlets for the sample and RNA extraction reagents. The first paper layer was designed to collect these reagents and deliver them to the sample in sequence with set delays in between. The detection spots were cutouts from a commercial filter paper pretreated with chemicals for cell lysis and preservation of the extracted nucleic acid and were positioned to receive flow from paper layers both above and below. Among those five detection spots, three central ones (purple) were used for extraction and detection of viral RNA from SARS-CoV-2, influenza A and B viruses, respectively. Two peripheral spots (white) were pretreated to serve as positive (PC) and negative controls (NC). The detection spots were also coupled to a second paper layer below, designed to release the RT-LAMP buffer once the viral RNA is extracted. A double-sided tape between the two paper layers not only held them together but also ensured the RNA extraction and the RT-LAMP reagents remained isolated except on the detection spots. The components that gave our assay the multiplex detection capability were the dried primer mixtures specifically targeting SARS-CoV-2, influenza A and B viruses deposited in front of the detection spots to be carried by the RT-LAMP buffer flow.

The assay was physically programmed via features imprinted on paper for automated sequential execution of a set of chemical processes to extract the viral RNA. The fluidic circuit was constructed on paper using two water-insoluble inks with differing affinities to the laminating polymer tape: One (brown) that permanently adhered to the polymer tape to define the flow boundaries and another (green) that gradually delaminated from the polymer tape when wetted to create delay lines (timers) on the flow paths at strategically chosen nodes. Desired delays between flows of different reagents were set by tuning the width of the timers. The assay geometry was optimized to achieve the desired reagent flow sequence with target incubation times through testing with dye solutions (Fig. 18C). In the optimized design, the deposited 20 µL sample (represented by yellow dye) was funneled towards the detection spots as a thin timer blocked the backflow until the next reagent arrived. 30 µL of proteinase K solution (green) reached the detection spots in 3 min, while 60 µL of DI water for washing (red) took 10 minutes to surpass a thick timer. 100 µL of RT-LAMP buffer (blue), flowed on the paper layer below, arrived at the detection spots in 20 min, a time duration set for completing the RNA extraction process.

Once all reagents were delivered to the detection spots, the colorimetric RT-LAMP reaction was initiated by triggering the exothermic reaction in the IH module with water poured into the designated reservoir (Fig. 18D). To minimize thermal loss and evaporation, the inlets were sealed with tape (Fig. 15), and the device was covered for 1 hour to complete the RT-LAMP. At the end of the process, amplicons from a specific gene of the target virus were detected from a change in the color of the detection spots. Among the three central detection spots, the one next to the PC spot targeted the SARS-CoV-2, while the middle spot and the next one screened for influenza A and B viruses, respectively. A saliva sample was only scored positive for any one of the three viruses if there was a color change (from pink to yellow) in the corresponding detection spot along with a color change in the PC spot and no change in NC spot color to ensure against artifacts.

Characterization and optimization of viral RNA extraction.

To determine the optimum substrate for building the detection spots in our assay, the performance of commercially available RNA sample papers was compared. Because both RNA extraction and amplification were to be performed on the detection spots, our experiments aimed to identify a medium that was not only efficient in extracting the virus RNA but also compatible with the colorimetric RT-LAMP process. Three commercial nucleic acid sampling papers were characterized along with cellulose and nitrocellulose paper as controls using samples prepared by spiking inactivated SARS-CoV-2 intact virus particles into saliva samples collected from healthy donors under Georgia-Tech IRB approved protocol. The amount of viral RNA on each paper was quantified by subjecting 2.5 mm-diameter discs punched out from papers to real-time RT-LAMP. The FTA Elute card (Whatman) required the least number of amplification cycles for a detectable RNA signal yielding the smallest cycle threshold (Ct) value of 24.9, while measured Ct values obtained from FTA card and RNASound care were 41.02 and 37.15, respectively (Fig. 19A). The cellulose and nitrocellulose papers did not show any amplification since neither of those incorporated chemicals were needed for RNA extraction. Comparing the tested substrates based on ΔCt values, obtained by subtracting the Ct value from that of the PC, it was concluded that the FTA Elute card could extract viral RNA with the highest efficiency and was also compatible with RT-LAMP reaction (Fig. 19B).

The FTA Elute card was tested when integrated into the assay as detection spots and the RNA extraction performance was investigated. The paper device was programmed to automatically deliver the same set of reagents to the FTA Elute card spots. Once the SARS-CoV-2-spiked saliva samples were processed on the device, the extracted viral RNA was recovered by separating the spots from the device and amplified through real-time RT-LAMP. The results demonstrated successful extraction of SARS-CoV-2 RNA from the sample on the device (Fig. 19C). The Ct value was measured to be similar to those achieved by manual processing of the saliva sample with the FTA Elute card following the manufacturer's instructions (24.9 vs. 28.07), showing the automated RNA extraction coordinate by the built-in flow control within the device was equally efficient.

Autonomous RNA amplification and colorimetric detection.

To amplify the extracted and purified viral RNA directly on the device without relying on an external process, heat was internally generated to carry out the RT-LAMP reaction through an exothermic reaction:

CaO (*s*) *+* H₂O (*l*) → Ca(OH)₂ (*s*) Δ*Hᵣ* = -65.2 kJ/mol

In a reservoir below the extracted RNA, a CaO powder was made to react with water that was continuously carried from the neighboring reservoir via a cellulose paper strip (Fig. 20A). While the exothermic reaction produced sufficient heat, it failed to provide an isothermal environment with the device temperature overshooting to levels (approximately 90 °C) that could inactivate the Bst polymerase (Fig. 20B). In an attempt to regulate the device temperature at a level optimal for the RT-LAMP reaction (60 - 72 °C), the reaction chamber was surrounded with candelilla wax that melted at approximately 68 - 72 °C. Melting of the wax was confirmed to not only rectify temperature spikes but also store the heat to be dissipated at the desired temperature (Fig. 20B). With this setup, it took approximately 10 mins to reach the desired temperature range, which was well before the 20 mins mark for the arrival of RT-LAMP buffer at the detection spot. Furthermore, the isothermal conditions could be maintained for the desired duration depending on the starting amount of CaO in the reaction chamber (e.g., 5 g of CaO powder was found to produce approximately 68 °C for > 90 mins) (Fig. 20C). As a functional test, the designed IH module was employed to perform a conventional tube-based RT-LAMP reaction and confirmed successful RNA amplification (Figs. 19A-19C).

Next, the colorimetric output of the assay was investigated to determine the amount of time required for the RNA amplification produce a signal that could reliably be detected. Saliva samples spiked with 1,000 copies of SARS-CoV-2 were processed on the complete assay and the gradual color changes on the SARS-CoV-2 detection spot could visually be observed over time (Fig. 20D). Beyond 40 mins, the SARS-CoV-2 spot color changed from pink to yellow as well as the PC spot color. On the other hand, the NC spot remained to be pink with no observable color changes validating the amplification reaction as the source of the colorimetric signal. The amplification reaction on each spot was independently verified by adding a fluorescent intercalating dye to the reaction (Figs. 17A-17C). Normalized color intensities at the PC, NC, and SARS-CoV-2 spots were calculated from camera images via digital image processing (Fig. 20E). The measurements showed the colors began changing after 20 mins right when the RNA amplification started with the arrival of the RT-LAMP buffer. The largest change in the colorimetric signal was between 20 - 40 mins followed by increasingly diminishing gains. Based on these results, 60 minutes was sufficiently long to reliably lead the assay results.

Multiplex detection of SARS-CoV-2, influenza A and B viruses.

Next, using the optimized POC toolkit and the assay protocol was tested and for multiplex detection of SARS-CoV-2, influenza A and B viruses in saliva samples. Samples spiked with controlled amounts of either one of those virus types were processed on the device with no external interference except for depositing the saliva sample, reagents, and water into the designated ports at the onset. To determine the limit of detection for the assay, samples were processed with virus concentrations varying from 10 to 10⁵ copies per 20 µL of saliva and evaluated color changes in the detection spots after 60 minutes (Fig. 21A). For all samples tested, a concentration of 10 virus copies was found to be below the limit of detection of our assay as the detection spot color was indistinguishable from that of the negative control. However, 100 copies of either one of the three viruses produced a measurable change in the color intensity of the corresponding detection spot (Fig. 21B). Increasing the virus concentration to 1,000 copies led to drastic changes in the spot color intensities, while further increases in virus amount produce less pronounced increases in the colorimetric signal.

The assay was also successful in discriminating SARS-CoV-2 from the influenza A and B viruses. The colorimetric signals were produced exclusively from the detection spots corresponding to the target virus type with no noticeable cross-talk between detection spots reserved for a different virus type (Fig. 21B). Furthermore, experiments with control samples with no spiked viruses did not produce false positive signals further validating the specificity of our assay. These results also demonstrate the feasibility of scaling the technology for multiplex detection of more virus types or even different strains of SARS-CoV-2 on the same assay.

### Conclusion

Symptoms due to COVID-19 could easily be mixed with those from the common flu. A POC assay was developed that can perform multiplex molecular detection of SARS-CoV-2, influenza A and B viruses down to 5 copies/µL in human saliva samples. Despite the purposely frugal nature of the assay presented in this work, multi-step, multi-reagent chemical reactions for virus RNA extraction, purification and amplification could all be autonomously performed within the device without compromising on the sensitivity or specificity of any of those reactions. This work contributes to the efforts in addressing the critical need for POC testing to positively identify COVID-19 infections by simplifying conventionally complex and labor-intensive genetic assays.

### Materials and Methods

Fabrication of the extraction and detection module (ED module).

To fabricate the first and second paper layers, the tissue paper (Kimtech Science^{®} Kimwipes delicate task wipers, Kimberly-Clark, Irving, TX) was immersed in a blocking buffer (0.25% (v/v) Tween-20 and 5 % (w/v) bovine serum albumin in PBS) for 1 hour. After washing in PBS and fully drying, one side of the tissue paper (Kimtech Science^{®} Kimwipes delicate task wipers, Kimberly-Clark, Irving, TX) was covered with clear tape, followed by line drawing using an automatic drawing machine (Silhouette CAMEO^{®}, Silhouette America, Lindon, Utah) with two water-insoluble inks. Sharpie^{®} metallic permanent marker was used for drawing the channel boundaries. Sharpie^{®} ultra-fine point marker was used to create timers. Each of 5 µL primer mixtures targeting SARS-CoV-2, influenza A and B virus were dropped and dried on the assigned locations in the second paper layers. The double-sided tape was then attached for aligning and bonding the first and second layer, and five holes as the location of detection spots were punched using a 2.5 mm biopsy punch. To prepare the detection spots, five spots were punched out from a Whatman FTA Elute Card using a 2.5 mm biopsy punch. Three spots were placed in the central three holes in the aligned layers. The two remaining spots were washed and located in the remaining holes at both ends, serving as PC and NC. 1,000 copies of the SARS-CoV-2 PC template (N gene) and SARS-CoV-2 primer (New England BioLabs, MA) were pre-dried on the PC spot. The primer mixtures targeting SARS-CoV-2, influenza A and B virus were dried on the NC spot. After placing the detection spots in the designated locations, the device was sealed with clear tape on the top (with four inlet holes) and bottom.

Capillary flow control in paper.

The capillary flow on a laminated paper can be controlled by drawing patterns on the paper with two water-insoluble inks with different levels of hydrophobicity. The region patterned with a more hydrophobic ink (Sharpie^{®} metallic permanent marker) is permanently tethered to the sheath tape with no void formation, acting as channel boundaries to guide the flow. The region patterned with the less hydrophobic ink (Sharpie^{®} ultra-fine point marker) eventually separates from the sheath tape when wetted, leaving the void to be formed at the interface between the patterned region and the tape. The flow is delayed until the void becomes large enough to allow the flow to proceed, acting as a timer. By imprinting and modifying the timer's geometry (e.g., thickness or number) at strategic nodes, different flow delays are assigned to different reagents, enabling sequential flow into the desired locations following a programmed sequence.

Fabrication of the internal heat module (IH module).

All of the IH module components were designed using Solidworks software (SolidWorks Corp., Waltham, MA). The designs were printed using a 3D printer (ProJet 3510 HD) with VisiJet^{®} M3-X as a structural material. To dewax the uncured supporting material, the printed components were soaked in mineral oil (Durvet, Blue Springs, MO) at 65 °C with sonication for 1 hour, and washed with soapy water, DI water, and ethanol, sequentially. After drying, the filler primer (Rust-Oleum, Automotive Filler Primer Spray) was sprayed on the components to render the surface smooth. After drying for 10 min, white paint (Rust-Oleum, Painters Touch 2X Spray Paint Matte White) was sprayed on them and fully dried. The pellets of candelilla wax (Tm = 68 - 72 °C) were melted in a laboratory oven at 80 °C and pour the melted wax into the wax reservoir in the IH module. After solidifying the wax at room temperature, the strip of cellulose paper was located across the reservoirs of water and CaO powder, and 5 g of CaO powder was loaded into the center reservoir.

Characterization of commercial RNA sampling papers.

To compare the RNA extraction performance and compatibility with RT-LAMP, three commercial nucleic-acid sampling papers (Whatman FTA Card, Whatman FTA Elute Card (GE Healthcare, UK), and RNASound RNA Sampling cards (FortiusBio LLC, CA)) were tested with cellulose and nitrocellulose paper as controls. 50 µL of saliva samples spiked with an inactivated SARS-CoV-2 intact virus particle were applied onto each paper and the samples were air-dried for more than three hours. The dried samples on paper were punched out using a 2.5 mm biopsy punch, and each of them was placed into sterile microcentrifuge tubes for the following washing steps. The punched discs were then triple-washed in 500 µL of DI water through gentle vortexing and centrifugation. Following the final wash, the washed discs were used for real-time RT-LAMP which was conducted on QuantStudio 6 Flex Real-Time PCR System (Applied Biosystems, Foster City, CA). Whenever the reaction was conducted, the PC and NC were operated together.

Preparation of real-time/colorimetric RT-LAMP buffer.

The reaction buffer for real-time/colorimetric RT-LAMP was obtained from SARS-CoV-2 Rapid Colorimetric LAMP Assay Kit (New England BioLabs, MA). For real-time RT-LAMP, the buffer consists of 12.5 µL of WarmStart^{®} colorimetric LAMP 2X master mix with UDG, 2.5 µL of guanidine hydrochloride (0.4 M), 2.5 µL of primer mixture, 3.5 µL of nuclease-free water, and 2 µL of fluorescent intercalating dye (1 µM, SYTO 9 green fluorescent nucleic acid stain, ThermoFisher Scientific, MA). Either 2 µL of SARS-CoV-2 PC or nuclease-free water were added to the prepared buffer for PC and NC reaction. For testing RNA sampling papers, the punched papers and additional 2 µL of nuclease-free water were added to the prepared buffer. For colorimetric RT-LAMP, the buffer consists of 12.5 µL of WarmStart^{®} colorimetric LAMP 2X master mix with UDG, 2.5 µL of guanidine hydrochloride (0.4 M), and 10 µL of nuclease-free water. The principle for colorimetric detection is based on sensing of pH change. The protons are released during the RT-LAMP as the by-products and are accumulated in the buffer solution, which decreases pH significantly. The pH decrease in the buffer can visually be detected using pH-sensitive dyes (e.g., phenol red). (Tanner et al., Zhang et al., Rapid Molecular Detection of SARS-CoV-2.)

Primer mixtures targeting SARS-CoV-2, influenza A and B viruses.

The primer mixture targeting SARS-CoV-2 was obtained from SARS-CoV-2 Rapid Colorimetric LAMP Assay Kit. The primer sequences targeting influenza A and B viruses were referred to previous literature (Kubo et al., Mahony et al.) and synthesized by oligo synthesis service (ThermoFisher Scientific, CA); each primer concentration was 8 µM forward/reverse inner primers, 4 µM loop primers, and 2 µM forward/reverse outer primers. 5 µL of each primer mixture was dried on the assigned locations in the second paper layer. All primer sequences are listed in Table 2 and the accompanying sequence listing.

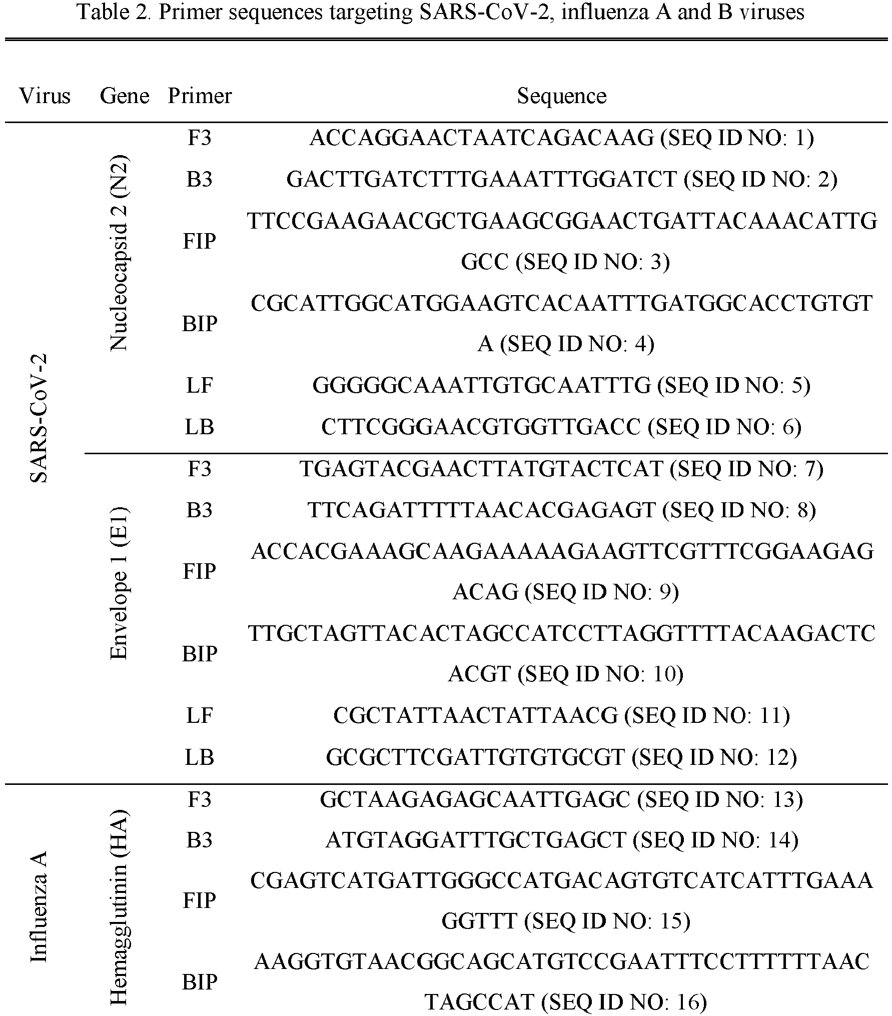

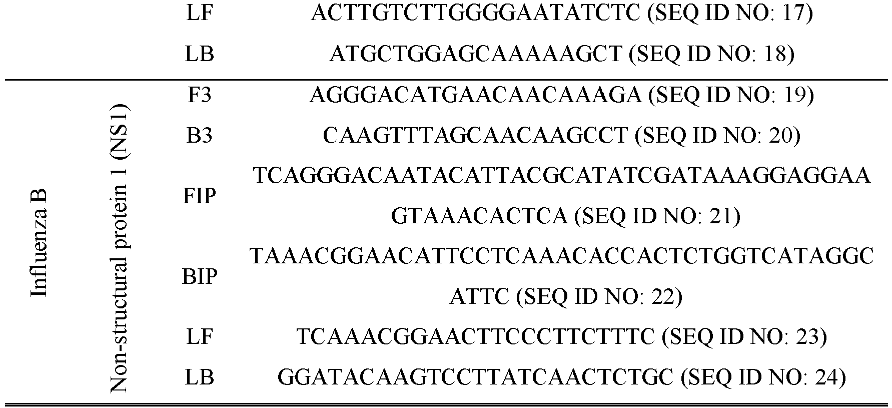

### Virus sample preparation.

The inactivated viruses of SARS-CoV-2 (NATtrol^{™} SARS-CoV-2 External Run Controls), influenza A (NATtrol^{™} Influenza A H1N1 External Run Controls) and B (NATtrol^{™} Influenza B External Run Controls), which have been chemically modified to render them noninfectious but are intact virus particles, were purchased from ZeptoMetrix Corporation (Buffalo, NY, USA). Saliva samples were collected from healthy donors according to protocols approved by Institutional Review Board (IRB) of Georgia Institute of Technology. To estimate virus copy number in samples, each undiluted virus sample was spiked to the collected saliva and the spiked samples were processed using the ED module. The detection spots were taken out from the device by tweezer and the extracted viral RNA was eluted in 20 µL of nuclease-free water. The concentration of the eluted RNA was quantified using NanoDrop 1000 Spectrophotometer (ThermoFisher Scientific, Wilmington, DE, USA) and converted copy number based RNA genome size of each virus: 30 kb for SARS-CoV-2, 13.5 kb for influenza A, and 14.5 kb for influenza B virus. Then, the spiked samples were serially diluted to control the virus copy number (10⁵ - 10 copies per 20 µL).

### Quantification of color intensity.

The images of the five detection spots were captured after the RT-LAMP reaction and were individually analyzed using the ImageJ program (http://imagej.nih.gov/ij/). The raw images were split into separate RGB channels, and the green channel image was used for quantification; the green is a complementary color of pink-colored RT-LAMP buffer. Measured color intensities in each spot were divided with the intensity in the NC spot for normalization.

### Example 13: Modulation of capillary flow delay by different materials

In addition to timer geometries, different material properties of the timer can contribute to manipulating capillary flow delay. The following equation represents the required energy to fully delaminate one layer from another substrate layer when two layers are immersed in liquid. $Δ E = \left({G}_{ts}-γ\left({cosθ}_{1}+{cosθ}_{2}\right)\right) \left(1-ρ\right) b ∗ l$ where, *Gₜₛ* is interfacial adhesion energy in dry air, *γ* is liquid surface tension, *θ*₁ and *θ*₂ are contact angles of two layers, *ρ* is porosity, *b* is the width of the layer, and *l* is the peeling distance. From the equation, the contact angle is one of the material properties that can affect the delamination energy, where higher hydrophobicity (e.g., higher contact angle) can increase the delamination energy. Thus, if different delaminating inks have different contact angles, the timers created by these inks can produce different flow delays even if their geometry remains the same.

### Example 14: Effect of different inks/materials on capillary flow delay

Three different color delaminating inks (green, red, and black) were chosen and the contact angle of the marked paper which was painted with these three inks was measured (Fig. 22A). It was observed that each ink showed a different contact angle, green (approximately 109°) < red (approximately 115°) < black (approximately 127°). The timers drawn with these inks showed different capillary flow delays; the higher the contact angle, the longer the flow delay (Fig. 22B). But this does not necessarily mean the contact angle will always dominate the delay function. It is possible that significant differences in interfacial adhesion energy between tape and paper substrate can dominate the delay behavior.

### Example 15: Sequential delivery of multiple solutions

Multiple solutions were sequentially delivered using different colored timers on a test platform where four inlet branches merged into a single channel (Figs. 23A-23B). Different colored timers were placed in each branch to differentially delay the flow between different branches: the first branch has no timer, the second branch has a green timer, the third branch has a red timer, and the fourth branch has a black timer. The four dyed solutions (water) simultaneously introduced to the test platform reach the shared channel sequentially, with the flow from the branch without a timer arriving first (yellow) and the one from the branch equipped with a black timer arriving last (blue) (Figs. 23A-23B). In contrast, a control experiment on an otherwise identical device with no timers resulted in the simultaneous arrival of all dyed solutions to the shared channel.

### Example 16: Characterization of different color delaminating inks

The capillary flow delay from these three inks was characterized by changing the timer geometry. The length of delays generated by three colored timers of different widths (0.5 to 1.5 mm) was measured in independent experiments by monitoring the progress of dyed solutions through these timers (Fig. 24A). The delays introduced by three different colored timers increased with their width nonlinearly (Fig. 24B) as the 0.5-mm-wide timer resulted in an average flow delay of 37.6 s (green), 70 s (red), and 94.1 s (black), while a 1.5-mm-wide timer produced an average flow delay of approximately 6 minutes (green), approximately 8.5 minutes (red), and approximately 12 minutes (black). Cascaded timer arrangements (1 to 8 timers) were also studied to achieve linear control over the flow delay (Fig. 24C). The dependence of the total flow delay on the number of timers was linear in all difference colored timers (Fig. 24D).

### Example 17: Effect of sheath tape on capillary flow delay

Different types of sheath tapes have different contact angles (*θ*) on the adhesive side which can also contribute to modulating delamination energy (e.g., capillary flow delay). To investigate the effect of the sheath tape on the delay, the contact angles of the six different sheath tapes were measured (Fig. 25A) and the flow delay produced by a green 0.5 mm-timer laminated to the corresponding tape was also measured (Fig. 25B). There was a correlation of the flow delay with the hydrophobicity of the tape, where the flow delay increased as the tape surface was more hydrophobic. Furthermore, when the tape surfaces were treated with a corona discharge to render the surface more hydrophilic (Fig. 25A), the flow delays were decreased as the same trend with the decreasing of the contact angle depending on the types of tape (Fig. 25B). Tape 2 which didn't show a significant change in contact angle after corona treatment remains the same flow delay.

### Example 18: Active control of delaminating timer

Once the substrate detaches from the laminating tape in the timer, the flow through the timer could be stopped by closing the gap between wo layers by applying force by using magnets as shown in Figs. 23A-23B. The flow can also be released on demand by separating the two materials to recreate the gap. This would effectively provide an active control on the capillary flow.

### References

Tian, H.; Liu, Y.; Li, Y.; Wu, C. H.; Chen, B.; Kraemer, M. U. G.; Li, B.; Cai, J.; Xu, B.; Yang, Q.; et al. An Investigation of Transmission Control Measures during the First 50 Days of the COVID-19 Epidemic in China. Science. 2020, 368, 638-642.
Zhang, J.; Litvinova, M.; Liang, Y.; Wang, Y.; Wang, W.; Zhao, S.; Wu, Q.; Merler, S.; Viboud, C.; Vespignani, A.; et al. Changes in Contact Patterns Shape the Dynamics of the COVID-19 Outbreak in China. Science. 2020, 368, 1481-1486.
Salje, H.; Kiem, C. T.; Lefrancq, N.; Courtejoie, N.; Bosetti, P.; Paireau, J.; Andronico, A.; Hozé, N.; Richet, J.; Dubost, C. L.; et al. Estimating the Burden of SARS-CoV-2 in France. Science. 2020, 369, 208-211.
Chinazzi, M.; Davis, J. T.; Ajelli, M.; Gioannini, C.; Litvinova, M.; Merler, S.; Pastore y Piontti, A.; Mu, K.; Rossi, L.; Sun, K.; et al. The Effect of Travel Restrictions on the Spread of the 2019 Novel Coronavirus (COVID-19) Outbreak. Science. 2020, 368, 395-400
Menni, C.; Valdes, A. M.; Freidin, M. B.; Sudre, C. H.; Nguyen, L. H.; Drew, D. A.; Ganesh, S.; Varsavsky, T.; Cardoso, M. J.; El-Sayed Moustafa, J. S.; et al. Real-Time Tracking of Self-Reported Symptoms to Predict Potential COVID-19. Nat. Med. 2020, 26, 1037-1040.
Liu, Y.; Ning, Z.; Chen, Y.; Guo, M.; Liu, Y.; Gali, N. K.; Sun, L.; Duan, Y.; Cai, J.; Westerdahl, D.; et al. Aerodynamic Analysis of SARS-CoV-2 in Two Wuhan Hospitals. Nature 2020, 582, 557-560.
Wölfel, R.; Corman, V. M.; Guggemos, W.; Seilmaier, M.; Zange, S.; Müller, M. A.; Niemeyer, D.; Jones, T. C.; Vollmar, P.; Rothe, C.; et al. Virological Assessment of Hospitalized Patients with COVID-2019. Nature 2020, 581, 465-469.
Carter, L. J.; Garner, L. V.; Smoot, J. W.; Li, Y.; Zhou, Q.; Saveson, C. J.; Sasso, J. M.; Gregg, A. C.; Soares, D. J.; Beskid, T. R.; et al. Assay Techniques and Test Development for COVID-19 Diagnosis. ACS Cent. Sci. 2020, 6, 591-605.
van Kasteren, P. B.; van der Veer, B.; van den Brink, S.; Wijsman, L.; de Jonge, J.; van den Brandt, A.; Molenkamp, R.; Reusken, C. B. E. M.; Meijer, A. Comparison of Seven Commercial RT-PCR Diagnostic Kits for COVID-19. J. Clin. Virol. 2020, 128, 104412.
Udugama, B.; Kadhiresan, P.; Kozlowski, H. N.; Malekjahani, A.; Osborne, M.; Li, V. Y. C.; Chen, H.; Mubareka, S.; Gubbay, J. B.; Chan, W. C. W. Diagnosing COVID-19: The Disease and Tools for Detection. ACS Nano 2020, 14, 3822-3835.
Centers for Disease Control and Prevention. CDC's Diagnostic Multiplex Assay for Flu and COVID-19 at Public Health Laboratories and Supplies. https://www.cdc.gov/coronavirus/2019-ncov/lab/multiplex.html (2020).
Zhao, Y.; Chen, F.; Li, Q.; Wang, L.; Fan, C. Isothermal Amplification of Nucleic Acids. Chem. Rev. 2015, 115, 12491-12545.
Qian, J.; Boswell, S. A.; Chidley, C.; Lu, Z. xiang; Pettit, M. E.; Gaudio, B. L.; Fajnzylber, J. M.; Ingram, R. T.; Ward, R. H.; Li, J. Z.; et al. An Enhanced Isothermal Amplification Assay for Viral Detection. Nat. Commun. 2020, 11, 5920.
Becherer, L.; Borst, N.; Bakheit, M.; Frischmann, S.; Zengerle, R.; Von Stetten, F. Loop-Mediated Isothermal Amplification (LAMP)-Review and Classification of Methods for Sequence-Specific Detection. Anal. Methods 2020, 12, 717-746.
Liu, W.; Dong, D.; Yang, Z.; Zou, D.; Chen, Z.; Yuan, J.; Huang, L. Polymerase Spiral Reaction (PSR): A Novel Isothermal Nucleic Acid Amplification Method. Sci. Rep. 2015, 5, 12723.
Gill, P.; Ghaemi, A. Nucleic Acid Isothermal Amplification Technologies - A Review. Nucleosides, Nucleotides and Nucleic Acids 2008, 27, 224-243.
Notomi, T.; Okayama, H.; Masubuchi, H.; Yonekawa, T.; Watanabe, K.; Amino, N.; Hase, T. Loop-Mediated Isothermal Amplification of DNA. Nucleic Acids Res. 2000, 28, E63.
Trinh, T. N. D.; Lee, N. Y. A Foldable Isothermal Amplification Microdevice for Fuchsin-Based Colorimetric Detection of Multiple Foodborne Pathogens. Lab Chip 2019, 19, 1397-1405.
Lee, D.; Kim, Y. T.; Lee, J. W.; Kim, D. H.; Seo, T. S. An Integrated Direct Loop-Mediated Isothermal Amplification Microdevice Incorporated with an Immunochromatographic Strip for Bacteria Detection in Human Whole Blood and Milk without a Sample Preparation Step. Biosens. Bioelectron. 2016, 79, 273-279.
Lee, D.; Shin, Y.; Chung, S.; Hwang, K. S.; Yoon, D. S.; Lee, J. H. Simple and Highly Sensitive Molecular Diagnosis of Zika Virus by Lateral Flow Assays. Anal. Chem. 2016, 88, 12272-12278.
Connelly, J. T.; Rolland, J. P.; Whitesides, G. M. "Paper Machine" for Molecular Diagnostics. Anal. Chem. 2015, 87, 7595-7601.
Zhang, H.; Xu, Y.; Fohlerova, Z.; Chang, H.; Iliescu, C.; Neuzil, P. LAMP-on-a-Chip: Revising Microfluidic Platforms for Loop-Mediated DNA Amplification. Trends Anal. Chem. 2019, 113, 44-53.
Seok, Y.; Joung, H. A.; Byun, J. Y.; Jeon, H. S.; Shin, S. J.; Kim, S.; Shin, Y. B.; Han, H. S.; Kim, M. G. A Paper-Based Device for Performing Loop-Mediated Isothermal Amplification with Real-Time Simultaneous Detection of Multiple DNA Targets. Theranostics 2017, 7, 2220-2230.
Kaarj, K.; Akarapipad, P.; Yoon, J. Y. Simpler, Faster, and Sensitive Zika Virus Assay Using Smartphone Detection of Loop-Mediated Isothermal Amplification on Paper Microfluidic Chips. Sci. Rep. 2018, 8, 12438.
Kaur, N.; Toley, B. J. Paper-Based Nucleic Acid Amplification Tests for Point-of-Care Diagnostics. Analyst 2018, 143, 2213-2234.
Choi, J. R.; Hu, J.; Gong, Y.; Feng, S.; Wan Abas, W. A. B.; Pingguan-Murphy, B.; Xu, F. An Integrated Lateral Flow Assay for Effective DNA Amplification and Detection at the Point of Care. Analyst 2016, 141, 2930-2939.
Choi, J. R.; Hu, J.; Tang, R.; Gong, Y.; Feng, S.; Ren, H.; Wen, T.; Li, X. J.; Wan Abas, W. A. B.; Pingguan-Murphy, B.; et al. An Integrated Paper-Based Sample-to-Answer Biosensor for Nucleic Acid Testing at the Point of Care. Lab Chip 2016, 16, 611-621.
Batule, B. S.; Seok, Y.; Kim, M. G. Paper-Based Nucleic Acid Testing System for Simple and Early Diagnosis of Mosquito-Borne RNA Viruses from Human Serum. Biosens. Bioelectron. 2020, 151, 111998.
Tanner, N. A.; Zhang, Y.; Evans, T. C. Visual Detection of Isothermal Nucleic Acid Amplification Using PH-Sensitive Dyes. Biotechniques 2015, 58, 59-68.
Zhang, Y.; Odiwuor, N.; Xiong, J.; Sun, L.; Nyaruaba, R. O.; Wei, H.; Tanner, N. Rapid Molecular Detection of SARS-CoV-2 (COVID-19) Virus RNA Using Colorimetric LAMP. Preprint at https://www.medrxiv.org/content/10.1101/2020.02.26.20028373v1 (2020).
Kubo, T.; Agoh, M.; Mai, L. Q.; Fukushima, K.; Nishimura, H.; Yamaguchi, A.; Hirano, M.; Yoshikawa, A.; Hasebe, F.; Kohno, S.; et al. Development of a Reverse Transcription-Loop-Mediated Isothermal Amplification Assay for Detection of Pandemic (H1N1) 2009 Virus as a Novel Molecular Method for Diagnosis of Pandemic Influenza in Resource-Limited Settings. J. Clin. Microbiol. 2010, 48, 728-735.
Mahony, J.; Chong, S.; Bulir, D.; Ruyter, A.; Mwawasi, K.; Waltho, D. Multiplex Loop-Mediated Isothermal Amplification (M-LAMP) Assay for the Detection of Influenza A/H1, A/H3 and Influenza B Can Provide a Specimen-to-Result Diagnosis in 40min with Single Genome Copy Sensitivity. J. Clin. Virol. 2013, 58, 127-131.
E. K. Sackmann, A. L. Fulton, D. J. Beebe, The present and future role of microfluidics in biomedical research. Nature. 507, 181-189 (2014).
M. M. Gong, D. Sinton, Turning the Page: Advancing Paper-Based Microfluidics for Broad Diagnostic Application. Chem. Rev. 117, 8447-8480 (2017).
A. K. Yetisen, M. S. Akram, C. R. Lowe, Paper-based microfluidic point-of-care diagnostic devices. Lab Chip. 13, 2210-2251 (2013).
G. A. Posthuma-Trumpie, J. Korf, A. Van Amerongen, Lateral flow (immuno)assay: Its strengths, weaknesses, opportunities and threats. A literature survey. Anal. Bioanal. Chem. 393, 569-582 (2009).
D. Lee et al., Simple and highly sensitive molecular diagnosis of Zika virus by lateral flow assays. Anal. Chem. 88, 12272-12278 (2016).
R. Banerjee, A. Jaiswal, Recent advances in nanoparticle-based lateral flow immunoassay as a point-of-care diagnostic tool for infectious agents and diseases. Analyst. 143, 1970-1996 (2018).
T. Kong et al., A fast, reconfigurable flow switch for paper microfluidics based on selective wetting of folded paper actuator strips. Lab Chip. 17, 3621-3633 (2017).
M. S. Verma et al., Sliding-strip microfluidic device enables ELISA on paper. Biosens. Bioelectron. 99, 77-84 (2018).
E. Fu et al., Two-dimensional paper network format that enables simple multistep assays for use in low-resource settings in the context of malaria antigen detection. Anal. Chem. 84, 4574-4579 (2012).
B. J. Toley et al., Tunable-Delay Shunts for Paper Microfluidic Devices. Anal. Chem. 85, 11545-11552 (2013).
B. Lutz et al., Dissolvable fluidic time delays for programming multi-step assays in instrument-free paper diagnostics. Lab Chip. 13, 2840-2847 (2013).
C. M. Cheng et al., Paper-based ELISA. Angew. Chemie - Int. Ed. 49, 4771-4774 (2010).
A. W. Martinez, S. T. Phillips, M. J. Butte, G. M. Whitesides, Patterned paper as a platform for inexpensive, low-volume, portable bioassays. Angew. Chemie - Int. Ed. 46, 1318-1320 (2007).
A. W. Martinez, S. T. Phillips, G. M. Whitesides, Three-dimensional microfluidic devices fabricated in layered paper and tape. Proc. Natl. Acad. Sci. U. S. A. 105, 19606-19611 (2008).
Y. Lu, W. Shi, L. Jiang, J. Qin, B. Lin, Rapid prototyping of paper-based microfluidics with wax for low-cost, portable bioassay. Electrophoresis. 30, 1497-1500 (2009).
Y. Lu, W. Shi, J. Qin, B. Lin, Fabrication and characterization of paper-based microfluidics prepared in nitrocellulose membrane by Wax printing. Anal. Chem. 82, 329-335 (2010).
D. A. Bruzewicz, M. Reches, G. M. Whitesides, Low-cost printing of poly(dimethylsiloxane) barriers to define microchannels in paper. Anal. Chem. 80, 3387-3392 (2008).
X. Li, J. Tian, G. Garnier, W. Shen, Fabrication of paper-based microfluidic sensors by printing. Colloids Surfaces B Biointerfaces. 76, 564-570 (2010).
K. Abe, K. Suzuki, D. Citterio, Inkjet-printed microfluidic multianalyte chemical sensing paper. Anal. Chem. 80, 6928-6934 (2008).
K. Abe, K. Kotera, K. Suzuki, D. Citterio, Inkjet-printed paperfluidic immuno-chemical sensing device. Anal. Bioanal. Chem. 398, 885-893 (2010).
X. Li, J. Tian, T. Nguyen, W. Shen, Paper-based microfluidic devices by plasma treatment. Anal. Chem. 80, 9131-9134 (2008).
X. Li, J. Tian, W. Shen, Progress in patterned paper sizing for fabrication of paper-based microfluidic sensors. Cellulose. 17, 649-659 (2010).
G. Chitnis, Z. Ding, C. L. Chang, C. A. Savran, B. Ziaie, Laser-treated hydrophobic paper: An inexpensive microfluidic platform. Lab Chip. 11, 1161-1165 (2011).
K. L. Dornelas, N. Dossi, E. Piccin, A simple method for patterning poly(dimethylsiloxane) barriers in paper using contact-printing with low-cost rubber stamps. Anal. Chim. Acta. 858, 82-90 (2015).
E. Fu, B. Lutz, P. Kauffman, P. Yager, Controlled reagent transport in disposable 2D paper networks. Lab Chip. 10, 918-920 (2010).
A. Apilux, Y. Ukita, M. Chikae, O. Chailapakul, Y. Takamura, Development of automated paper-based devices for sequential multistep sandwich enzyme-linked immunosorbent assays using inkjet printing. Lab Chip. 13, 126-135 (2013).
J. H. Shin, J. Park, S. H. Kim, J. K. Park, Programmed sample delivery on a pressurized paper. Biomicrofluidics. 8, 0541211 (2014).
X. Li, P. Zwanenburg, X. Liu, Magnetic timing valves for fluid control in paper-based microfluidics. Lab Chip. 13, 2609-2614 (2013).
M. Fratzl et al., Magnetic Two-Way Valves for Paper-Based Capillary-Driven Microfluidic Devices. ACS Omega. 3, 2049-2057 (2018).
H. Chen, J. Cogswell, C. Anagnostopoulos, M. Faghri, A fluidic diode, valves, and a sequential-loading circuit fabricated on layered paper. Lab Chip. 12, 2909-2913 (2012).
B. J. Toley et al., A versatile valving toolkit for automating fluidic operations in paper microfluidic devices. Lab Chip. 15, 1432-1444 (2015).
B. M. Cummins, R. Chinthapatla, F. S. Ligler, G. M. Walker, Time-Dependent Model for Fluid Flow in Porous Materials with Multiple Pore Sizes. Anal. Chem. 89, 4377-4381 (2017).
V. V. Doña, C. A. Fossati, F. G. Chirdo, Interference of denaturing and reducing agents on the antigen/antibody interaction. Impact on the performance of quantitative immunoassays in gliadin analysis. Eur. Food Res. Technol. 226, 591-602 (2008).
S. Jahanshahi-Anbuhi et al., Creating fast flow channels in paper fluidic devices to control timing of sequential reactions. Lab Chip. 12, 5079-5085 (2012).
V. R. N. Telis, J. Telis-Romero, H. B. Mazzotti, A. L. Gabas, Viscosity of aqueous carbohydrate solutions at different temperatures and concentrations. Int. J. Food Prop. 10, 185-195 (2007).
J. B. Segur, H. E. Oberstar, Viscosity of glycerol and its aqueous solutions. Ind. Eng. Chem. Res. 43, 2117-2120 (1951).

## Claims

1. A device, comprising:
(i) a first adhesive material;
(ii) a substrate configured to move a fluid by capillary action, wherein the substrate has a first substrate side and a second substrate side opposite the first substrate side, further wherein the first substrate side is coupled to and covered by at least a portion of the first adhesive material;
(iii) a second adhesive material, wherein the second adhesive material is coupled to at least a portion of the second substrate side;
(iv) a first line of a non-delaminating ink and a second line of the non-delaminating ink disposed on the first substrate side, wherein the first line and the second line define a channel;
(v) one or more lines of a delaminating ink extending between the first line and the second line disposed on the first substrate side,
wherein the non-delaminating ink is comparatively more hydrophobic than the delaminating ink and remains tethered between the first substrate side and the at least a portion of the first adhesive material for a longer period of time than the delaminating ink; and
wherein delamination between the first substrate side and the at least a portion of the first adhesive material temporarily delays movement of the fluid by capillary action.

2. The device of claim 1, wherein the device comprises a lateral flow assay (LFA).

3. The device of any one of claims 1-2, wherein the non-delaminating ink comprises a hydrophobic resin.

4. The device of any one of claims 1-3, wherein the widths of the one or more lines of the delaminating ink are each at least 0.1 mm.

5. The device of any one of claims 1-4, further comprising at least one sample inlet and at least one auxiliary inlet.

6. The device of any one of claims 1-5, wherein a contact angle between the delaminating ink and the substrate is from 0° to 180°.

7. The device of any one of claims 1-6, wherein the first adhesive material is treated with corona discharge.

8. The device of any one of claims 1-7, further comprising chemicals to modulate adhesion and/or hydrophobicity.

9. The device of any one of claims 1-8, wherein the first adhesive material, the second adhesive material, and/or the substrate are treated with biomolecules.

10. A method of detecting a target material in a sample, the method comprising testing the sample on the device of any one of claims 1-9.

11. The method of claim 10, wherein the sample comprises urine, blood, plasma, serum, sweat, saliva, or any combination thereof.

12. The method of claim 10 or 11, wherein a first mechanical force is applied to the first substrate side, and wherein a second mechanical force is applied to the second substrate side opposite the first mechanical force.

13. The method of claim 12, wherein the one or more lines of the delaminating ink is disposed between the first mechanical force and the second mechanical force.

14. The method of any one of claims 12-13, wherein the first mechanical force and the second mechanical force are produced by two attracting magnets.

15. The device of any one of claims 1-9, wherein the device further comprises a target antigen LFA strip disposed between at least a portion of the second adhesive material and at least a portion of the second substrate side.

## Patentansprüche

1. Eine Vorrichtung, umfassend:
(i) ein erstes Klebematerial;
(ii) ein Substrat, das dazu konfiguriert ist, ein Fluid durch Kapillarwirkung zu bewegen,
wobei das Substrat eine erste Substratseite und eine zweite Substratseite gegenüber der ersten Substratseite aufweist, wobei ferner die erste Substratseite mit mindestens einem Teil des ersten Klebematerials gekoppelt und von diesem bedeckt ist;
(iii) ein zweites Klebematerial, wobei das zweite Klebematerial mit mindestens einem Teil der zweiten Substratseite gekoppelt ist;
(iv) eine erste Linie einer nicht delaminierenden Tinte und eine zweite Linie der nicht delaminierenden Tinte, die auf der ersten Substratseite angeordnet sind, wobei die erste Linie und die zweite Linie einen Kanal definieren;
(v) eine oder mehrere Linien einer delaminierenden Tinte, die sich zwischen der auf der ersten Substratseite angeordneten ersten Linie und zweiten Linie erstrecken,
wobei die nicht delaminierende Tinte vergleichsweise hydrophober als die delaminierende Tinte ist und zwischen der ersten Substratseite und dem mindestens einen Teil des ersten Klebematerials für einen längeren Zeitraum als die delaminierende Tinte gebunden bleibt; und
wobei eine Delamination zwischen der ersten Substratseite und dem mindestens einen Teil des ersten Klebematerials die Bewegung des Fluids durch Kapillarwirkung vorübergehend verzögert.

2. Die Vorrichtung aus Anspruch 1, wobei die Vorrichtung einen Lateral-Flow-Assay (LFA) umfasst.

3. Die Vorrichtung aus einem der Ansprüche 1-2, wobei die nicht delaminierende Tinte ein hydrophobes Harz umfasst.

4. Die Vorrichtung aus einem der Ansprüche 1-3, wobei die Breiten der einen oder mehreren Linien der delaminierenden Tinte jeweils mindestens 0,1 mm betragen.

5. Die Vorrichtung aus einem der Ansprüche 1-4, ferner umfassend mindestens einen Probeneinlass und mindestens einen Hilfseinlass.

6. Die Vorrichtung aus einem der Ansprüche 1-5, wobei ein Kontaktwinkel zwischen der delaminierenden Tinte und dem Substrat 0° bis 180° beträgt.

7. Die Vorrichtung aus einem der Ansprüche 1-6, wobei das erste Klebematerial mit Koronaentladung behandelt ist.

8. Die Vorrichtung aus einem der Ansprüche 1-7, ferner umfassend Chemikalien zum Modulieren der Adhäsion und/oder der Hydrophobizität.

9. Die Vorrichtung aus einem der Ansprüche 1-8, wobei das erste Klebematerial, das zweite Klebematerial und/oder das Substrat mit Biomolekülen behandelt sind.

10. Ein Verfahren zum Nachweisen eines Zielmaterials in einer Probe, wobei das Verfahren das Testen der Probe auf der Vorrichtung aus einem der Ansprüche 1-9 umfasst.

11. Das Verfahren aus Anspruch 10, wobei die Probe Urin, Blut, Plasma, Serum, Schweiß, Speichel oder eine beliebige Kombination davon umfasst.

12. Das Verfahren aus Anspruch 10 oder 11, wobei eine erste mechanische Kraft auf die erste Substratseite ausgeübt wird, und wobei eine zweite mechanische Kraft gegenüber der ersten mechanischen Kraft auf die zweite Substratseite ausgeübt wird.

13. Das Verfahren aus Anspruch 12, wobei die eine oder mehreren Linien der delaminierenden Tinte zwischen der ersten mechanischen Kraft und der zweiten mechanischen Kraft angeordnet sind.

14. Das Verfahren aus einem der Ansprüche 12-13, wobei die erste mechanische Kraft und die zweite mechanische Kraft durch zwei einander anziehende Magnete erzeugt werden.

15. Die Vorrichtung aus einem der Ansprüche 1-9, wobei die Vorrichtung ferner einen LFA-Streifen für ein Zielantigen umfasst, der zwischen mindestens einem Teil des zweiten Klebematerials und mindestens einem Teil der zweiten Substratseite angeordnet ist.

## Revendications

1. Un dispositif, comprenant :
(i) un premier matériau adhésif ;
(ii) un substrat configuré pour déplacer un fluide par capillarité,
dans lequel le substrat a une première face du substrat et une deuxième face du substrat opposée à la première face du substrat, dans lequel en outre la première face du substrat est couplée à au moins une partie du premier matériau adhésif et recouverte par celle-ci ;
(iii) un deuxième matériau adhésif, dans lequel le deuxième matériau adhésif est couplé à au moins une partie de la deuxième face du substrat ;
(iv) une première ligne d'une encre non délaminante et une deuxième ligne de l'encre non délaminante disposées sur la première face du substrat,
dans lequel la première ligne et la deuxième ligne définissent un canal ;
(v) une ou plusieurs lignes d'une encre délaminante s'étendant entre la première ligne et la deuxième ligne disposées sur la première face du substrat,
dans lequel l'encre non délaminante est comparativement plus hydrophobe que l'encre délaminante et reste attachée entre la première face du substrat et ladite au moins une partie du premier matériau adhésif pendant une durée plus longue que l'encre délaminante ; et
dans lequel une délamination entre la première face du substrat et ladite au moins une partie du premier matériau adhésif retarde temporairement le déplacement du fluide par capillarité.

2. Le dispositif de la revendication 1, dans lequel le dispositif comprend un essai à flux latéral (LFA).

3. Le dispositif de l'une quelconque des revendications 1-2, dans lequel l'encre non délaminante comprend une résine hydrophobe.

4. Le dispositif de l'une quelconque des revendications 1-3, dans lequel les largeurs de la ou des lignes de l'encre délaminante sont chacune d'au moins 0,1 mm.

5. Le dispositif de l'une quelconque des revendications 1-4, comprenant en outre au moins une entrée d'échantillon et au moins une entrée auxiliaire.

6. Le dispositif de l'une quelconque des revendications 1-5, dans lequel un angle de contact entre l'encre délaminante et le substrat est compris entre 0° et 180°.

7. Le dispositif de l'une quelconque des revendications 1-6, dans lequel le premier matériau adhésif est traité par décharge corona.

8. Le dispositif de l'une quelconque des revendications 1-7, comprenant en outre des produits chimiques pour moduler l'adhésion et/ou l'hydrophobicité.

9. Le dispositif de l'une quelconque des revendications 1-8, dans lequel le premier matériau adhésif, le deuxième matériau adhésif, et/ou le substrat sont traités avec des biomolécules.

10. Un procédé de détection d'un matériau cible dans un échantillon, le procédé comprenant le test de l'échantillon sur le dispositif de l'une quelconque des revendications 1-9.

11. Le procédé de la revendication 10, dans lequel l'échantillon comprend de l'urine, du sang, du plasma, du sérum, de la sueur, de la salive, ou une quelconque combinaison de ceux-ci.

12. Le procédé de la revendication 10 ou 11, dans lequel une première force mécanique est appliquée sur la première face du substrat, et dans lequel une deuxième force mécanique est appliquée sur la deuxième face du substrat à l'opposé de la première force mécanique.

13. Le procédé de la revendication 12, dans lequel la ou les lignes de l'encre délaminante sont disposées entre la première force mécanique et la deuxième force mécanique.

14. Le procédé de l'une quelconque des revendications 12-13, dans lequel la première force mécanique et la deuxième force mécanique sont produites par deux aimants qui s'attirent.

15. Le dispositif de l'une quelconque des revendications 1-9, dans lequel le dispositif comprend en outre une bandelette de LFA pour un antigène cible disposée entre au moins une partie du deuxième matériau adhésif et au moins une partie de la deuxième face du substrat.
